(19) Europäisches Patentamt
European Patent Office
Office européen des brevets

(11) EP 0 604 983 B1

(12) EUROPEAN PATENT SPECIFICATION

(45) Date of publication and mention of the grant of the patent: **20.11.1996 Bulletin 1996/47**

(51) Int. Cl.[6]: **C07D 277/20**, C07D 257/04, C07D 277/34, C07D 277/36, A61K 31/44, A61K 31/425, C07D 417/12

(21) Application number: **93121020.7**

(22) Date of filing: **28.12.1993**

(54) **Naphthalene derivatives**
Naphthalin-Derivate
Dérivés du naphtalène

(84) Designated Contracting States:
**AT BE CH DE DK ES FR GB GR IE IT LI LU NL PT SE**

(30) Priority: **28.12.1992 JP 349172/92**

(43) Date of publication of application: **06.07.1994 Bulletin 1994/27**

(73) Proprietor: **Mitsubishi Chemical Corporation**
**Chiyoda-ku Tokyo (JP)**

(72) Inventors:
- **Ueno, Hiroaki**
  **San Diego, CA 92122 (US)**
- **Oe, Takayuki**
  **Yokohama-shi, Kanagawa-ken (JP)**
- **Suehiro, Ichiro**
  **Yokohama-shi, Kanagawa-ken (JP)**
- **Nakamura, Fumiko**
  **Yokohama-shi, Kanagawa-ken (JP)**

(74) Representative: **Hansen, Bernd, Dr. Dipl.-Chem. et al**
**Hoffmann, Eitle & Partner,**
**Patentanwälte,**
**Arabellastrasse 4**
**81925 München (DE)**

(56) References cited:
**EP-A- 0 155 845**
**EP-A- 0 193 256**
**EP-A- 0 207 581**
**EP-A- 0 207 605**
**EP-A- 0 283 035**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 604 983 B1

Printed by Rank Xerox (UK) Business Services
2.13.8/3.4

## Description

Background of the Invention

The present invention relates to novel naphthalene derivatives. In particular, it relates to novel naphthalene derivatives useful for reducing blood sugar and blood lipid levels.

Diabetes is a compound disease caused by hyperglycemia which results from dysfunction of insulin which reduces blood sugar level. Diabetes can be classified into several types based on etiology. Among others, two types of diabetes are most important, one of which is insulin-dependent diabetes mellitus (type I diabetes) which is caused by insulin deficiency and requires insulin supply for the treatment of the disease, and noninsulin-dependent diabetes mellitus (type II diabetes) which is caused by abnormalities of insulin receptors or sugar transporting carriers in spite of sufficient production of insulin.

At present, the treatment of noninsulin-dependent diabetes mellitus is mainly carried out by a combination of ergotherapy, alimentary therapy, and oral administration of anti-hyperglycemic agents, and for severer conditions, insulin preparations are used. As anti-hyperglycemic agents for oral administration there are used sulfonylureas (for example, tolbutamide, acetohexamide, tolazamide, glibenclamide, etc.) and biguanides. However, biguanides are scarcely used because of their side effects such as lactic acidosis and the like. On the other hand, sulfonylureas show potent anti-hyperglycemic activity but can sometimes induce hypoglycemia. Accordingly, sulfonylureas must be used very carefully. In addition, a phenomenon knows as "secondary failure" is seen during the use of sulfonylureas for a long period of time, which means gradual decrease of effectiveness.

Although a variety of new anti-hyperglycemic agents having less side effects than sulfonylureas have been currently developed, most of them have not been put into practical use due to their insufficient activities and side effects.

In recent years, insulin-resistance ameriolating agents have attracted the attention of people concerned, which reduce blood sugar level by ameriolating insulin-resistance in peripheral tissues, which is one of the causes of noninsulin-dependent diabetes mellitus. However, conventional insulin-resistance ameriolating agents are unsatisfactory because of their insufficient desirable effect and undersirable side effects, and it has long been desired to develop new agents which have more powerful effect and less side effects.

Japanese patent publication (Kokai) No. 48471/1984 discloses thiazolidine derivatives which reduce blood sugar and triglyceride levels in blood plasma. The derivatives are represented by the following formula:

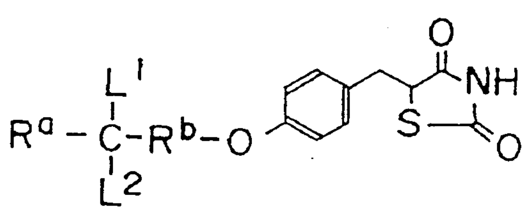

wherein each of $L^1$ and $L^2$ is defined as hydrogen when $R^a$ is a suitably substituted phenyl, and $R^b$ is a bond or a lower alkylene.

Japanese patent publication (Kokai) No. 267580/1988 discloses thiazolidinedione derivatives having an ability of reducing blood sugar and blood lipid levels, which are represented by the following formula:

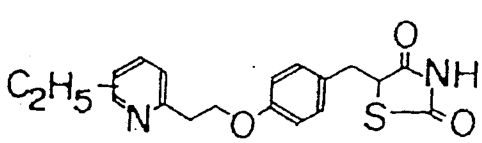

Further, US patent No. 4,703,052 describes thiazolidinedione derivatives having an ability of reducing blood sugar and blood lipid levels, which are represented by the following formula:

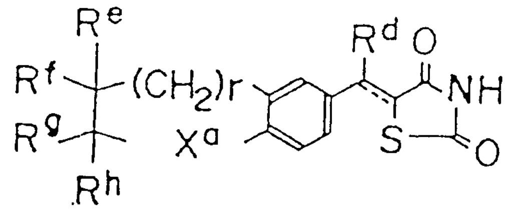

wherein the dotted line is an arbitrary bond; $R^c$ is hydrogen, methyl or ethyl, $X^a$ is O, S, SO, $SO_2$, $CH_2$, CO, CHOH or $NR^h$ ($R^h$ is hydrogen) or acyl group; $R^d$, $R^e$ and $R^f$ are hydrogen or methyl; and $R^g$ is a substituted phenyl, benzyl, phenethyl or styryl.

British patent No. 8713861 discloses thiazolidinedione derivatives having an ability of reducing blood sugar and blood lipid levels, which are represented by the following formula:

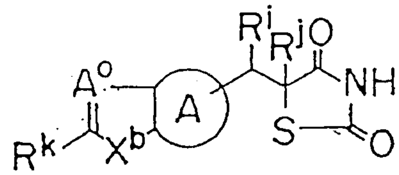

wherein $A^o$ represents nitrogen or $R^t$-C(=)- moiety, $R^k$ represents $R^t$-$Y^a$-Z wherein $R^t$ represents a substituted or unsubstituted phenyl, pyridyl or oxazolyl group, $Y^a$ represents $-(CH_2)n^a-$ ($n^a$ stands for an integer of 0 to 6) and Z represents $-CH_2-$, -CH(OH)- or -CO-; each of $R^i$ and $R^j$ represents hydrogen or $R^i$ and $R^j$ combine together to form a bond; A represents a residue of a benzene ring; and $X^b$ represents O or S).

Further, Japanese patent publication (Kokai) No. 56675/ 1989 discloses thiazolidinedione derivatives having an ability of reducing blood sugar level, which are represented by the following formula:

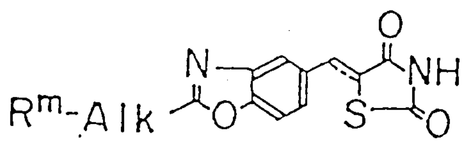

wherein $R^m$ represents phenyl, naphthyl, cycloalkyl or heterocycle, all of which may be substituted; Alk represents a single bond, lower alkenylene, lower alkynylene, or lower alkylene which may be substituted; and the dotted line represents a bond which may be a double bond.

Other thiazidine dione derivatives with hypoglycemic activity are for example known from EP-A-0 207 581, EP-A-0 193 256 and EP-A-0 155 845.

As described above, among thiazolidinedione derivatives having an ability of reducing blood sugar and blood lipid lebels, and which have been disclosed so far, there has been no compound wherein the aromatic ring moiety to which 5-(2,4-thiazolidinedione)-methyl group or 5-(2,4-thiazolidinedione)-methylene group is attached has a naphthalene structure.

On the other hand, US patent No. 4,997,948 issued to Zask et al. discloses naphthalenylsulfonyl thiazolidinedione derivatives having an ability of reducing blood sugar level, which are represented by the following formula:

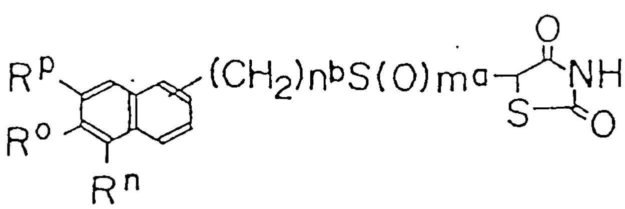

wherein $R^n$ represents hydrogen, bromine, chlorine, trifluoromethyl or difluoroethyl; $R^o$ represents hydrogen, hydroxyl, methoxyl or ethoxyl when $R^p$ represents hydrogen, or both $R^o$ and $R^p$ represent methoxycarbonyloxyl or ethoxycarbonyloxyl; $m^a$ represent 0 or 2; and $n^b$ represents 0 or 1. However, their effect of reducing blood sugar can not be said to be sufficient.

Further, Zask et al., J. Med. Chem. , 33 (5): 1418-1423 (1990) discloses thiazolidine derivatives showing the effect of reducing blood sugar level, which are represented by the following formula:

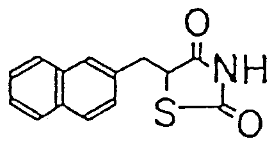

but such compounds can not be said to have sufficient effect on reducing blood sugar level.

Keath et al., J. Med. Chem., 32 (1): 11-13 (1989) discloses tetrazole derivatives showing an effect of reducing blood sugar level, which are represents by the following formula:

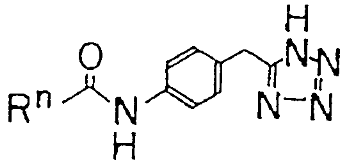

wherein $R^n$ represents $C_1$ - $C_{10}$ perfluoroalkyl.

European patent publication No. 393941 discloses naphthalenylalkyl-3H-1,2,3,5-oxathiadiazole-2-oxides showing the blood sugar level reducing effect, which are represented by the following formula:

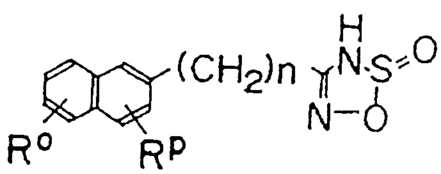

wherein $R^o$ and $R^p$ represent independently hydrogen, lower alkyl having 1 to 6 carbon atoms, lower alkoxyl having 1 to 6 carbon atoms, halogen, ethynyl, nitrile, methylthio, trifluoromethyl, vinyl, nitro or halogen-substituted benzyloxyl; and n represents 0 to 4.

Further, European patent publication No. 343643 discloses a compound represented by the following formula:

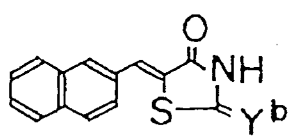

wherein $Y^b$ represents an oxygen atom or sulfur atom, which are compounds having a structure similar to that of the compounds of the present invention. They are different from the compounds of the present invention in the substituents attached to the naphthalene ring. In addition, the above publication describes that the object is to use for treatment of allergy or inflammation and it refers to nothing for the reduction of the blood sugar and blood lipid levels, which is the object of the present invention.

Summary of the Invention

The subject matter of the present invention is to provide novel naphthalene derivatives exhibiting the excellent effect on reducing blood sugar and blood lipid levels.

The inventors of the present invention synthesized various compounds and evaluated their effect on reducing blood sugar and blood lipid levels. Consequently, it was found that novel naphthalene derivatives represented by the general formula I are excellent in said effect. The present invention has been accomplished based on such finding.

Namely, the gist of the present invention exists in providing naphthalene derivatives represented by the following formula (I):

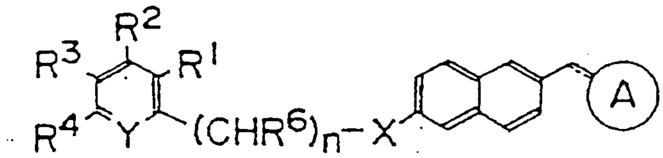

- - - (I)

wherein the symbol

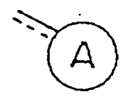

represents

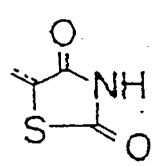

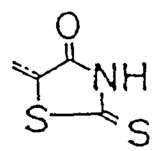

or

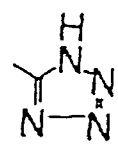

;

-X- represents -O- or -S-; =Y- represents =N- or =CR$^5$-; each of R$^1$, R$^2$, R$^3$, R$^4$ and R$^5$ represents independently hydrogen, halogen, alkyl, aryl, alkoxy, alkoxyalkoxy, aryloxy, alkanoyloxy, arylcarbonyloxy, carboxy, alkoxycarbonyl, aryloxycarbonyl, carbamoyl, alkylaminocarbonyl, arylaminocarbonyl, amino, alkylamino, alkanoylamino, arylcarbonylamino, ethylenedioxymethyl, formyl, cyano, nitro or trihalomethyl; R$^6$ represents hydrogen, alkyl which may be substituted or aryl which may be substituted; n represents an integer of 0 to 3; and the dotted and solid lines show that the bond may be a single or double bond; or a pharmaceutically acceptable salt thereof.

Detailed Description of the Invention

The present invention is detailedly described below. The compound of the present invention is a naphthalene derivatives represented by the following general formula (I):

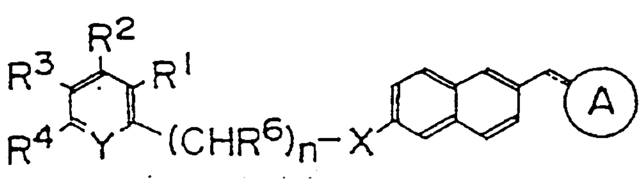

- - - (I)

wherein the symbol

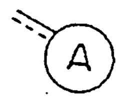

represents

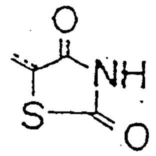

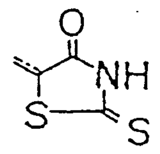 or 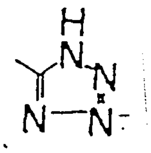 ;

-X- represents -O- or -S-;
=Y- represents =N- or =$CR^5$-;
each of $R^1$, $R^2$, $R^3$, $R^4$ and $R^5$ represents independently hydrogen, halogen (fluorine, chlorine, bromine, iodine, etc.), $C_1$-$C_8$ alkyl (methyl, butyl, octyl, etc.), $C_6$-$C_{12}$ aryl (phenyl, naphthyl, etc.), $C_1$-$C_8$ alkoxy (methoxy, butoxy, octyloxy, etc.), $C_2$-$C_6$ alkoxyalkoxy (methoxymethoxy, methoxyethoxy, ethoxyethoxy, methoxypentoxy, etc.), $C_6$-$C_{12}$ aryloxy (phenyloxy, naphthyloxy, etc.), $C_2$-$C_9$ alkanoyloxy (acetoxy, valeryloxy, hexanoyloxy, etc.), $C_7$-$C_{13}$ arylcarbonyloxy (benzoyloxy, naphthylcarbonyloxy, etc.), carboxy, $C_2$-$C_9$ alkoxycarbonyl (methoxycarbonyl, butyloxycarbonyl, octyloxycarbonyl, etc.), $C_7$-$C_{13}$ aryloxycarbonyl (phenyloxycarbonyl, naphthyloxycarbonyl, etc.), carbamoyl, $C_2$-$C_9$ alkylaminocarbonyl (methylaminocarbonyl, butylaminocarbonyl, octylaminocarbonyl, dimethylaminocarbonyl, dibutylaminocarbonyl, etc.), $C_7$-$C_{13}$ arylaminocarbonyl (phenylaminocarbonyl, naphthylaminocarbonyl, etc.), amino, $C_1$-$C_8$ alkylamino (methylamino, butylamino, octylamino, dimethylamino, dibutylamino, etc.), $C_2$-$C_9$ alkanoylamino (acetylamino, valerylamino, hexanoylamino, etc.), $C_7$-$C_{13}$ arylcarbonylamino (benzoylamino, naphthylcarbonylamino, etc.), ethylenedioxymethyl, formyl, cyano, nitro or trihalomethyl (trifluoromethyl, trichloromethyl, tribromomethyl, triiodomethyl, etc.);
$R^6$ represents hydrogen, $C_1$-$C_8$ alkyl (methyl, butyl, octyl, etc.) which may be substituted by one or more substituents selected from the group consisting of phenyl, halogen (fluorine, chlorine, bromine, iodine, etc.), nitro and cyano, or $C_6$-$C_{12}$ aryl (phenyl, naphthyl, etc.) which may be substitued by one or more substituents selected from the group consisting of $C_1$-$C_8$ alkyl (methyl, butyl, octyl, etc.), halogen (fluorine, chlorine, iodine, etc.), nitro and cyano; n represents an integer of 0 to 3; and the dotted line shows that the bond at the corresponding position may be a double bond; or a pharmaceutically acceptable salt thereof.

Preferred compounds in the present invention include a compound represented by formula (I) wherein each of $R^1$, $R^2$, $R^3$, $R^4$ and $R^5$ represents independently hydrogen, halogen, $C_1$-$C_8$ alkyl, $C_1$-$C_8$ alkoxy, $C_2$-$C_6$ alkoxyalkoxy, $C_2$-$C_9$ alkanoyloxy, $C_7$-$C_{13}$ arylcarbonyloxy, carboxy, $C_2$-$C_9$ alkoxycarbony, carbamoyl, $C_2$-$C_9$ alkylaminocarbonyl, $C_7$-$C_{13}$ arylaminocarbonyl, amino, $C_1$-$C_8$ alkylamino, $C_2$-$C_9$ alkanoylamino, $C_7$-$C_{13}$ arylcarbonylamino, ethylenedioxymethyl, formyl, cyano, nitro or trihalomethyl; $R^6$ represents hydrogen, $C_1$-$C_8$ alkyl, or $C_6$-$C_{12}$ aryl which may be substituted by halogen.

Especially preferred compounds of the present invention include a compound represented by formula (I) wherein -X- represents -O-; =Y- represents =$CR^5$-; each of $R^1$, $R^2$, $R^3$, $R^4$ and $R^5$ represents independently hydrogen, halogen, $C_1$-$C_5$ alkyl, $C_1$-$C_5$ alkoxy, $C_2$-$C_6$ alkoxyalkoxy, $C_2$-$C_6$ alkanoyloxy, carboxy, $C_2$-$C_6$ alkoxycarbonyl, $C_7$-$C_{13}$ arylaminocarbonyl, amino, $C_2$-$C_6$ alkanoylamino, ethylenedioxymethyl, formyl, cyano, nitro or trihalomethyl; $R^6$ represents hydrogen, $C_1$-$C_5$ alkyl or $C_6$-$C_{12}$ aryl which may be substituted by halogen.

Further, most preferable compounds of the present invention include a compound represented by the formula (I) wherein the symbol

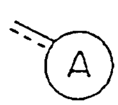

represents

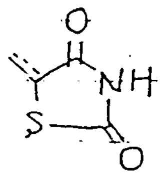 ;

-X- represents -O-; =Y- represents =$CR^5$-; each of $R^1$, $R^2$, $R^3$ and $R^4$ represents independently hydrogen or halogen; $R^5$ represents hydrogen; $R^6$ represents hydrogen; n represents 1; and the bond represented by the dotted and solid lines is a single bond.

Salts with a naphthalene derivatives represented by the above formula (I) include salts with the non-toxic bases, and preferable salts include salts with inorganic bases such as sodium salts, potassium salts and the like, and salts with organic bases such as ammonium salts, trimethylamine salts.

The present invention include compounds which contain an asymmetric carbon atom. In this case, the present invention also includes the isolated stereoisomers and the mixture of the stereoisomers.

The particular examples of the compounds of the present invention are shown in Tables 1, 2, 3 and 4.

The compounds in Table 1 (compound Nos. 8 - 614) are represented by the following formula (I-a):

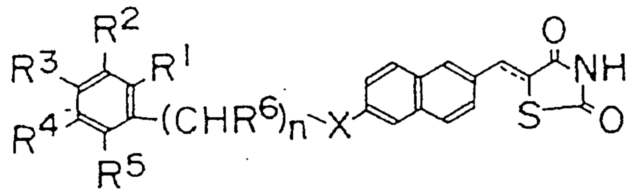

(I-a)

The compounds in Table 2 (compound Nos. 615 - 718) are represented by the following formula (I-b):

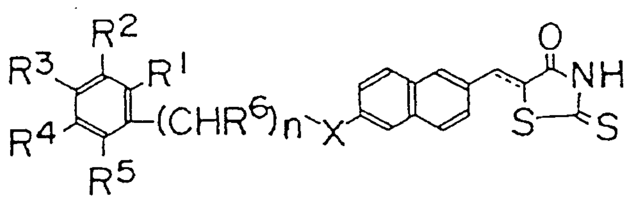

(I-b)

The compounds in Table 3 (compound Nos. 719 - 770) are represented by the following formula (I-c):

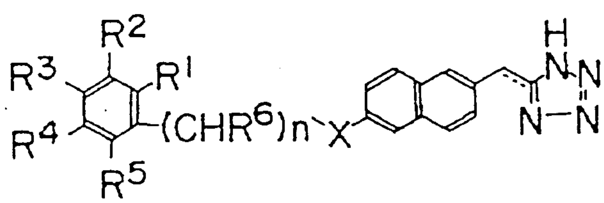

(I-c)

The compounds in Table 4 (compound Nos. 771 - 822) are represented by the following formula (I-d):

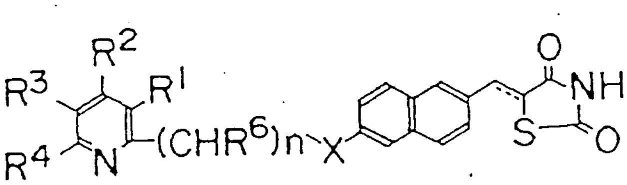

(I-d)

The right end columns in the tables show the bond represented by the dotted and solid lines is either a single bond or double bond. The letter of "n" positioned at the right side of alkyl groups in the tables shows that the corresponding alkyl group is a linear chain.

Table-1

| Compound No. | $R^1$ | $R^2$ | $R^3$ | $R^4$ | $R^5$ | $R^6$ | n | X | 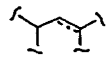 |
|---|---|---|---|---|---|---|---|---|---|
| 1 | -H | -H | -H | -H | -H | — | 0 | O | |
| 2 | -H | -H | -H | -H | -H | -H | 1 | O | |
| 3 | -H | -H | -H | -H | -H | -H | 2 | O | |
| 4 | -H | -H | -H | -H | -H | -H | 3 | O | |
| 5 | -F | -H | -H | -H | -H | -H | 1 | O | |
| 6 | -H | -F | -H | -H | -H | -H | 1 | O | |
| 7 | -H | -H | -F | -H | -H | -H | 1 | O | |
| 8 | -Cl | -H | -H | -H | -H | -H | 1 | O | |
| 9 | -H | -Cl | -H | -H | -H | -H | 1 | O | |
| 10 | -H | -H | -Cl | -H | -H | -H | 1 | O | A single bond |
| 11 | -Br | -H | -H | -H | -H | -H | 1 | O | |
| 12 | -H | -Br | -H | -H | -H | -H | 1 | O | |
| 13 | -H | -H | -Br | -H | -H | -H | 1 | O | |
| 14 | -I | -H | -H | -H | -H | -H | 1 | O | |
| 15 | -H | -I | -H | -H | -H | -H | 1 | O | |
| 16 | -H | -H | -I | -H | -H | -H | 1 | O | |
| 17 | $-CH_3$ | -H | -H | -H | -H | -H | 1 | O | |
| 18 | -H | $-CH_3$ | -H | -H | -H | -H | 1 | O | |
| 19 | -H | -H | $-CH_3$ | -H | -H | -H | 1 | O | |
| 20 | $-C_2H_5$ | -H | -H | -H | -H | -H | 1 | O | |
| 21 | -H | $-C_2H_5$ | -H | -H | -H | -H | 1 | O | |
| 22 | -H | -H | $-C_2H_5$ | -H | -H | -H | 1 | O | |
| 23 | $-C_3H_7{}^n$ | -H | -H | -H | -H | -H | 1 | O | |
| 24 | -H | $-C_3H_7{}^n$ | -H | -H | -H | -H | 1 | O | |
| 25 | -H | -H | $-C_3H_7{}^n$ | -H | -H | -H | 1 | O | |
| 26 | $-CH(CH_3)_2$ | -H | -H | -H | -H | -H | 1 | O | |

Table-1 (continued)

| Compound No. | $R^1$ | $R^2$ | $R^3$ | $R^4$ | $R^5$ | $R^6$ | n | X | 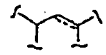 |
|---|---|---|---|---|---|---|---|---|---|
| 27 | $-H$ | $-CH(CH_3)_2$ | $-H$ | $-H$ | $-H$ | $-H$ | 1 | O | A single bond |
| 28 | $-H$ | $-H$ | $-CH(CH_3)_2$ | $-H$ | $-H$ | $-H$ | 1 | O | |
| 29 | $-C_4H_9^n$ | $-H$ | $-H$ | $-H$ | $-H$ | $-H$ | 1 | O | |
| 30 | $-C_5H_{11}^n$ | $-H$ | $-H$ | $-H$ | $-H$ | $-H$ | 1 | O | |
| 31 | $-C_6H_{13}^n$ | $-H$ | $-H$ | $-H$ | $-H$ | $-H$ | 1 | O | |
| 32 | $-C_7H_{15}^n$ | $-H$ | $-H$ | $-H$ | $-H$ | $-H$ | 1 | O | |
| 33 | $-OCH_3$ | $-H$ | $-H$ | $-H$ | $-H$ | $-H$ | 1 | O | |
| 34 | $-H$ | $-OCH_3$ | $-H$ | $-H$ | $-H$ | $-H$ | 1 | O | |
| 35 | $-H$ | $-H$ | $-OCH_3$ | $-H$ | $-H$ | $-H$ | 1 | O | |
| 36 | $-OC_2H_5$ | $-H$ | $-H$ | $-H$ | $-H$ | $-H$ | 1 | O | |
| 37 | $-H$ | $-OC_2H_5$ | $-H$ | $-H$ | $-H$ | $-H$ | 1 | O | |
| 38 | $-H$ | $-H$ | $-OC_2H_5$ | $-H$ | $-H$ | $-H$ | 1 | O | |
| 39 | $-OC_3H_7^n$ | $-H$ | $-H$ | $-H$ | $-H$ | $-H$ | 1 | O | |
| 40 | $-H$ | $-OC_3H_7^n$ | $-H$ | $-H$ | $-H$ | $-H$ | 1 | O | |
| 41 | $-H$ | $-H$ | $-OC_3H_7^n$ | $-H$ | $-H$ | $-H$ | 1 | O | |
| 42 | $-OCH(CH_3)_2$ | $-H$ | $-H$ | $-H$ | $-H$ | $-H$ | 1 | O | |
| 43 | $-H$ | $-OCH(CH_3)_2$ | $-H$ | $-H$ | $-H$ | $-H$ | 1 | O | |
| 44 | $-H$ | $-H$ | $-OCH(CH_3)_2$ | $-H$ | $-H$ | $-H$ | 1 | O | |
| 45 | $-OC_4H_9^n$ | $-H$ | $-H$ | $-H$ | $-H$ | $-H$ | 1 | O | |
| 46 | $-OC_5H_{11}^n$ | $-H$ | $-H$ | $-H$ | $-H$ | $-H$ | 1 | O | |
| 47 | $-OC_6H_{13}^n$ | $-H$ | $-H$ | $-H$ | $-H$ | $-H$ | 1 | O | |
| 48 | $-OC_7H_{15}^n$ | $-H$ | $-H$ | $-H$ | $-H$ | $-H$ | 1 | O | |
| 49 | $-OCOCH_3$ | $-H$ | $-H$ | $-H$ | $-H$ | $-H$ | 1 | O | |
| 50 | $-H$ | $-OCOCH_3$ | $-H$ | $-H$ | $-H$ | $-H$ | 1 | O | |
| 51 | $-H$ | $-H$ | $-OCOCH_3$ | $-H$ | $-H$ | $-H$ | 1 | O | |
| 52 | $-OCOC_2H_5$ | $-H$ | $-H$ | $-H$ | $-H$ | $-H$ | 1 | O | |

Table-1 (continued)

| Compound No. | $R^1$ | $R^2$ | $R^3$ | $R^4$ | $R^5$ | $R^6$ | n | X | 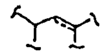 |
|---|---|---|---|---|---|---|---|---|---|
| 53 | $-OCOC_3H_7^n$ | $-H$ | $-H$ | $-H$ | $-H$ | $-H$ | 1 | O | |
| 54 | $-OCOCH(CH_3)_2$ | $-H$ | $-H$ | $-H$ | $-H$ | $-H$ | 1 | O | |
| 55 | $-OCOC_4H_9^n$ | $-H$ | $-H$ | $-H$ | $-H$ | $-H$ | 1 | O | |
| 56 | $-OCOC_5H_{11}^n$ | $-H$ | $-H$ | $-H$ | $-H$ | $-H$ | 1 | O | |
| 57 | $-OCOC_6H_{13}^n$ | $-H$ | $-H$ | $-H$ | $-H$ | $-H$ | 1 | O | |
| 58 | $-OCOC_7H_{15}^n$ | $-H$ | $-H$ | $-H$ | $-H$ | $-H$ | 1 | O | |
| 59 | $-OCOC_6H_5$ | $-H$ | $-H$ | $-H$ | $-H$ | $-H$ | 1 | O | |
| 60 | $-H$ | $-OCOC_6H_5$ | $-H$ | $-H$ | $-H$ | $-H$ | 1 | O | |
| 61 | $-H$ | $-H$ | $-OCOC_6H_5$ | $-H$ | $-H$ | $-H$ | 1 | O | |
| 62 | $-CN$ | $-H$ | $-H$ | $-H$ | $-H$ | $-H$ | 1 | O | |
| 63 | $-H$ | $-CN$ | $-H$ | $-H$ | $-H$ | $-H$ | 1 | O | |
| 64 | $-H$ | $-H$ | $-CN$ | $-H$ | $-H$ | $-H$ | 1 | O | A single bond |
| 65 | $-NO_2$ | $-H$ | $-H$ | $-H$ | $-H$ | $-H$ | 1 | O | |
| 66 | $-H$ | $-NO_2$ | $-H$ | $-H$ | $-H$ | $-H$ | 1 | O | |
| 67 | $-H$ | $-H$ | $-NO_2$ | $-H$ | $-H$ | $-H$ | 1 | O | |
| 68 | $-COOH$ | $-H$ | $-H$ | $-H$ | $-H$ | $-H$ | 1 | O | |
| 69 | $-H$ | $-COOH$ | $-H$ | $-H$ | $-H$ | $-H$ | 1 | O | |
| 70 | $-H$ | $-H$ | $-COOH$ | $-H$ | $-H$ | $-H$ | 1 | O | |
| 71 | $-COOCH_3$ | $-H$ | $-H$ | $-H$ | $-H$ | $-H$ | 1 | O | |
| 72 | $-H$ | $-COOCH_3$ | $-H$ | $-H$ | $-H$ | $-H$ | 1 | O | |
| 73 | $-H$ | $-H$ | $-COOCH_3$ | $-H$ | $-H$ | $-H$ | 1 | O | |
| 74 | $-COOC_2H_5$ | $-H$ | $-H$ | $-H$ | $-H$ | $-H$ | 1 | O | |
| 75 | $-H$ | $-COOC_2H_5$ | $-H$ | $-H$ | $-H$ | $-H$ | 1 | O | |
| 76 | $-H$ | $-H$ | $-COOC_2H_5$ | $-H$ | $-H$ | $-H$ | 1 | O | |
| 77 | $-COOC_3H_7^n$ | $-H$ | $-H$ | $-H$ | $-H$ | $-H$ | 1 | O | |
| 78 | $-H$ | $-COOC_3H_7^n$ | $-H$ | $-H$ | $-H$ | $-H$ | 1 | O | |

Table-1 (continued)

| Compound No. | $R^1$ | $R^2$ | $R^3$ | $R^4$ | $R^5$ | $R^6$ | n | X | 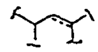 |
|---|---|---|---|---|---|---|---|---|---|
| 79 | −H | −H | $-COOC_3H_7{}^n$ | −H | −H | −H | 1 | O | |
| 80 | $-COOCH(CH_3)_2$ | −H | −H | −H | −H | −H | 1 | O | |
| 81 | −H | $-COOCH(CH_3)_2$ | −H | −H | −H | −H | 1 | O | |
| 82 | −H | −H | $-COOCH(CH_3)_2$ | −H | −H | −H | 1 | O | |
| 83 | $-COOC_4H_9{}^n$ | −H | −H | −H | −H | −H | 1 | O | |
| 84 | $-COOC_5H_{11}{}^n$ | −H | −H | −H | −H | −H | 1 | O | |
| 85 | $-COOC_6H_{13}{}^n$ | −H | −H | −H | −H | −H | 1 | O | |
| 86 | $-COOC_7H_{15}{}^n$ | −H | −H | −H | −H | −H | 1 | O | |
| 87 | $-CONH_2$ | −H | −H | −H | −H | −H | 1 | O | |
| 88 | −H | $-CONH_2$ | −H | −H | −H | −H | 1 | O | |
| 89 | −H | −H | $-CONH_2$ | −H | −H | −H | 1 | O | A single bond |
| 90 | $-CONHCH_3$ | −H | −H | −H | −H | −H | 1 | O | |
| 91 | −H | $-CONHCH_3$ | −H | −H | −H | −H | 1 | O | |
| 92 | −H | −H | $-CONHCH_3$ | −H | −H | −H | 1 | O | |
| 93 | $-CONHC_2H_5$ | −H | −H | −H | −H | −H | 1 | O | |
| 94 | $-CONHC_3H_7{}^n$ | −H | −H | −H | −H | −H | 1 | O | |
| 95 | $-CONHC_4H_9{}^n$ | −H | −H | −H | −H | −H | 1 | O | |
| 96 | $-CONHC_5H_{11}{}^n$ | −H | −H | −H | −H | −H | 1 | O | |
| 97 | $-CONHC_6H_{13}{}^n$ | −H | −H | −H | −H | −H | 1 | O | |
| 98 | $-CONHC_7H_{15}{}^n$ | −H | −H | −H | −H | −H | 1 | O | |
| 99 | $-CONHC_6H_5$ | −H | −H | −H | −H | −H | 1 | O | |
| 100 | −H | $-CONHC_6H_5$ | −H | −H | −H | −H | 1 | O | |
| 101 | −H | −H | $-CONHC_6H_5$ | −H | −H | −H | 1 | O | |
| 102 | $-CON(CH_3)_2$ | −H | −H | −H | −H | −H | 1 | O | |
| 103 | −H | $-CON(CH_3)_2$ | −H | −H | −H | −H | 1 | O | |
| 104 | −H | −H | $-CON(CH_3)_2$ | −H | −H | −H | 1 | O | |

Table-1 (continued)

| Compound No. | $R^1$ | $R^2$ | $R^3$ | $R^4$ | $R^5$ | $R^6$ | n | X | 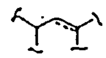 |
|---|---|---|---|---|---|---|---|---|---|
| 105 | $-NH_2$ | $-H$ | $-H$ | $-H$ | $-H$ | $-H$ | 1 | O | A single bond |
| 106 | $-H$ | $-NH_2$ | $-H$ | $-H$ | $-H$ | $-H$ | 1 | O | |
| 107 | $-H$ | $-H$ | $-NH_2$ | $-H$ | $-H$ | $-H$ | 1 | O | |
| 108 | $-NHCH_3$ | $-H$ | $-H$ | $-H$ | $-H$ | $-H$ | 1 | O | |
| 109 | $-H$ | $-NHCH_3$ | $-H$ | $-H$ | $-H$ | $-H$ | 1 | O | |
| 110 | $-H$ | $-H$ | $-NHCH_3$ | $-H$ | $-H$ | $-H$ | 1 | O | |
| 111 | $-NHC_2H_5$ | $-H$ | $-H$ | $-H$ | $-H$ | $-H$ | 1 | O | |
| 112 | $-NHC_3H_7{}^n$ | $-H$ | $-H$ | $-H$ | $-H$ | $-H$ | 1 | O | |
| 113 | $-NHCH(CH_3)_2$ | $-H$ | $-H$ | $-H$ | $-H$ | $-H$ | 1 | O | |
| 114 | $-NHC_4H_9{}^n$ | $-H$ | $-H$ | $-H$ | $-H$ | $-H$ | 1 | O | |
| 115 | $-NHC_5H_{11}{}^n$ | $-H$ | $-H$ | $-H$ | $-H$ | $-H$ | 1 | O | |
| 116 | $-NHC_6H_{13}{}^n$ | $-H$ | $-H$ | $-H$ | $-H$ | $-H$ | 1 | O | |
| 117 | $-NHC_7H_{15}{}^n$ | $-H$ | $-H$ | $-H$ | $-H$ | $-H$ | 1 | O | |
| 118 | $-N(CH_3)_2$ | $-H$ | $-H$ | $-H$ | $-H$ | $-H$ | 1 | O | |
| 119 | $-H$ | $-N(CH_3)_2$ | $-H$ | $-H$ | $-H$ | $-H$ | 1 | O | |
| 120 | $-H$ | $-H$ | $-N(CH_3)_2$ | $-H$ | $-H$ | $-H$ | 1 | O | |
| 121 | $-NHCOCH_3$ | $-H$ | $-H$ | $-H$ | $-H$ | $-H$ | 1 | O | |
| 122 | $-H$ | $-NHCOCH_3$ | $-H$ | $-H$ | $-H$ | $-H$ | 1 | O | |
| 123 | $-H$ | $-H$ | $-NHCOCH_3$ | $-H$ | $-H$ | $-H$ | 1 | O | |
| 124 | $-NHCOC_2H_5$ | $-H$ | $-H$ | $-H$ | $-H$ | $-H$ | 1 | O | |
| 125 | $-NHCOC_3H_7{}^n$ | $-H$ | $-H$ | $-H$ | $-H$ | $-H$ | 1 | O | |
| 126 | $-NHCOCH(CH_3)_2$ | $-H$ | $-H$ | $-H$ | $-H$ | $-H$ | 1 | O | |
| 127 | $-NHCOC_4H_9{}^n$ | $-H$ | $-H$ | $-H$ | $-H$ | $-H$ | 1 | O | |

Table-1 (continued)

| Compound No. | $R^1$ | $R^2$ | $R^3$ | $R^4$ | $R^5$ | $R^6$ | n | X | 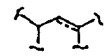 |
|---|---|---|---|---|---|---|---|---|---|
| 128 | $-NHCOC_5H_{11}^n$ | $-H$ | $-H$ | $-H$ | $-H$ | $-H$ | 1 | O | |
| 129 | $-NHCOC_6H_{13}^n$ | $-H$ | $-H$ | $-H$ | $-H$ | $-H$ | 1 | O | |
| 130 | $-NHCOC_7H_{15}^n$ | $-H$ | $-H$ | $-H$ | $-H$ | $-H$ | 1 | O | |
| 131 | $-NHCOC_6H_5$ | $-H$ | $-H$ | $-H$ | $-H$ | $-H$ | 1 | O | |
| 132 | $-H$ | $-NHCOC_6H_5$ | $-H$ | $-H$ | $-H$ | $-H$ | 1 | O | |
| 133 | $-H$ | $-H$ | $-NHCOC_6H_5$ | $-H$ | $-H$ | $-H$ | 1 | O | |
| 134 | $-CHO$ | $-H$ | $-H$ | $-H$ | $-H$ | $-H$ | 1 | O | |
| 135 | $-H$ | $-CHO$ | $-H$ | $-H$ | $-H$ | $-H$ | 1 | O | |
| 136 | $-H$ | $-H$ | $-CHO$ | $-H$ | $-H$ | $-H$ | 1 | O | |
| 137 | 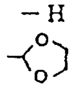 | $-H$ | $-H$ | $-H$ | $-H$ | $-H$ | 1 | O | |
| 138 | $-H$ | 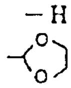 | $-H$ | $-H$ | $-H$ | $-H$ | 1 | O | A single bond |
| 139 | $-H$ | $-H$ | 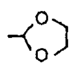 | $-H$ | $-H$ | $-H$ | 1 | O | |
| 140 | $-CF_3$ | $-H$ | $-H$ | $-H$ | $-H$ | $-H$ | 1 | O | |
| 141 | $-H$ | $-CF_3$ | $-H$ | $-H$ | $-H$ | $-H$ | 1 | O | |
| 142 | $-H$ | $-H$ | $-CF_3$ | $-H$ | $-H$ | $-H$ | 1 | O | |
| 143 | $-CCl_3$ | $-H$ | $-H$ | $-H$ | $-H$ | $-H$ | 1 | O | |
| 144 | $-H$ | $-CCl_3$ | $-H$ | $-H$ | $-H$ | $-H$ | 1 | O | |
| 145 | $-H$ | $-H$ | $-CCl_3$ | $-H$ | $-H$ | $-H$ | 1 | O | |
| 146 | $-F$ | $-F$ | $-H$ | $-H$ | $-H$ | $-H$ | 1 | O | |
| 147 | $-F$ | $-H$ | $-F$ | $-H$ | $-H$ | $-H$ | 1 | O | |
| 148 | $-F$ | $-H$ | $-H$ | $-F$ | $-H$ | $-H$ | 1 | O | |
| 149 | $-F$ | $-H$ | $-H$ | $-H$ | $-F$ | $-H$ | 1 | O | |
| 150 | $-H$ | $-F$ | $-F$ | $-H$ | $-H$ | $-H$ | 1 | O | |
| 151 | $-H$ | $-H$ | $-H$ | $-F$ | $-H$ | $-H$ | 1 | O | |
| 152 | $-H$ | $-H$ | $-H$ | $-H$ | $-F$ | $-H$ | 1 | O | |
| 153 | $-F$ | $-H$ | $-F$ | $-H$ | $-F$ | $-H$ | 1 | O | |

Table-1 (continued)

| Compound No. | $R^1$ | $R^2$ | $R^3$ | $R^4$ | $R^5$ | $R^6$ | n | X | 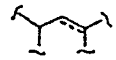 |
|---|---|---|---|---|---|---|---|---|---|
| 154 | −F | −F | −F | −F | −F | −H | 1 | O | |
| 155 | −Cl | −Cl | −H | −H | −H | −H | 1 | O | |
| 156 | −Cl | −H | −Cl | −H | −H | −H | 1 | O | |
| 157 | −Cl | −H | −H | −Cl | −H | −H | 1 | O | |
| 158 | −Cl | −H | −H | −H | −Cl | −H | 1 | O | |
| 159 | −H | −Cl | −Cl | −H | −H | −H | 1 | O | |
| 160 | −H | −Cl | −H | −Cl | −H | −H | 1 | O | |
| 161 | −H | −Cl | −H | −H | −Cl | −H | 1 | O | |
| 162 | −Cl | −H | −Cl | −H | −Cl | −H | 1 | O | |
| 163 | −Cl | −Cl | −Cl | −Cl | −Cl | −H | 1 | O | A single bond |
| 164 | $-CF_3$ | −H | $-CF_3$ | −H | −H | −H | 1 | O | |
| 165 | −H | $-CF_3$ | −H | $-CF_3$ | −H | −H | 1 | O | |
| 166 | −Cl | −H | −F | −H | −H | −H | 1 | O | |
| 167 | −Cl | −H | −H | −H | −F | −H | 1 | O | |
| 168 | −F | $-CF_3$ | −H | −H | −H | −H | 1 | O | |
| 169 | −F | −H | $-CF_3$ | −H | −H | −H | 1 | O | |
| 170 | −F | −H | −H | $-CF_3$ | −H | −H | 1 | O | |
| 171 | −F | −H | −H | −H | $-CF_3$ | −H | 1 | O | |
| 172 | −H | −F | $-CF_3$ | −H | −H | −H | 1 | O | |
| 173 | −H | −F | −H | $-CF_3$ | −H | −H | 1 | O | |
| 174 | $-NO_2$ | $-NO_2$ | −H | −H | −H | −H | 1 | O | |
| 175 | $-NO_2$ | −H | $-NO_2$ | −H | −H | −H | 1 | O | |
| 176 | $-NO_2$ | −H | −H | $-NO_2$ | −H | −H | 1 | O | |
| 177 | $-NO_2$ | −H | −H | −H | $-NO_2$ | −H | 1 | O | |
| 178 | −H | $-NO_2$ | $-NO_2$ | −H | −H | −H | 1 | O | |
| 179 | −H | $-NO_2$ | −H | $-NO_2$ | −H | −H | 1 | O | |

Table-1 (continued)

| Compound No. | $R^1$ | $R^2$ | $R^3$ | $R^4$ | $R^5$ | $R^6$ | n | X | 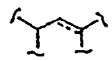 |
|---|---|---|---|---|---|---|---|---|---|
| 180 | $-H$ | $-NO_2$ | $-H$ | $-H$ | $-NO_2$ | $-H$ | 1 | O | |
| 181 | $-F$ | $-H$ | $-H$ | $-NO_2$ | $-H$ | $-H$ | 1 | O | |
| 182 | $-H$ | $-NO_2$ | $-F$ | $-H$ | $-H$ | $-H$ | 1 | O | |
| 183 | $-H$ | $-H$ | $-H$ | $-H$ | $-H$ | — | 0 | S | |
| 184 | $-H$ | $-H$ | $-H$ | $-H$ | $-H$ | $-H$ | 1 | S | |
| 185 | $-H$ | $-H$ | $-H$ | $-H$ | $-H$ | $-H$ | 2 | S | |
| 186 | $-H$ | $-H$ | $-H$ | $-H$ | $-H$ | $-H$ | 3 | S | A single bond |
| 187 | $-F$ | $-H$ | $-H$ | $-H$ | $-H$ | $-H$ | 1 | S | |
| 188 | $-Cl$ | $-H$ | $-H$ | $-H$ | $-H$ | $-H$ | 1 | S | |
| 189 | $-Br$ | $-H$ | $-H$ | $-H$ | $-H$ | $-H$ | 1 | S | |
| 190 | $-I$ | $-H$ | $-H$ | $-H$ | $-H$ | $-H$ | 1 | S | |
| 191 | $-CH_3$ | $-H$ | $-H$ | $-H$ | $-H$ | $-H$ | 1 | S | |
| 192 | $-C_2H_5$ | $-H$ | $-H$ | $-H$ | $-H$ | $-H$ | 1 | S | |
| 193 | $-C_3H_7^n$ | $-H$ | $-H$ | $-H$ | $-H$ | $-H$ | 1 | S | |
| 194 | $-CH(CH_3)_2$ | $-H$ | $-H$ | $-H$ | $-H$ | $-H$ | 1 | S | |
| 195 | $-C_4H_9^n$ | $-H$ | $-H$ | $-H$ | $-H$ | $-H$ | 1 | S | |

Table-1 (continued)

| Compound No. | $R^1$ | $R^2$ | $R^3$ | $R^4$ | $R^5$ | $R^6$ | n | X | 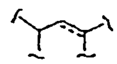 |
|---|---|---|---|---|---|---|---|---|---|
| 196 | $-C_5H_{11}{}^n$ | -H | -H | -H | -H | -H | 1 | S | |
| 197 | $-C_6H_{13}{}^n$ | -H | -H | -H | -H | -H | 1 | S | |
| 198 | $-C_7H_{15}{}^n$ | -H | -H | -H | -H | -H | 1 | S | |
| 199 | $-OCH_3$ | -H | -H | -H | -H | -H | 1 | S | |
| 200 | $-OC_2H_5$ | -H | -H | -H | -H | -H | 1 | S | |
| 201 | $-OC_3H_7{}^n$ | -H | -H | -H | -H | -H | 1 | S | |
| 202 | $-OCH(CH_3)_2$ | -H | -H | -H | -H | -H | 1 | S | |
| 203 | $-OC_4H_9{}^n$ | -H | -H | -H | -H | -H | 1 | S | |
| 204 | $-OC_5H_{11}{}^n$ | -H | -H | -H | -H | -H | 1 | S | |
| 205 | $-OC_6H_{13}{}^n$ | -H | -H | -H | -H | -H | 1 | S | |
| 206 | $-OC_7H_{15}{}^n$ | -H | -H | -H | -H | -H | 1 | S | |
| 207 | $-OCOCH_3$ | -H | -H | -H | -H | -H | 1 | S | A single bond |
| 208 | $-OCOC_2H_5$ | -H | -H | -H | -H | -H | 1 | S | |
| 209 | $-OCOC_3H_7{}^n$ | -H | -H | -H | -H | -H | 1 | S | |
| 210 | $-OCOCH(CH_3)_2$ | -H | -H | -H | -H | -H | 1 | S | |
| 211 | $-OCOC_4H_9{}^n$ | -H | -H | -H | -H | -H | 1 | S | |
| 212 | $-OCOC_5H_{11}{}^n$ | -H | -H | -H | -H | -H | 1 | S | |
| 213 | $-OCOC_6H_{13}{}^n$ | -H | -H | -H | -H | -H | 1 | S | |
| 214 | $-OCOC_7H_{15}{}^n$ | -H | -H | -H | -H | -H | 1 | S | |
| 215 | $-OCOC_6H_5$ | -H | -H | -H | -H | -H | 1 | S | |
| 216 | -CN | -H | -H | -H | -H | -H | 1 | S | |
| 217 | $-NO_2$ | -H | -H | -H | -H | -H | 1 | S | |
| 218 | -COOH | -H | -H | -H | -H | -H | 1 | S | |
| 219 | $-COOCH_3$ | -H | -H | -H | -H | -H | 1 | S | |
| 220 | $-COOC_2H_5$ | -H | -H | -H | -H | -H | 1 | S | |
| 221 | $-COOC_3H_7{}^n$ | -H | -H | -H | -H | -H | 1 | S | |

Table-1 (continued)

| Compound No. | $R^1$ | $R^2$ | $R^3$ | $R^4$ | $R^5$ | $R^6$ | n | X | 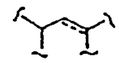 |
|---|---|---|---|---|---|---|---|---|---|
| 222 | $-COOCH(CH_3)_2$ | -H | -H | -H | -H | -H | 1 | S | - |
| 223 | $-COOC_4H_9{}^n$ | -H | -H | -H | -H | -H | 1 | S | - |
| 224 | $-COOC_5H_{11}{}^n$ | -H | -H | -H | -H | -H | 1 | S | - |
| 225 | $-COOC_6H_{13}{}^n$ | -H | -H | -H | -H | -H | 1 | S | - |
| 226 | $-COOC_7H_{15}{}^n$ | -H | -H | -H | -H | -H | 1 | S | - |
| 227 | $-CONH_2$ | -H | -H | -H | -H | -H | 1 | S | |
| 228 | $-CONHCH_3$ | -H | -H | -H | -H | -H | 1 | S | |
| 229 | $-CONHC_2H_5$ | -H | -H | -H | -H | -H | 1 | S | |
| 230 | $-CONHC_3H_7{}^n$ | -H | -H | -H | -H | -H | 1 | S | |
| 231 | $-CONHC_4H_9{}^n$ | -H | -H | -H | -H | -H | 1 | S | |
| 232 | $-CONHC_5H_{11}{}^n$ | -H | -H | -H | -H | -H | 1 | S | A single bond |
| 233 | $-CONHC_6H_{13}{}^n$ | -H | -H | -H | -H | -H | 1 | S | |
| 234 | $-CONHC_7H_{15}{}^n$ | -H | -H | -H | -H | -H | 1 | S | |
| 235 | $-CONHC_6H_5$ | -H | -H | -H | -H | -H | 1 | S | |
| 236 | $-CON(CH_3)_2$ | -H | -H | -H | -H | -H | 1 | S | |
| 237 | $-NH_2$ | -H | -H | -H | -H | -H | 1 | S | |
| 238 | $-NHCH_3$ | -H | -H | -H | -H | -H | 1 | S | |
| 239 | $-NHC_2H_5$ | -H | -H | -H | -H | -H | 1 | S | |
| 240 | $-NHC_3H_7{}^n$ | -H | -H | -H | -H | -H | 1 | S | |
| 241 | $-NHC_4H_9{}^n$ | -H | -H | -H | -H | -H | 1 | S | |
| 242 | $-NHC_5H_{11}{}^n$ | -H | -H | -H | -H | -H | 1 | S | |
| 243 | $-NHC_6H_{13}{}^n$ | -H | -H | -H | -H | -H | 1 | S | |
| 244 | $-NHC_7H_{15}{}^n$ | -H | -H | -H | -H | -H | 1 | S | |
| 245 | $-N(CH_3)_2$ | -H | -H | -H | -H | -H | 1 | S | |
| 246 | $-NHCOCH_3$ | -H | -H | -H | -H | -H | 1 | S | |

Table-1 (continued)

| Compound No. | $R^1$ | $R^2$ | $R^3$ | $R^4$ | $R^5$ | $R^6$ | n | X | 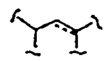 |
|---|---|---|---|---|---|---|---|---|---|
| 247 | $-NHCOC_2H_5$ | −H | −H | −H | −H | −H | 1 | S | |
| 248 | $-NHCOC_3H_7^n$ | −H | −H | −H | −H | −H | 1 | S | |
| 249 | $-NHCOC_4H_9^n$ | −H | −H | −H | −H | −H | 1 | S | |
| 250 | $-NHCOC_5H_{11}^n$ | −H | −H | −H | −H | −H | 1 | S | |
| 251 | $-NHCOC_6H_{13}^n$ | −H | −H | −H | −H | −H | 1 | S | |
| 252 | $-NHCOC_7H_{15}^n$ | −H | −H | −H | −H | −H | 1 | S | |
| 253 | $-NHCOC_6H_5$ | −H | −H | −H | −H | −H | 1 | S | |
| 254 | −CHO | −H | −H | −H | −H | −H | 1 | S | |
| 255 | 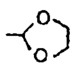 | −H | −H | −H | −H | −H | 1 | S | |
| 256 | $-CF_3$ | −H | −H | −H | −H | −H | 1 | S | A single bond |
| 257 | $-CCl_3$ | −H | −H | −H | −H | −H | 1 | S | |
| 258 | −F | −F | −H | −H | −H | −H | 1 | S | |
| 259 | −F | −H | −F | −H | −H | −H | 1 | S | |
| 260 | −F | −H | −H | −F | −H | −H | 1 | S | |
| 261 | −F | −H | −H | −H | −F | −H | 1 | S | |
| 262 | −H | −F | −F | −H | −H | −H | 1 | S | |
| 263 | −H | −F | −H | −F | −H | −H | 1 | S | |
| 264 | −H | −F | −H | −H | −F | −H | 1 | S | |
| 265 | −F | −H | −F | −H | −F | −H | 1 | S | |
| 266 | −F | −F | −F | −F | −F | −H | 1 | S | |
| 267 | −Cl | −Cl | −H | −H | −H | −H | 1 | S | |
| 268 | −Cl | −H | −Cl | −H | −H | −H | 1 | S | |
| 269 | −Cl | −H | −H | −Cl | −H | −H | 1 | S | |
| 270 | −Cl | −H | −H | −H | −Cl | −H | 1 | S | |
| 271 | −H | −Cl | −Cl | −H | −H | −H | 1 | S | |
| 272 | −H | −H | −H | −Cl | −H | −H | 1 | S | |

Table-1 (continued)

| Compound No. | $R^1$ | $R^2$ | $R^3$ | $R^4$ | $R^5$ | $R^6$ | n | X | 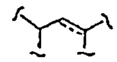 |
|---|---|---|---|---|---|---|---|---|---|
| 273 | -H | -Cl | -H | -H | -Cl | -H | 1 | S | |
| 274 | -Cl | -H | -Cl | -H | -Cl | -H | 1 | S | |
| 275 | -Cl | -Cl | -Cl | -Cl | -Cl | -H | 1 | S | |
| 276 | $-CF_3$ | -H | $-CF_3$ | -H | -H | -H | 1 | S | |
| 277 | -H | $-CF_3$ | -H | $-CF_3$ | -H | -H | 1 | S | |
| 278 | -Cl | -H | -F | -H | -H | -H | 1 | S | |
| 279 | -Cl | -H | -H | -H | -F | -H | 1 | S | |
| 280 | -F | $-CF_3$ | -H | -H | -H | -H | 1 | S | |
| 281 | -F | -H | $-CF_3$ | -H | -H | -H | 1 | S | |
| 282 | -F | -H | -H | $-CF_3$ | -H | -H | 1 | S | A single bond |
| 283 | -F | -H | -H | -H | $-CF_3$ | -H | 1 | S | |
| 284 | -H | -F | $-CF_3$ | -H | -H | -H | 1 | S | |
| 285 | -H | -F | -H | $-CF_3$ | -H | -H | 1 | S | |
| 286 | $-NO_2$ | $-NO_2$ | -H | -H | -H | -H | 1 | S | |
| 287 | $-NO_2$ | -H | $-NO_2$ | -H | -H | -H | 1 | S | |
| 288 | $-NO_2$ | -H | -H | $-NO_2$ | -H | -H | 1 | S | |
| 289 | $-NO_2$ | -H | -H | -H | $-NO_2$ | -H | 1 | S | |
| 290 | -H | $-NO_2$ | $-NO_2$ | -H | -H | -H | 1 | S | |
| 291 | -H | $-NO_2$ | -H | $-NO_2$ | -H | -H | 1 | S | |
| 292 | -H | $-NO_2$ | -H | -H | $-NO_2$ | -H | 1 | S | |
| 293 | -F | -H | -H | $-NO_2$ | -H | -H | 1 | S | |
| 294 | -H | $-NO_2$ | -F | -H | -H | -H | 1 | S | |

Table-1 (continued)

| Compound No. | $R^1$ | $R^2$ | $R^3$ | $R^4$ | $R^5$ | $R^6$ | n | X | 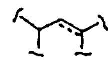 |
|---|---|---|---|---|---|---|---|---|---|
| 295 | -H | -H | -H | -H | -H | — | 0 | O | |
| 296 | -H | -H | -H | -H | -H | -H | 1 | O | |
| 297 | -H | -H | -H | -H | -H | -H | 2 | O | |
| 298 | -H | -H | -H | -H | -H | -H | 3 | O | |
| 299 | -F | -H | -H | -H | -H | -H | 1 | O | |
| 300 | -H | -F | -H | -H | -H | -H | 1 | O | |
| 301 | -H | -H | -F | -H | -H | -H | 1 | O | |
| 302 | -Cl | -H | -H | -H | -H | -H | 1 | O | |
| 303 | -H | -Cl | -H | -H | -H | -H | 1 | O | |
| 304 | -H | -H | -Cl | -H | -H | -H | 1 | O | |
| 305 | -Br | -H | -H | -H | -H | -H | 1 | O | A double bond |
| 306 | -H | -Br | -H | -H | -H | -H | 1 | O | |
| 307 | -H | -H | -Br | -H | -H | -H | 1 | O | |
| 308 | -I | -H | -H | -H | -H | -H | 1 | O | |
| 309 | -H | -I | -H | -H | -H | -H | 1 | O | |
| 310 | -H | -H | -I | -H | -H | -H | 1 | O | |
| 311 | $-CH_3$ | -H | -H | -H | -H | -H | 1 | O | |
| 312 | -H | $-CH_3$ | -H | -H | -H | -H | 1 | O | |
| 313 | -H | -H | $-CH_3$ | -H | -H | -H | 1 | O | |
| 314 | $-C_2H_5$ | -H | -H | -H | -H | -H | 1 | O | |
| 315 | -H | $-C_2H_5$ | -H | -H | -H | -H | 1 | O | |
| 316 | -H | -H | $-C_2H_5$ | -H | -H | -H | 1 | O | |
| 317 | $-C_3H_7^n$ | -H | -H | -H | -H | -H | 1 | O | |
| 318 | -H | $-C_3H_7^n$ | -H | -H | -H | -H | 1 | O | |
| 319 | -H | -H | $-C_3H_7^n$ | -H | -H | -H | 1 | O | |
| 320 | $-CH(CH_3)_2$ | -H | -H | -H | -H | -H | 1 | O | |

Table-1 (continued)

| Compound No. | $R^1$ | $R^2$ | $R^3$ | $R^4$ | $R^5$ | $R^6$ | n | X | 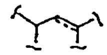 |
|---|---|---|---|---|---|---|---|---|---|
| 321 | $-H$ | $-CH(CH_3)_2$ | $-H$ | $-H$ | $-H$ | $-H$ | 1 | O | |
| 322 | $-H$ | $-H$ | $-CH(CH_3)_2$ | $-H$ | $-H$ | $-H$ | 1 | O | |
| 323 | $-C_4H_9^n$ | $-H$ | $-H$ | $-H$ | $-H$ | $-H$ | 1 | O | |
| 324 | $-C_5H_{11}^n$ | $-H$ | $-H$ | $-H$ | $-H$ | $-H$ | 1 | O | |
| 325 | $-C_6H_{13}^n$ | $-H$ | $-H$ | $-H$ | $-H$ | $-H$ | 1 | O | |
| 326 | $-C_7H_{15}^n$ | $-H$ | $-H$ | $-H$ | $-H$ | $-H$ | 1 | O | |
| 327 | $-OCH_3$ | $-H$ | $-H$ | $-H$ | $-H$ | $-H$ | 1 | O | |
| 328 | $-H$ | $-OCH_3$ | $-H$ | $-H$ | $-H$ | $-H$ | 1 | O | |
| 329 | $-H$ | $-H$ | $-OCH_3$ | $-H$ | $-H$ | $-H$ | 1 | O | |
| 330 | $-OC_2H_5$ | $-H$ | $-H$ | $-H$ | $-H$ | $-H$ | 1 | O | |
| 331 | $-H$ | $-OC_2H_5$ | $-H$ | $-H$ | $-H$ | $-H$ | 1 | O | A double bond |
| 332 | $-H$ | $-H$ | $-OC_2H_5$ | $-H$ | $-H$ | $-H$ | 1 | O | |
| 333 | $-OC_3H_7^n$ | $-H$ | $-H$ | $-H$ | $-H$ | $-H$ | 1 | O | |
| 334 | $-H$ | $-OC_3H_7^n$ | $-H$ | $-H$ | $-H$ | $-H$ | 1 | O | |
| 335 | $-H$ | $-H$ | $-OC_3H_7^n$ | $-H$ | $-H$ | $-H$ | 1 | O | |
| 336 | $-OCH(CH_3)_2$ | $-H$ | $-H$ | $-H$ | $-H$ | $-H$ | 1 | O | |
| 337 | $-H$ | $-OCH(CH_3)_2$ | $-H$ | $-H$ | $-H$ | $-H$ | 1 | O | |
| 338 | $-H$ | $-H$ | $-OCH(CH_3)_2$ | $-H$ | $-H$ | $-H$ | 1 | O | |
| 339 | $-OC_4H_9^n$ | $-H$ | $-H$ | $-H$ | $-H$ | $-H$ | 1 | O | |
| 340 | $-OC_5H_{11}^n$ | $-H$ | $-H$ | $-H$ | $-H$ | $-H$ | 1 | O | |
| 341 | $-OC_6H_{13}^n$ | $-H$ | $-H$ | $-H$ | $-H$ | $-H$ | 1 | O | |
| 342 | $-OC_7H_{15}^n$ | $-H$ | $-H$ | $-H$ | $-H$ | $-H$ | 1 | O | |
| 343 | $-OCOCH_3$ | $-H$ | $-H$ | $-H$ | $-H$ | $-H$ | 1 | O | |
| 344 | $-H$ | $-OCOCH_3$ | $-H$ | $-H$ | $-H$ | $-H$ | 1 | O | |
| 345 | $-H$ | $-H$ | $-OCOCH_3$ | $-H$ | $-H$ | $-H$ | 1 | O | |
| 346 | $-OCOC_2H_5$ | $-H$ | $-H$ | $-H$ | $-H$ | $-H$ | 1 | O | |

Table-1 (continued)

| Compound No. | $R^1$ | $R^2$ | $R^3$ | $R^4$ | $R^5$ | $R^6$ | n | X | 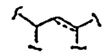 |
|---|---|---|---|---|---|---|---|---|---|
| 347 | $-OCOC_3H_7{}^n$ | −H | −H | −H | −H | −H | 1 | O | |
| 348 | $-OCOCH(CH_3)_2$ | −H | −H | −H | −H | −H | 1 | O | |
| 349 | $-OCOC_4H_9{}^n$ | −H | −H | −H | −H | −H | 1 | O | |
| 350 | $-OCOC_5H_{11}{}^n$ | −H | −H | −H | −H | −H | 1 | O | |
| 351 | $-OCOC_6H_{13}{}^n$ | −H | −H | −H | −H | −H | 1 | O | |
| 352 | $-OCOC_7H_{16}{}^n$ | −H | −H | −H | −H | −H | 1 | O | |
| 353 | $-OCOC_6H_5$ | −H | −H | −H | −H | −H | 1 | O | |
| 354 | −H | $-OCOC_6H_5$ | −H | −H | −H | −H | 1 | O | |
| 355 | −H | −H | $-OCOC_6H_5$ | −H | −H | −H | 1 | O | |
| 356 | −CN | −H | −H | −H | −H | −H | 1 | O | |
| 357 | −H | −CN | −H | −H | −H | −H | 1 | O | |
| 358 | −H | −H | −CN | −H | −H | −H | 1 | O | A double bond |
| 359 | $-NO_2$ | −H | −H | −H | −H | −H | 1 | O | |
| 360 | −H | $-NO_2$ | −H | −H | −H | −H | 1 | O | |
| 361 | −H | −H | $-NO_2$ | −H | −H | −H | 1 | O | |
| 362 | −COOH | −H | −H | −H | −H | −H | 1 | O | |
| 363 | −H | −COOH | −H | −H | −H | −H | 1 | O | |
| 364 | −H | −H | −COOH | −H | −H | −H | 1 | O | |
| 365 | $-COOCH_3$ | −H | −H | −H | −H | −H | 1 | O | |
| 366 | −H | $-COOCH_3$ | −H | −H | −H | −H | 1 | O | |
| 367 | −H | −H | $-COOCH_3$ | −H | −H | −H | 1 | O | |
| 368 | $-COOC_2H_5$ | −H | −H | −H | −H | −H | 1 | O | |
| 369 | −H | $-COOC_2H_5$ | −H | −H | −H | −H | 1 | O | |
| 370 | −H | −H | $-COOC_2H_5$ | −H | −H | −H | 1 | O | |
| 371 | $-COOC_3H_7{}^n$ | −H | −H | −H | −H | −H | 1 | O | |
| 372 | −H | $-COOC_3H_7{}^n$ | −H | −H | −H | −H | 1 | O | |

Table-1 (continued)

| Compound No. | $R^1$ | $R^2$ | $R^3$ | $R^4$ | $R^5$ | $R^6$ | n | X | 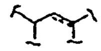 |
|---|---|---|---|---|---|---|---|---|---|
| 373 | -H | -H | $-COOC_3H_7{}^n$ | -H | -H | -H | 1 | O | |
| 374 | $-COOCH(CH_3)_2$ | -H | -H | -H | -H | -H | 1 | O | |
| 375 | -H | $-COOCH(CH_3)_2$ | -H | -H | -H | -H | 1 | O | |
| 376 | -H | -H | $-COOCH(CH_3)_2$ | -H | -H | -H | 1 | O | |
| 377 | $-COOC_4H_9{}^n$ | -H | -H | -H | -H | -H | 1 | O | |
| 378 | $-COOC_5H_{11}{}^n$ | -H | -H | -H | -H | -H | 1 | O | |
| 379 | $-COOC_6H_{13}{}^n$ | -H | -H | -H | -H | -H | 1 | O | |
| 380 | $-COOC_7H_{15}{}^n$ | -H | -H | -H | -H | -H | 1 | O | |
| 381 | $-CONH_2$ | -H | -H | -H | -H | -H | 1 | O | |
| 382 | -H | $-CONH_2$ | -H | -H | -H | -H | 1 | O | |
| 383 | -H | -H | $-CONH_2$ | -H | -H | -H | 1 | O | A double bond |
| 384 | $-CONHCH_3$ | -H | -H | -H | -H | -H | 1 | O | |
| 385 | -H | $-CONHCH_3$ | -H | -H | -H | -H | 1 | O | |
| 386 | -H | -H | $-CONHCH_3$ | -H | -H | -H | 1 | O | |
| 387 | $-CONHC_2H_5$ | -H | -H | -H | -H | -H | 1 | O | |
| 388 | $-CONHC_3H_7{}^n$ | -H | -H | -H | -H | -H | 1 | O | |
| 389 | $-CONHC_4H_9{}^n$ | -H | -H | -H | -H | -H | 1 | O | |
| 380 | $-CONHC_5H_{11}{}^n$ | -H | -H | -H | -H | -H | 1 | O | |
| 391 | $-CONHC_6H_{13}{}^n$ | -H | -H | -H | -H | -H | 1 | O | |
| 392 | $-CONHC_7H_{15}{}^n$ | -H | -H | -H | -H | -H | 1 | O | |
| 393 | $-CONHC_6H_5$ | -H | -H | -H | -H | -H | 1 | O | |
| 394 | -H | $-CONHC_6H_5$ | -H | -H | -H | -H | 1 | O | |
| 395 | -H | -H | $-CONHC_6H_5$ | -H | -H | -H | 1 | O | |
| 396 | $-CON(CH_3)_2$ | -H | -H | -H | -H | -H | 1 | O | |
| 397 | -H | $-CON(CH_3)_2$ | -H | -H | -H | -H | 1 | O | |
| 398 | -H | -H | $-CON(CH_3)_2$ | -H | -H | -H | 1 | O | |

Table-1 (continued)

| Compound No. | $R^1$ | $R^2$ | $R^3$ | $R^4$ | $R^5$ | $R^6$ | n | X | 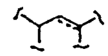 |
|---|---|---|---|---|---|---|---|---|---|
| 399 | $-NH_2$ | −H | −H | −H | −H | −H | 1 | O | |
| 400 | −H | $-NH_2$ | −H | −H | −H | −H | 1 | O | |
| 401 | −H | −H | $-NH_2$ | −H | −H | −H | 1 | O | |
| 402 | $-NHCH_3$ | −H | −H | −H | −H | −H | 1 | O | |
| 403 | −H | $-NHCH_3$ | −H | −H | −H | −H | 1 | O | |
| 404 | −H | −H | $-NHCH_3$ | −H | −H | −H | 1 | O | |
| 405 | $-NHC_2H_5$ | −H | −H | −H | −H | −H | 1 | O | |
| 406 | $-NHC_3H_7^n$ | −H | −H | −H | −H | −H | 1 | O | |
| 407 | $-NHCH(CH_3)_2$ | −H | −H | −H | −H | −H | 1 | O | |
| 408 | $-NHC_4H_9^n$ | −H | −H | −H | −H | −H | 1 | O | A double bond |
| 409 | $-NHC_5H_{11}^n$ | −H | −H | −H | −H | −H | 1 | O | |
| 410 | $-NHC_6H_{13}^n$ | −H | −H | −H | −H | −H | 1 | O | |
| 411 | $-NHC_7H_{15}^n$ | −H | −H | −H | −H | −H | 1 | O | |
| 412 | $-N(CH_3)_2$ | −H | −H | −H | −H | −H | 1 | O | |
| 413 | −H | $-N(CH_3)_2$ | −H | −H | −H | −H | 1 | O | |
| 414 | −H | −H | $-N(CH_3)_2$ | −H | −H | −H | 1 | O | |
| 415 | $-NHCOCH_3$ | −H | −H | −H | −H | −H | 1 | O | |
| 416 | −H | $-NHCOCH_3$ | −H | −H | −H | −H | 1 | O | |
| 417 | −H | −H | $-NHCOCH_3$ | −H | −H | −H | 1 | O | |
| 418 | $-NHCOC_2H_5$ | −H | −H | −H | −H | −H | 1 | O | |
| 419 | $-NHCOC_3H_7^n$ | −H | −H | −H | −H | −H | 1 | O | |
| 420 | $-NHCOCH(CH_3)_2$ | −H | −H | −H | −H | −H | 1 | O | |
| 421 | $-NHCOC_4H_9^n$ | −H | −H | −H | −H | −H | 1 | O | |

Table-1 (continued)

| Compound No. | $R^1$ | $R^2$ | $R^3$ | $R^4$ | $R^5$ | $R^6$ | n | X | 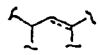 |
|---|---|---|---|---|---|---|---|---|---|
| 422 | $-NHCOC_5H_{11}{}^n$ | $-H$ | $-H$ | $-H$ | $-H$ | $-H$ | 1 | O | |
| 423 | $-NHCOC_6H_{13}{}^n$ | $-H$ | $-H$ | $-H$ | $-H$ | $-H$ | 1 | O | |
| 424 | $-NHCOC_7H_{15}{}^n$ | $-H$ | $-H$ | $-H$ | $-H$ | $-H$ | 1 | O | |
| 425 | $-NHCOC_6H_5$ | $-H$ | $-H$ | $-H$ | $-H$ | $-H$ | 1 | O | |
| 426 | $-H$ | $-NHCOC_6H_5$ | $-H$ | $-H$ | $-H$ | $-H$ | 1 | O | |
| 427 | $-H$ | $-H$ | $-NHCOC_6H_5$ | $-H$ | $-H$ | $-H$ | 1 | O | |
| 428 | $-CHO$ | $-H$ | $-H$ | $-H$ | $-H$ | $-H$ | 1 | O | |
| 429 | $-H$ | $-CHO$ | $-H$ | $-H$ | $-H$ | $-H$ | 1 | O | |
| 430 | $-H$ | $-H$ | $-CHO$ | $-H$ | $-H$ | $-H$ | 1 | O | |
| 431 | 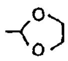 | $-H$ | $-H$ | $-H$ | $-H$ | $-H$ | 1 | O | |
| 432 | $-H$ | 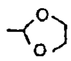 | $-H$ | $-H$ | $-H$ | $-H$ | 1 | O | |
| 433 | $-H$ | $-H$ | 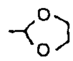 | $-H$ | $-H$ | $-H$ | 1 | O | A double bond |
| 434 | $-CF_3$ | $-H$ | $-H$ | $-H$ | $-H$ | $-H$ | 1 | O | |
| 435 | $-H$ | $-CF_3$ | $-H$ | $-H$ | $-H$ | $-H$ | 1 | O | |
| 436 | $-H$ | $-H$ | $-CF_3$ | $-H$ | $-H$ | $-H$ | 1 | O | |
| 437 | $-CCl_3$ | $-H$ | $-H$ | $-H$ | $-H$ | $-H$ | 1 | O | |
| 438 | $-H$ | $-CCl_3$ | $-H$ | $-H$ | $-H$ | $-H$ | 1 | O | |
| 439 | $-H$ | $-H$ | $-CCl_3$ | $-H$ | $-H$ | $-H$ | 1 | O | |
| 440 | $-F$ | $-F$ | $-H$ | $-H$ | $-H$ | $-H$ | 1 | O | |
| 441 | $-F$ | $-H$ | $-F$ | $-H$ | $-H$ | $-H$ | 1 | O | |
| 442 | $-F$ | $-H$ | $-H$ | $-F$ | $-H$ | $-H$ | 1 | O | |
| 443 | $-F$ | $-H$ | $-H$ | $-H$ | $-F$ | $-H$ | 1 | O | |
| 444 | $-H$ | $-F$ | $-F$ | $-H$ | $-H$ | $-H$ | 1 | O | |
| 445 | $-H$ | $-H$ | $-H$ | $-F$ | $-H$ | $-H$ | 1 | O | |
| 446 | $-H$ | $-H$ | $-H$ | $-H$ | $-F$ | $-H$ | 1 | O | |
| 447 | $-F$ | $-H$ | $-F$ | $-H$ | $-F$ | $-H$ | 1 | O | |

Table-1 (continued)

| Compound No. | $R^1$ | $R^2$ | $R^3$ | $R^4$ | $R^5$ | $R^6$ | n | X | 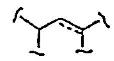 |
|---|---|---|---|---|---|---|---|---|---|
| 448 | -F | -F | -F | -F | -F | -H | 1 | O | |
| 449 | -Cl | -Cl | -H | -H | -H | -H | 1 | O | |
| 450 | -Cl | -H | -Cl | -H | -H | -H | 1 | O | |
| 451 | -Cl | -H | -H | -Cl | -H | -H | 1 | O | |
| 452 | -Cl | -H | -H | -H | -Cl | -H | 1 | O | |
| 453 | -H | -Cl | -Cl | -H | -H | -H | 1 | O | |
| 454 | -H | -Cl | -H | -Cl | -H | -H | 1 | O | |
| 455 | -H | -Cl | -H | -H | -Cl | -H | 1 | O | |
| 456 | -Cl | -H | -Cl | -H | -Cl | -H | 1 | O | |
| 457 | -Cl | -Cl | -Cl | -Cl | -Cl | -H | 1 | O | |
| 458 | $-CF_3$ | -H | $-CF_3$ | -H | -H | -H | 1 | O | A double bond |
| 459 | -H | $-CF_3$ | -H | $-CF_3$ | -H | -H | 1 | O | |
| 460 | -Cl | -H | -F | -H | -H | -H | 1 | O | |
| 461 | -Cl | -H | -H | -H | -F | -H | 1 | O | |
| 462 | -F | $-CF_3$ | -H | -H | -H | -H | 1 | O | |
| 463 | -F | -H | $-CF_3$ | -H | -H | -H | 1 | O | |
| 464 | -F | -H | -H | $-CF_3$ | -H | -H | 1 | O | |
| 465 | -F | -H | -H | -H | $-CF_3$ | -H | 1 | O | |
| 466 | -H | -F | $-CF_3$ | -H | -H | -H | 1 | O | |
| 467 | -H | -F | -H | $-CF_3$ | -H | -H | 1 | O | |
| 468 | $-NO_2$ | $-NO_2$ | -H | -H | -H | -H | 1 | O | |
| 469 | $-NO_2$ | -H | $-NO_2$ | -H | -H | -H | 1 | O | |
| 470 | $-NO_2$ | -H | -H | $-NO_2$ | -H | -H | 1 | O | |
| 471 | $-NO_2$ | -H | -H | -H | $-NO_2$ | -H | 1 | O | |
| 472 | -H | $-NO_2$ | $-NO_2$ | -H | -H | -H | 1 | O | |
| 473 | -H | $-NO_2$ | -H | $-NO_2$ | -H | -H | 1 | O | |

Table-1 (continued)

| Compound No. | $R^1$ | $R^2$ | $R^3$ | $R^4$ | $R^5$ | $R^6$ | n | X | 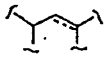 |
|---|---|---|---|---|---|---|---|---|---|
| 474 | -H | $-NO_2$ | -H | -H | $-NO_2$ | -H | 1 | O | |
| 475 | -F | -H | -H | $-NO_2$ | -H | -H | 1 | O | |
| 476 | -H | $-NO_2$ | -F | -H | -H | -H | 1 | O | |
| 477 | -H | -H | -H | -H | -H | — | 0 | S | |
| 478 | -H | -H | -H | -H | -H | -H | 1 | S | |
| 479 | -H | -H | -H | -H | -H | -H | 2 | S | |
| 480 | -H | -H | -H | -H | -H | -H | 3 | S | A double bond |
| 481 | -F | -H | -H | -H | -H | -H | 1 | S | |
| 482 | -Cl | -H | -H | -H | -H | -H | 1 | S | |
| 483 | -Br | -H | -H | -H | -H | -H | 1 | S | |
| 484 | -I | -H | -H | -H | -H | -H | 1 | S | |
| 485 | $-CH_3$ | -H | -H | -H | -H | -H | 1 | S | |
| 486 | $-C_2H_5$ | -H | -H | -H | -H | -H | 1 | S | |
| 487 | $-C_3H_7^n$ | -H | -H | -H | -H | -H | 1 | S | |
| 488 | $-CH(CH_3)_2$ | -H | -H | -H | -H | -H | 1 | S | |
| 489 | $-C_4H_9^n$ | -H | -H | -H | -H | -H | 1 | S | |

Table-1 (continued)

| Compound No. | $R^1$ | $R^2$ | $R^3$ | $R^4$ | $R^5$ | $R^6$ | n | X | 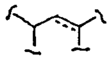 |
|---|---|---|---|---|---|---|---|---|---|
| 490 | $-C_5H_{11}{}^n$ | -H | -H | -H | -H | -H | 1 | S | |
| 491 | $-C_6H_{13}{}^n$ | -H | -H | -H | -H | -H | 1 | S | |
| 492 | $-C_7H_{15}{}^n$ | -H | -H | -H | -H | -H | 1 | S | |
| 493 | $-OCH_3$ | -H | -H | -H | -H | -H | 1 | S | |
| 494 | $-OC_2H_5$ | -H | -H | -H | -H | -H | 1 | S | |
| 495 | $-OC_3H_7{}^n$ | -H | -H | -H | -H | -H | 1 | S | |
| 496 | $-OCH(CH_3)_2$ | -H | -H | -H | -H | -H | 1 | S | |
| 497 | $-OC_4H_9{}^n$ | -H | -H | -H | -H | -H | 1 | S | |
| 498 | $-OC_5H_{11}{}^n$ | -H | -H | -H | -H | -H | 1 | S | |
| 499 | $-OC_6H_{13}{}^n$ | -H | -H | -H | -H | -H | 1 | S | |
| 500 | $-OC_7H_{15}{}^n$ | -H | -H | -H | -H | -H | 1 | S | A double bond |
| 501 | $-OCOCH_3$ | -H | -H | -H | -H | -H | 1 | S | |
| 502 | $-OCOC_2H_5$ | -H | -H | -H | -H | -H | 1 | S | |
| 503 | $-OCOC_3H_7{}^n$ | -H | -H | -H | -H | -H | 1 | S | |
| 504 | $-OCOCH(CH_3)_2$ | -H | -H | -H | -H | -H | 1 | S | |
| 505 | $-OCOC_4H_9{}^n$ | -H | -H | -H | -H | -H | 1 | S | |
| 506 | $-OCOC_5H_{11}{}^n$ | -H | -H | -H | -H | -H | 1 | S | |
| 507 | $-OCOC_6H_{13}{}^n$ | -H | -H | -H | -H | -H | 1 | S | |
| 508 | $-OCOC_7H_{15}{}^n$ | -H | -H | -H | -H | -H | 1 | S | |
| 509 | $-OCOC_6H_5$ | -H | -H | -H | -H | -H | 1 | S | |
| 510 | $-CN$ | -H | -H | -H | -H | -H | 1 | S | |
| 511 | $-NO_2$ | -H | -H | -H | -H | -H | 1 | S | |
| 512 | $-COOH$ | -H | -H | -H | -H | -H | 1 | S | |
| 513 | $-COOCH_3$ | -H | -H | -H | -H | -H | 1 | S | |
| 514 | $-COOC_2H_5$ | -H | -H | -H | -H | -H | 1 | S | |
| 515 | $-COOC_3H_7{}^n$ | -H | -H | -H | -H | -H | 1 | S | |

Table-1 (continued)

| Compound No. | $R^1$ | $R^2$ | $R^3$ | $R^4$ | $R^5$ | $R^6$ | n | X | 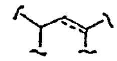 |
|---|---|---|---|---|---|---|---|---|---|
| 516 | $-COOCH(CH_3)_2$ | -H | -H | -H | -H | -H | 1 | S | |
| 517 | $-COOC_4H_9{}^n$ | -H | -H | -H | -H | -H | 1 | S | |
| 518 | $-COOC_5H_{11}{}^n$ | -H | -H | -H | -H | -H | 1 | S | |
| 519 | $-COOC_6H_{13}{}^n$ | -H | -H | -H | -H | -H | 1 | S | |
| 520 | $-COOC_7H_{15}{}^n$ | -H | -H | -H | -H | -H | 1 | S | |
| 521 | $-CONH_2$ | -H | -H | -H | -H | -H | 1 | S | |
| 522 | $-CONHCH_3$ | -H | -H | -H | -H | -H | 1 | S | |
| 523 | $-CONHC_2H_5$ | -H | -H | -H | -H | -H | 1 | S | |
| 524 | $-CONHC_3H_7{}^n$ | -H | -H | -H | -H | -H | 1 | S | |
| 525 | $-CONHC_4H_9{}^n$ | -H | -H | -H | -H | -H | 1 | S | |
| 526 | $-CONHC_5H_{11}{}^n$ | -H | -H | -H | -H | -H | 1 | S | A double bond |
| 527 | $-CONHC_6H_{13}{}^n$ | -H | -H | -H | -H | -H | 1 | S | |
| 528 | $-CONHC_7H_{15}{}^n$ | -H | -H | -H | -H | -H | 1 | S | |
| 529 | $-CONHC_6H_5$ | -H | -H | -H | -H | -H | 1 | S | |
| 530 | $-CON(CH_3)_2$ | -H | -H | -H | -H | -H | 1 | S | |
| 531 | $-NH_2$ | -H | -H | -H | -H | -H | 1 | S | |
| 532 | $-NHCH_3$ | -H | -H | -H | -H | -H | 1 | S | |
| 533 | $-NHC_2H_5$ | -H | -H | -H | -H | -H | 1 | S | |
| 534 | $-NHC_3H_7{}^n$ | -H | -H | -H | -H | -H | 1 | S | |
| 535 | $-NHC_4H_9{}^n$ | -H | -H | -H | -H | -H | 1 | S | |
| 536 | $-NHC_5H_{11}{}^n$ | -H | -H | -H | -H | -H | 1 | S | |
| 537 | $-NHC_6H_{13}{}^n$ | -H | -H | -H | -H | -H | 1 | S | |
| 538 | $-NHC_7H_{15}{}^n$ | -H | -H | -H | -H | -H | 1 | S | |
| 539 | $-N(CH_3)_2$ | -H | -H | -H | -H | -H | 1 | S | |
| 540 | $-NHCOCH_3$ | -H | -H | -H | -H | -H | 1 | S | |

Table-1 (continued)

| Compound No. | $R^1$ | $R^2$ | $R^3$ | $R^4$ | $R^5$ | $R^6$ | n | X | 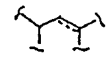 |
|---|---|---|---|---|---|---|---|---|---|
| 541 | $-NHCOC_2H_5$ | $-H$ | $-H$ | $-H$ | $-H$ | $-H$ | 1 | S | |
| 542 | $-NHCOC_3H_7{}^n$ | $-H$ | $-H$ | $-H$ | $-H$ | $-H$ | 1 | S | |
| 543 | $-NHCOC_4H_9{}^n$ | $-H$ | $-H$ | $-H$ | $-H$ | $-H$ | 1 | S | |
| 544 | $-NHCOC_5H_{11}{}^n$ | $-H$ | $-H$ | $-H$ | $-H$ | $-H$ | 1 | S | |
| 545 | $-NHCOC_6H_{13}{}^n$ | $-H$ | $-H$ | $-H$ | $-H$ | $-H$ | 1 | S | |
| 546 | $-NHCOC_7H_{15}{}^n$ | $-H$ | $-H$ | $-H$ | $-H$ | $-H$ | 1 | S | |
| 547 | $-NHCOC_6H_5$ | $-H$ | $-H$ | $-H$ | $-H$ | $-H$ | 1 | S | |
| 548 | $-CHO$ | $-H$ | $-H$ | $-H$ | $-H$ | $-H$ | 1 | S | |
| 549 | 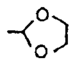 | $-H$ | $-H$ | $-H$ | $-H$ | $-H$ | 1 | S | |
| 550 | $-CF_3$ | $-H$ | $-H$ | $-H$ | $-H$ | $-H$ | 1 | S | |
| 551 | $-CCl_3$ | $-H$ | $-H$ | $-H$ | $-H$ | $-H$ | 1 | S | A double bond |
| 552 | $-F$ | $-F$ | $-H$ | $-H$ | $-H$ | $-H$ | 1 | S | |
| 553 | $-F$ | $-H$ | $-F$ | $-H$ | $-H$ | $-H$ | 1 | S | |
| 554 | $-F$ | $-H$ | $-H$ | $-F$ | $-H$ | $-H$ | 1 | S | |
| 555 | $-F$ | $-H$ | $-H$ | $-H$ | $-F$ | $-H$ | 1 | S | |
| 556 | $-H$ | $-F$ | $-F$ | $-H$ | $-H$ | $-H$ | 1 | S | |
| 557 | $-H$ | $-F$ | $-H$ | $-F$ | $-H$ | $-H$ | 1 | S | |
| 558 | $-H$ | $-F$ | $-H$ | $-H$ | $-F$ | $-H$ | 1 | S | |
| 559 | $-F$ | $-H$ | $-F$ | $-H$ | $-F$ | $-H$ | 1 | S | |
| 560 | $-F$ | $-F$ | $-F$ | $-F$ | $-F$ | $-H$ | 1 | S | |
| 561 | $-Cl$ | $-Cl$ | $-H$ | $-H$ | $-H$ | $-H$ | 1 | S | |
| 562 | $-Cl$ | $-H$ | $-Cl$ | $-H$ | $-H$ | $-H$ | 1 | S | |
| 563 | $-Cl$ | $-H$ | $-H$ | $-Cl$ | $-H$ | $-H$ | 1 | S | |
| 564 | $-Cl$ | $-H$ | $-H$ | $-H$ | $-Cl$ | $-H$ | 1 | S | |
| 565 | $-H$ | $-Cl$ | $-Cl$ | $-H$ | $-H$ | $-H$ | 1 | S | |
| 566 | $-H$ | $-H$ | $-H$ | $-Cl$ | $-H$ | $-H$ | 1 | S | |

Table-1 (continued)

| Compound No. | $R^1$ | $R^2$ | $R^3$ | $R^4$ | $R^5$ | $R^6$ | n | X | 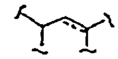 |
|---|---|---|---|---|---|---|---|---|---|
| 567 | -H | -Cl | -H | -H | -Cl | -H | 1 | S | A double bond |
| 568 | -Cl | -H | -Cl | -H | -Cl | -H | 1 | S | |
| 569 | -Cl | -Cl | -Cl | -Cl | -Cl | -H | 1 | S | |
| 570 | $-CF_3$ | -H | $-CF_3$ | -H | -H | -H | 1 | S | |
| 571 | -H | $-CF_3$ | -H | $-CF_3$ | -H | -H | 1 | S | |
| 572 | -Cl | -H | -F | -H | -H | -H | 1 | S | |
| 573 | -Cl | -H | -H | -H | -F | -H | 1 | S | |
| 574 | -F | $-CF_3$ | -H | -H | -H | -H | 1 | S | |
| 575 | -F | -H | $-CF_3$ | -H | -H | -H | 1 | S | |
| 576 | -F | -H | -H | $-CF_3$ | -H | -H | 1 | S | |
| 577 | -F | -H | -H | -H | $-CF_3$ | -H | 1 | S | |
| 578 | -H | -F | $-CF_3$ | -H | -H | -H | 1 | S | |
| 579 | -H | -F | -H | $-CF_3$ | -H | -H | 1 | S | |
| 580 | $-NO_2$ | $-NO_2$ | -H | -H | -H | -H | 1 | S | |
| 581 | $-NO_2$ | -H | $-NO_2$ | -H | -H | -H | 1 | S | |
| 582 | $-NO_2$ | -H | -H | $-NO_2$ | -H | -H | 1 | S | |
| 583 | $-NO_2$ | -H | -H | -H | $-NO_2$ | -H | 1 | S | |
| 584 | -H | $-NO_2$ | $-NO_2$ | -H | -H | -H | 1 | S | |
| 585 | -H | $-NO_2$ | -H | $-NO_2$ | -H | -H | 1 | S | |
| 586 | -H | $-NO_2$ | -H | -H | $-NO_2$ | -H | 1 | S | |
| 587 | -F | -H | -H | $-NO_2$ | -H | -H | 1 | S | |
| 588 | -H | $-NO_2$ | -F | -H | -H | -H | 1 | S | |

Table-1 (continued)

| Compound No. | $R^1$ | $R^2$ | $R^3$ | $R^4$ | $R^5$ | $R^6$ | n | X | 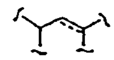 |
|---|---|---|---|---|---|---|---|---|---|
| 589 | -H | -H | -H | -H | -H | $-CH_3$ | 1 | O | A single bond |
| 590 | -H | -H | -H | -H | -H | $-C_2H_5$ | 1 | O | |
| 591 | -H | -H | -H | -H | -H | $-C_6H_5$ | 1 | O | |
| 592 | -F | -H | -H | -H | -H | $-CH_3$ | 1 | O | |
| 593 | -F | -H | -H | -H | -H | $-C_6H_5$ | 1 | O | |
| 594 | -H | -H | -F | -H | -H | $-CH_3$ | 1 | O | |
| 595 | -H | -H | -F | -H | -H | $-C_6H_5$ | 1 | O | |
| 596 | -Cl | -H | -H | -H | -H | $-CH_3$ | 1 | O | |
| 597 | -Cl | -H | -H | -H | -H | $-C_6H_5$ | 1 | O | |
| 598 | -H | -H | -Cl | -H | -H | $-CH_3$ | 1 | O | |
| 599 | -H | -H | -Cl | -H | -H | $-C_6H_5$ | 1 | O | |
| 600 | -H | -H | -F | -H | -H | $-C_6H_4$ (4-F) | 1 | O | |
| 601 | -H | -H | -Cl | -H | -H | $-C_6H_4$ (4-Cl) | 1 | O | A double bond |
| 602 | -H | -H | -H | -H | -H | $-CH_3$ | 1 | O | |
| 603 | -H | -H | -H | -H | -H | $-C_2H_5$ | 1 | O | |
| 604 | -H | -H | -H | -H | -H | $-C_6H_5$ | 1 | O | |
| 605 | -F | -H | -H | -H | -H | $-CH_3$ | 1 | O | |
| 606 | -F | -H | -H | -H | -H | $-C_2H_5$ | 1 | O | |
| 607 | -H | -H | -F | -H | -H | $-CH_3$ | 1 | O | |
| 608 | -H | -H | -F | -H | -H | $-C_2H_5$ | 1 | O | |
| 609 | -Cl | -H | -H | -H | -H | $-CH_3$ | 1 | O | |
| 610 | -Cl | -H | -H | -H | -H | $-C_2H_5$ | 1 | O | |
| 611 | -H | -H | -Cl | -H | -H | $-CH_3$ | 1 | O | |
| 612 | -H | -H | -Cl | -H | -H | $-C_2H_5$ | 1 | O | |
| 613 | -H | -H | -F | -H | -H | $-C_6H_4$ (4-F) | 1 | O | |
| 614 | -H | -H | -Cl | -H | -H | $-C_6H_4$ (4-Cl) | 1 | O | |

Table-2

| Compound No. | $R^1$ | $R^2$ | $R^3$ | $R^4$ | $R^5$ | $R^6$ | n | X | 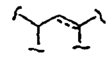 |
|---|---|---|---|---|---|---|---|---|---|
| 615 | −H | −H | −H | −H | −H | — | 0 | O | A single bond |
| 616 | −H | −H | −H | −H | −H | −H | 1 | O | |
| 617 | −H | −H | −H | −H | −H | −H | 2 | O | |
| 618 | −H | −H | −H | −H | −H | −H | 3 | O | |
| 619 | −F | −H | −H | −H | −H | −H | 1 | O | |
| 620 | −H | −H | −F | −H | −H | −H | 1 | O | |
| 621 | −Cl | −H | −H | −H | −H | −H | 1 | O | |
| 622 | −H | −H | −Cl | −H | −H | −H | 1 | O | |
| 623 | −Br | −H | −H | −H | −H | −H | 1 | O | |
| 624 | −H | −H | −Br | −H | −H | −H | 1 | O | |
| 625 | $-CH_3$ | −H | −H | −H | −H | −H | 1 | O | |
| 626 | −H | −H | $-CH_3$ | −H | −H | −H | 1 | O | |
| 627 | $-OCH_3$ | −H | −H | −H | −H | −H | 1 | O | |
| 628 | −H | −H | $-OCH_3$ | −H | −H | −H | 1 | O | |
| 629 | $-OCOCH_3$ | −H | −H | −H | −H | −H | 1 | O | |
| 630 | −H | −H | $-OCOCH_3$ | −H | −H | −H | 1 | O | |
| 631 | −CN | −H | −H | −H | −H | −H | 1 | O | |
| 632 | −H | −H | −CN | −H | −H | −H | 1 | O | |
| 633 | $-NO_2$ | −H | −H | −H | −H | −H | 1 | O | |
| 634 | −H | −H | $-NO_2$ | −H | −H | −H | 1 | O | |
| 635 | −COOH | −H | −H | −H | −H | −H | 1 | O | |
| 636 | −H | −H | −COOH | −H | −H | −H | 1 | O | |
| 637 | $-COOCH_3$ | −H | −H | −H | −H | −H | 1 | O | |
| 638 | −H | −H | $-COOCH_3$ | −H | −H | −H | 1 | O | |
| 639 | $-CONH_2$ | −H | −H | −H | −H | −H | 1 | O | |
| 640 | −H | −H | $-CONH_2$ | −H | −H | −H | 1 | O | |

Table-2 (continued)

| Compound No. | $R^1$ | $R^2$ | $R^3$ | $R^4$ | $R^5$ | $R^6$ | n | X | 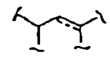 |
|---|---|---|---|---|---|---|---|---|---|
| 641 | $-CONHCH_3$ | $-H$ | $-H$ | $-H$ | $-H$ | $-H$ | 1 | O | A single bond |
| 642 | $-H$ | $-H$ | $-CONHCH_3$ | $-H$ | $-H$ | $-H$ | 1 | O | |
| 643 | $-NH_2$ | $-H$ | $-H$ | $-H$ | $-H$ | $-H$ | 1 | O | |
| 644 | $-H$ | $-H$ | $-NH_2$ | $-H$ | $-H$ | $-H$ | 1 | O | |
| 645 | $-NHCH_3$ | $-H$ | $-H$ | $-H$ | $-H$ | $-H$ | 1 | O | |
| 646 | $-H$ | $-H$ | $-NHCH_3$ | $-H$ | $-H$ | $-H$ | 1 | O | |
| 647 | $-NHCOCH_3$ | $-H$ | $-H$ | $-H$ | $-H$ | $-H$ | 1 | O | |
| 648 | $-H$ | $-H$ | $-NHCOCH_3$ | $-H$ | $-H$ | $-H$ | 1 | O | |
| 649 | $-CHO$ | $-H$ | $-H$ | $-H$ | $-H$ | $-H$ | 1 | O | |
| 650 | $-H$ | $-H$ | $-CHO$ | $-H$ | $-H$ | $-H$ | 1 | O | |
| 651 | $-CF_3$ | $-H$ | $-H$ | $-H$ | $-H$ | $-H$ | 1 | O | |
| 652 | $-H$ | $-H$ | $-CF_3$ | $-H$ | $-H$ | $-H$ | 1 | O | |
| 653 | $-CCl_3$ | $-H$ | $-H$ | $-H$ | $-H$ | $-H$ | 1 | O | |
| 654 | $-H$ | $-H$ | $-CCl_3$ | $-H$ | $-H$ | $-H$ | 1 | O | |
| 655 | $-F$ | $-H$ | $-F$ | $-H$ | $-H$ | $-H$ | 1 | O | |
| 656 | $-F$ | $-H$ | $-H$ | $-H$ | $-F$ | $-H$ | 1 | O | |
| 657 | $-F$ | $-H$ | $-F$ | $-H$ | $-F$ | $-H$ | 1 | O | |
| 658 | $-F$ | $-F$ | $-F$ | $-F$ | $-F$ | $-H$ | 1 | O | |
| 659 | $-Cl$ | $-H$ | $-Cl$ | $-H$ | $-H$ | $-H$ | 1 | O | |
| 660 | $-Cl$ | $-H$ | $-H$ | $-H$ | $-Cl$ | $-H$ | 1 | O | |
| 661 | $-Cl$ | $-H$ | $-Cl$ | $-H$ | $-Cl$ | $-H$ | 1 | O | |
| 662 | $-Cl$ | $-Cl$ | $-Cl$ | $-Cl$ | $-Cl$ | $-H$ | 1 | O | |
| 663 | $-CF_3$ | $-H$ | $-CF_3$ | $-H$ | $-H$ | $-H$ | 1 | O | |
| 664 | $-Cl$ | $-H$ | $-F$ | $-H$ | $-H$ | $-H$ | 1 | O | |
| 665 | $-Cl$ | $-H$ | $-H$ | $-H$ | $-F$ | $-H$ | 1 | O | |
| 666 | $-F$ | $-H$ | $-H$ | $-H$ | $-CF_3$ | $-H$ | 1 | O | |

Table-2 (continued)

| Compound No. | $R^1$ | $R^2$ | $R^3$ | $R^4$ | $R^5$ | $R^6$ | n | X | 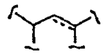 |
|---|---|---|---|---|---|---|---|---|---|
| 667 | -H | -H | -H | -H | -H | — | 0 | O | |
| 668 | -H | -H | -H | -H | -H | -H | 1 | O | |
| 669 | -H | -H | -H | -H | -H | -H | 2 | O | |
| 670 | -H | -H | -H | -H | -H | -H | 3 | O | |
| 671 | -F | -H | -H | -H | -H | -H | 1 | O | |
| 672 | -H | -H | -F | -H | -H | -H | 1 | O | |
| 673 | -Cl | -H | -H | -H | -H | -H | 1 | O | |
| 674 | -H | -H | -Cl | -H | -H | -H | 1 | O | |
| 675 | -Br | -H | -H | -H | -H | -H | 1 | O | |
| 676 | -H | -H | -Br | -H | -H | -H | 1 | O | |
| 677 | $-CH_3$ | -H | -H | -H | -H | -H | 1 | O | |
| 678 | -H | -H | $-CH_3$ | -H | -H | -H | 1 | O | A double bond |
| 679 | $-OCH_3$ | -H | -H | -H | -H | -H | 1 | O | |
| 680 | -H | -H | $-OCH_3$ | -H | -H | -H | 1 | O | |
| 681 | $-OCOCH_3$ | -H | -H | -H | -H | -H | 1 | O | |
| 682 | -H | -H | $-OCOCH_3$ | -H | -H | -H | 1 | O | |
| 683 | -CN | -H | -H | -H | -H | -H | 1 | O | |
| 684 | -H | -H | -CN | -H | -H | -H | 1 | O | |
| 685 | $-NO_2$ | -H | -H | -H | -H | -H | 1 | O | |
| 686 | -H | -H | $-NO_2$ | -H | -H | -H | 1 | O | |
| 687 | -COOH | -H | -H | -H | -H | -H | 1 | O | |
| 688 | -H | -H | -COOH | -H | -H | -H | 1 | O | |
| 689 | $-COOCH_3$ | -H | -H | -H | -H | -H | 1 | O | |
| 690 | -H | -H | $-COOCH_3$ | -H | -H | -H | 1 | O | |
| 691 | $-CONH_2$ | -H | -H | -H | -H | -H | 1 | O | |
| 692 | -H | -H | $-CONH_2$ | -H | -H | -H | 1 | O | |

Table-2 (continued)

| Compound No. | $R^1$ | $R^2$ | $R^3$ | $R^4$ | $R^5$ | $R^6$ | n | X | 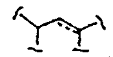 |
|---|---|---|---|---|---|---|---|---|---|
| 693 | $-CONHCH_3$ | $-H$ | $-H$ | $-H$ | $-H$ | $-H$ | 1 | O | A double bond |
| 694 | $-H$ | $-H$ | $-CONHCH_3$ | $-H$ | $-H$ | $-H$ | 1 | O | |
| 695 | $-NH_2$ | $-H$ | $-H$ | $-H$ | $-H$ | $-H$ | 1 | O | |
| 696 | $-H$ | $-H$ | $-NH_2$ | $-H$ | $-H$ | $-H$ | 1 | O | |
| 697 | $-NHCH_3$ | $-H$ | $-H$ | $-H$ | $-H$ | $-H$ | 1 | O | |
| 698 | $-H$ | $-H$ | $-NHCH_3$ | $-H$ | $-H$ | $-H$ | 1 | O | |
| 699 | $-NHCOCH_3$ | $-H$ | $-H$ | $-H$ | $-H$ | $-H$ | 1 | O | |
| 700 | $-H$ | $-H$ | $-NHCOCH_3$ | $-H$ | $-H$ | $-H$ | 1 | O | |
| 701 | $-CHO$ | $-H$ | $-H$ | $-H$ | $-H$ | $-H$ | 1 | O | |
| 702 | $-H$ | $-H$ | $-CHO$ | $-H$ | $-H$ | $-H$ | 1 | O | |
| 703 | $-CF_3$ | $-H$ | $-H$ | $-H$ | $-H$ | $-H$ | 1 | O | |
| 704 | $-H$ | $-H$ | $-CF_3$ | $-H$ | $-H$ | $-H$ | 1 | O | |
| 705 | $-CCl_3$ | $-H$ | $-H$ | $-H$ | $-H$ | $-H$ | 1 | O | |
| 706 | $-H$ | $-H$ | $-CCl_3$ | $-H$ | $-H$ | $-H$ | 1 | O | |
| 707 | $-F$ | $-H$ | $-F$ | $-H$ | $-H$ | $-H$ | 1 | O | |
| 708 | $-F$ | $-H$ | $-H$ | $-H$ | $-F$ | $-H$ | 1 | O | |
| 709 | $-F$ | $-H$ | $-F$ | $-H$ | $-F$ | $-H$ | 1 | O | |
| 710 | $-F$ | $-F$ | $-F$ | $-F$ | $-F$ | $-H$ | 1 | O | |
| 711 | $-Cl$ | $-H$ | $-Cl$ | $-H$ | $-H$ | $-H$ | 1 | O | |
| 712 | $-Cl$ | $-H$ | $-H$ | $-H$ | $-Cl$ | $-H$ | 1 | O | |
| 713 | $-Cl$ | $-H$ | $-Cl$ | $-H$ | $-Cl$ | $-H$ | 1 | O | |
| 714 | $-Cl$ | $-Cl$ | $-Cl$ | $-Cl$ | $-Cl$ | $-H$ | 1 | O | |
| 715 | $-CF_3$ | $-H$ | $-CF_3$ | $-H$ | $-H$ | $-H$ | 1 | O | |
| 716 | $-Cl$ | $-H$ | $-F$ | $-H$ | $-H$ | $-H$ | 1 | O | |
| 717 | $-Cl$ | $-H$ | $-H$ | $-H$ | $-F$ | $-H$ | 1 | O | |
| 718 | $-F$ | $-H$ | $-H$ | $-H$ | $-CF_3$ | $-H$ | 1 | O | |

Table-3

| Compound No. | $R^1$ | $R^2$ | $R^3$ | $R^4$ | $R^5$ | $R^6$ | n | X | 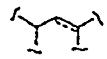 |
|---|---|---|---|---|---|---|---|---|---|
| 719 | −H | −H | −H | −H | −H | —— | 0 | O | |
| 720 | −H | −H | −H | −H | −H | −H | 1 | O | |
| 721 | −H | −H | −H | −H | −H | −H | 2 | O | |
| 722 | −H | −H | −H | −H | −H | −H | 3 | O | |
| 723 | −F | −H | −H | −H | −H | −H | 1 | O | |
| 724 | −H | −H | −F | −H | −H | −H | 1 | O | |
| 725 | −Cl | −H | −H | −H | −H | −H | 1 | O | |
| 726 | −H | −H | −Cl | −H | −H | −H | 1 | O | |
| 727 | −Br | −H | −H | −H | −H | −H | 1 | O | |
| 728 | −H | −H | −Br | −H | −H | −H | 1 | O | |
| 729 | $-CH_3$ | −H | −H | −H | −H | −H | 1 | O | |
| 730 | −H | −H | $-CH_3$ | −H | −H | −H | 1 | O | A single bond |
| 731 | $-OCH_3$ | −H | −H | −H | −H | −H | 1 | O | |
| 732 | −H | −H | $-OCH_3$ | −H | −H | −H | 1 | O | |
| 733 | $-OCOCH_3$ | −H | −H | −H | −H | −H | 1 | O | |
| 734 | −H | −H | $-OCOCH_3$ | −H | −H | −H | 1 | O | |
| 735 | −CN | −H | −H | −H | −H | −H | 1 | O | |
| 736 | −H | −H | −CN | −H | −H | −H | 1 | O | |
| 737 | $-NO_2$ | −H | −H | −H | −H | −H | 1 | O | |
| 738 | −H | −H | $-NO_2$ | −H | −H | −H | 1 | O | |
| 739 | −COOH | −H | −H | −H | −H | −H | 1 | O | |
| 740 | −H | −H | −COOH | −H | −H | −H | 1 | O | |
| 741 | $-COOCH_3$ | −H | −H | −H | −H | −H | 1 | O | |
| 742 | −H | −H | $-COOCH_3$ | −H | −H | −H | 1 | O | |
| 743 | $-CONH_2$ | −H | −H | −H | −H | −H | 1 | O | |
| 744 | −H | −H | $-CONH_2$ | −H | −H | −H | 1 | O | |

Table-3 (continued)

| Compound No. | $R^1$ | $R^2$ | $R^3$ | $R^4$ | $R^5$ | $R^6$ | n | X | 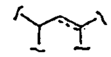 |
|---|---|---|---|---|---|---|---|---|---|
| 745 | $-CONHCH_3$ | -H | -H | -H | -H | -H | 1 | O | |
| 746 | -H | -H | $-CONHCH_3$ | -H | -H | -H | 1 | O | |
| 747 | $-NH_2$ | -H | -H | -H | -H | -H | 1 | O | |
| 748 | -H | -H | $-NH_2$ | -H | -H | -H | 1 | O | |
| 749 | $-NHCH_3$ | -H | -H | -H | -H | -H | 1 | O | |
| 750 | -H | -H | $-NHCH_3$ | -H | -H | -H | 1 | O | |
| 751 | $-NHCOCH_3$ | -H | -H | -H | -H | -H | 1 | O | |
| 752 | -H | -H | $-NHCOCH_3$ | -H | -H | -H | 1 | O | |
| 753 | -CHO | -H | -H | -H | -H | -H | 1 | O | |
| 754 | -H | -H | -CHO | -H | -H | -H | 1 | O | |
| 755 | $-CF_3$ | -H | -H | -H | -H | -H | 1 | O | A single bond |
| 756 | -H | -H | $-CF_3$ | -H | -H | -H | 1 | O | |
| 757 | $-CCl_3$ | -H | -H | -H | -H | -H | 1 | O | |
| 758 | -H | -H | $-CCl_3$ | -H | -H | -H | 1 | O | |
| 759 | -F | -H | -F | -H | -H | -H | 1 | O | |
| 760 | -F | -H | -H | -H | -F | -H | 1 | O | |
| 761 | -F | -H | -F | -H | -F | -H | 1 | O | |
| 762 | -F | -F | -F | -F | -F | -H | 1 | O | |
| 763 | -Cl | -H | -Cl | -H | -H | -H | 1 | O | |
| 764 | -Cl | -H | -H | -H | -Cl | -H | 1 | O | |
| 765 | -Cl | -H | -Cl | -H | -Cl | -H | 1 | O | |
| 766 | -Cl | -Cl | -Cl | -Cl | -Cl | -H | 1 | O | |
| 767 | $-CF_3$ | -H | $-CF_3$ | -H | -H | -H | 1 | O | |
| 768 | -Cl | -H | -F | -H | -H | -H | 1 | O | |
| 769 | -Cl | -H | -H | -H | -F | -H | 1 | O | |
| 770 | -F | -H | -H | -H | $-CF_3$ | -H | 1 | O | |

Table-4

| Compound No. | $R^1$ | $R^2$ | $R^3$ | $R^4$ | $R^6$ | n | X | 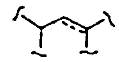 |
|---|---|---|---|---|---|---|---|---|
| 771 | $-H$ | $-H$ | $-H$ | $-H$ | —— | 0 | O | |
| 772 | $-H$ | $-H$ | $-H$ | $-H$ | $-H$ | 1 | O | |
| 773 | $-H$ | $-H$ | $-H$ | $-H$ | $-H$ | 2 | O | |
| 774 | $-H$ | $-H$ | $-H$ | $-H$ | $-H$ | 3 | O | |
| 775 | $-CH_3$ | $-H$ | $-H$ | $-H$ | $-H$ | 1 | O | |
| 776 | $-H$ | $-CH_3$ | $-H$ | $-H$ | $-H$ | 1 | O | |
| 777 | $-H$ | $-H$ | $-CH_3$ | $-H$ | $-H$ | 1 | O | |
| 778 | $-H$ | $-H$ | $-H$ | $-CH_3$ | $-H$ | 1 | O | |
| 779 | $-H$ | $-H$ | $-C_2H_5$ | $-H$ | $-H$ | 1 | O | |
| 780 | $-CH_3$ | $-H$ | $-H$ | $-H$ | $-H$ | 2 | O | |
| 781 | $-H$ | $-H$ | $-CH_3$ | $-H$ | $-H$ | 2 | O | |
| 782 | $-H$ | $-H$ | $-H$ | $-CH_3$ | $-H$ | 2 | O | A single bond |
| 783 | $-H$ | $-H$ | $-C_2H_5$ | $-H$ | $-H$ | 2 | O | |
| 784 | $-H$ | $-H$ | $-H$ | $-H$ | —— | 0 | S | |
| 785 | $-H$ | $-H$ | $-H$ | $-H$ | $-H$ | 1 | S | |
| 786 | $-H$ | $-H$ | $-H$ | $-H$ | $-H$ | 2 | S | |
| 787 | $-H$ | $-H$ | $-H$ | $-H$ | $-H$ | 3 | S | |
| 788 | $-CH_3$ | $-H$ | $-H$ | $-H$ | $-H$ | 1 | S | |
| 789 | $-H$ | $-CH_3$ | $-H$ | $-H$ | $-H$ | 1 | S | |
| 790 | $-H$ | $-H$ | $-CH_3$ | $-H$ | $-H$ | 1 | S | |
| 791 | $-H$ | $-H$ | $-H$ | $-CH_3$ | $-H$ | 1 | S | |
| 792 | $-H$ | $-H$ | $-C_2H_5$ | $-H$ | $-H$ | 1 | S | |
| 793 | $-CH_3$ | $-H$ | $-H$ | $-H$ | $-H$ | 2 | S | |
| 794 | $-H$ | $-H$ | $-CH_3$ | $-H$ | $-H$ | 2 | S | |
| 795 | $-H$ | $-H$ | $-H$ | $-CH_3$ | $-H$ | 2 | S | |
| 796 | $-H$ | $-H$ | $-C_2H_5$ | $-H$ | $-H$ | 2 | S | |

Table-4 (continued)

| Compound No. | $R^1$ | $R^2$ | $R^3$ | $R^4$ | $R^5$ | n | X | 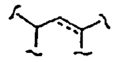 |
|---|---|---|---|---|---|---|---|---|
| 797 | -H | -H | -H | -H | — | 0 | O | A double bond |
| 798 | -H | -H | -H | -H | -H | 1 | O | |
| 799 | -H | -H | -H | -H | -H | 2 | O | |
| 800 | -H | -H | -H | -H | -H | 3 | O | |
| 801 | $-CH_3$ | -H | -H | -H | -H | 1 | O | |
| 802 | -H | $-CH_3$ | -H | -H | -H | 1 | O | |
| 803 | -H | -H | $-CH_3$ | -H | -H | 1 | O | |
| 804 | -H | -H | -H | $-CH_3$ | -H | 1 | O | |
| 805 | -H | -H | $-C_2H_5$ | -H | -H | 1 | O | |
| 806 | $-CH_3$ | -H | -H | -H | -H | 2 | O | |
| 807 | -H | -H | $-CH_3$ | -H | -H | 2 | O | |
| 808 | -H | -H | -H | $-CH_3$ | -H | 2 | O | |
| 809 | -H | -H | $-C_2H_5$ | -H | -H | 2 | O | |
| 810 | -H | -H | -H | -H | — | 0 | S | |
| 811 | -H | -H | -H | -H | -H | 1 | S | |
| 812 | -H | -H | -H | -H | -H | 2 | S | |
| 813 | -H | -H | -H | -H | -H | 3 | S | |
| 814 | $-CH_3$ | -H | -H | -H | -H | 1 | S | |
| 815 | -H | $-CH_3$ | -H | -H | -H | 1 | S | |
| 816 | -H | -H | $-CH_3$ | -H | -H | 1 | S | |
| 817 | -H | -H | -H | $-CH_3$ | -H | 1 | S | |
| 818 | -H | -H | $-C_2H_5$ | -H | -H | 1 | S | |
| 819 | $-CH_3$ | -H | -H | -H | -H | 2 | S | |
| 820 | -H | -H | $-CH_3$ | -H | -H | 2 | S | |
| 821 | -H | -H | -H | $-CH_3$ | -H | 2 | S | |
| 822 | -H | -H | $-C_2H_5$ | -H | -H | 2 | S | |

(2) The method of preparing the compounds of the present invention

The method of preparing the compounds of the present invention is explained for three cases classified depending on the kinds of the group represented by a ring A.

(1) The case wherein the ring A is 2,4-thiazolidinedione

The compound represented by the above formula (I) wherein the ring A is 2,4-thiazolidinedione can be prepared with the following five kinds of synthetic methods.

(Synthetic method-1)

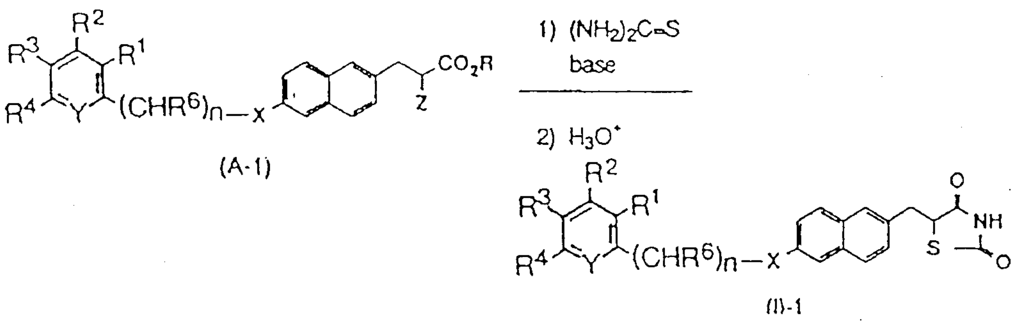


(A-1)

(I)-1

In the above formulae, X, Y, n, $R^1$, $R^2$, $R^3$, $R^4$ and $R^6$ are as defined above; Z represents halogen atom such as fluorine, chlorine, bromine, iodine or the like; and R represents a lower alkyl such as methyl, ethyl or the like.

In the reaction of the conversion of compound (A-1) into compound (I)-1, compound (A-1) is first reacted with thiourea in the presence of a base to form a 2-imino-4-thiazolidinone ring. At this time, sodium acetate, potassium acetate, sodium carbonate, potassium carbonate or the like can be used as a base, and an alcohol such as methanol, ethanol, propanol, methoxyethanol, ethoxyethanol or the like, dimethylsulfoxide (DMSO), dimethylformamide (DMF) or the like can be used as a solvent. Then, the 2-imino-4-thiazolidinone ring may be converted to a 4-thiazolidinedione ring by hydrolysis under acidic conditions to obtain compound (I)-1.

(Synthetic method-2)

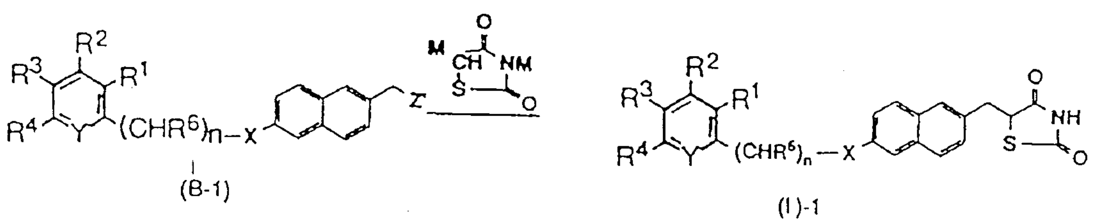

(B-1)

(I)-1

In the above formulae, X, Y, n, $R^1$, $R^2$ $R^3$, $R^4$ and $R^6$ are as defined above; Z represents a leaving group such as chlorine, bromine, iodine, $OSO_2CH_3$, $OSO_2C_6H_5(P\text{-}CH_3)$ or the like; and M represents a metal such as Li, Na, K, Mg or the like.

The reaction of the conversion of compound (B-1) to compound (I)-1 is carried out by reacting the former compound with a metal salt of a dianion of 2,4-thiazolidinone. As a metal salt, a salt of an alkali metal such as lithium, sodium, potassium or the like, or an alkaline earth-metal such as magnesium or the like can be used. The solvents to be used include an inert solvent such as diethyl ether, tetrahydrofuran (THF), dioxane, dimethoxymethane or the like.

(Synthetic method-3)

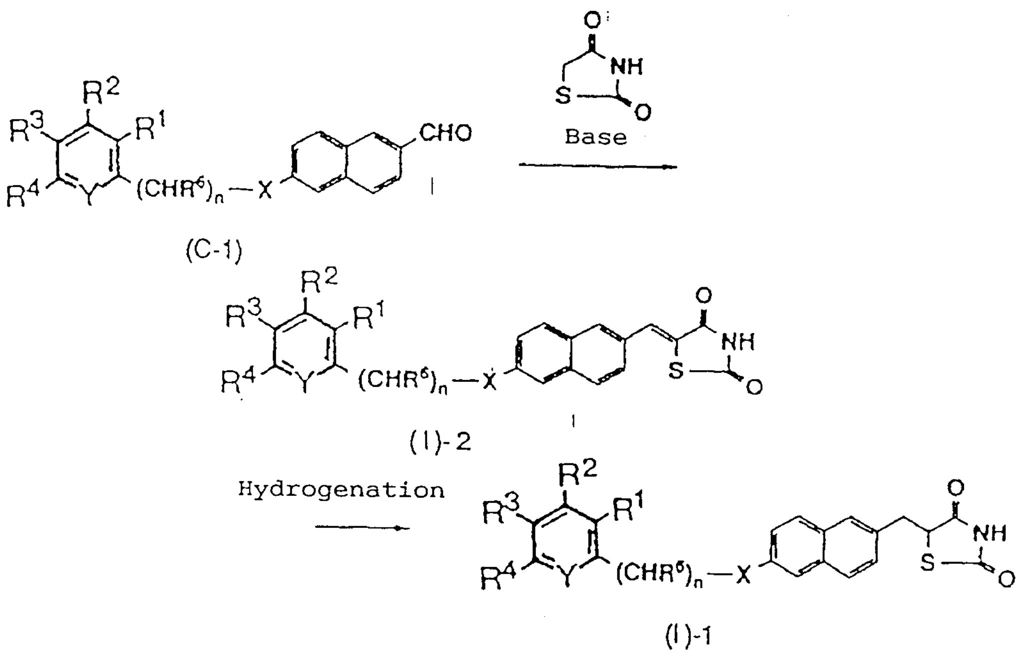


(C-1)

(I)-2

(I)-1

In the above formulae, X, Y, n, $R^1$, $R^2$, $R^3$, $R^4$ and $R^6$ are as defined above.

Compound (I)-2 can be obtained by condensing compound (C-1) with 2,4-thiazolidinone in the presence of a base under dehydration. In this case, as a base, an inorganic base such as sodium hydroxide, potassium hydroxide, sodium carbonate, potassium carbonate, sodium acetate, potassium acetate or the like, or an amine such as triethylamine, pyridine, piperidine, pyrrolidine, N-methylpiperidine, N-methylmorpholine or the like can be used, and as a solvent, an alcohol such as methanol, ethanol, 1-propanol, 2-propanol or the like can be used. Sometimes the reaction can be conducted without a solvent.

Compound (I)-1 can be synthesized by catalytically hydrogenating compound (I)-2 under hydrogen or in the presence of cyclohexene using as a catalyst a transition metal catalyst such as palladium, platinum, rhodium or the like, or a carrier holding it. At that time, as a solvent, an alcohol such as methanol, ethanol, 1-propanol, 2-propanol or the like, THF, dioxane, acetic acid or the like can be used.

(Synthetic method-4)

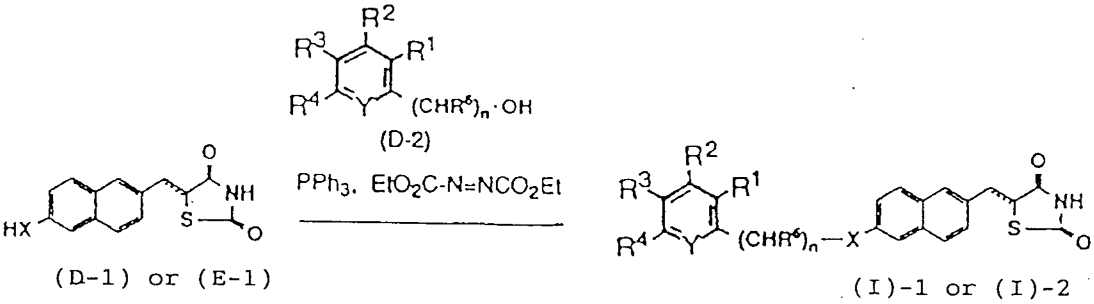


(D-2)

(D-1) or (E-1)

(I)-1 or (I)-2

In the above formulae, X, Y, $R^1$, $R^2$, $R^3$, $R^4$, $R^6$, n and dotted lines are as defined above.

Compound (I)-1 or (I)-2 wherein the dotted line in compound (I)-1 does not represent a bond and the dotted line in compound (I)-2 represents a bond can be synthesized by reacting compound (D-1) or (E-1) wherein the dotted line in compound (D-1) does not represent a bond and the dotted line in compound (E-1) represents a bond, respectively with alcohol compound (D-2) at the presence of triphenylphosphine and diethyl azodicarboxylate. At this time, as a solvent, toluene, THF, diethyl ether or dioxane can be used.

(Synthetic method-5)

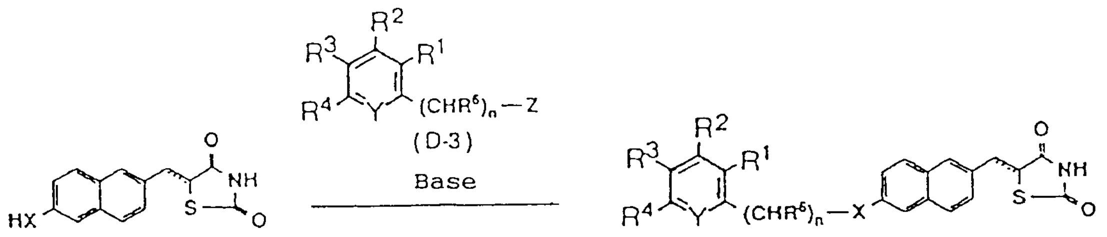

(D-3)

(D-1) or (E-1)

(I)-1 or (I)-2

In the above formulae, X, Y, $R^1$, $R^2$, $R^3$, $R^4$, $R^6$, n, Z and dotted lines are as defined above.

Compound (I)-1 or (I)-2 can be synthesized by reacting compound (D-1) or (E-1) respectively with halide (D-3) in the presence of a base. At this time, sodium hydride, potassium hydride, potassium carbonate, potassium carbonate, sodium carbonate or the like is used as a base, and THF, dioxane, diethyl ether, DMF, DMSO, N-methylpyrrolidone or the like is used as a solvent.

(2) The case wherein the ring A is rhodanine

The compound represented by the above formula (I) wherein the group A is rhodanine can be prepared by the following two kinds of synthetic methods.

(Synthetic method-6)

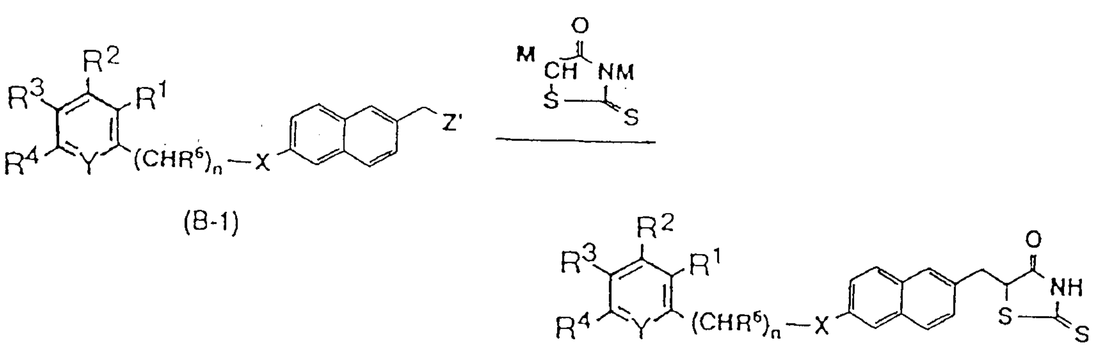
(B-1)

(I)-3

In the above formulae, X, Y, n, $R^1$, $R^2$, $R^3$, $R^4$, $R^6$, Z' and M are as defined above.

The reaction of the conversion of compound (B-1) into compound (I)-3 is performed by reacting compound (B-1) with a metal salt of a dianion of rhodanine. Solvents used include inert solvents such as diethyl ether, THF, dioxane, dimethoxymethane and the like.

(Synthetic method-7)

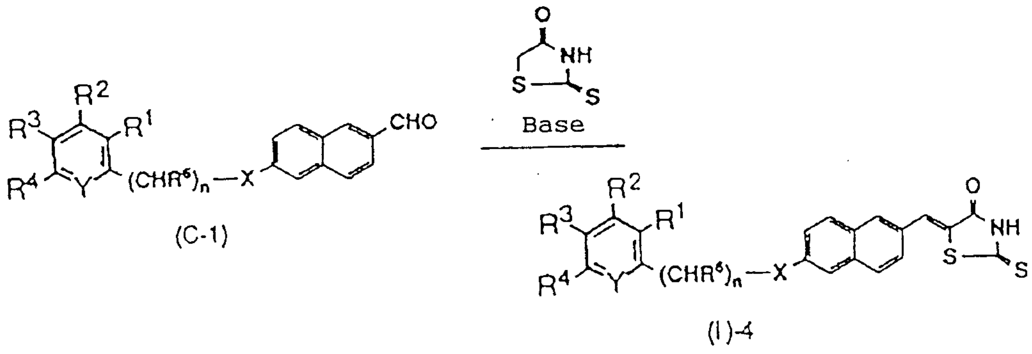


(C-1)

(I)-4

In the above formulae, X, Y, n, $R^1$, $R^2$, $R^3$, $R^4$, and $R^6$ are as defined above.

The reaction of the conversion of compound (C-1) into compound (I)-4 is carried out by condensing compound (C-1) with rhodanine with dehydrating in the presence of a base. Bases used include inorganic bases such as sodium hydroxide, potassium hydroxide, sodium carbonate, potassium carbonate, sodium acetate, potassium acetate and the like, and amines such triethylamine, pyridine, piperidine, pyrrolidine, N-methylpiperidine, N-methylmorpholine and the like. Solvents used include alcohols such as methanol, ethanol, 1-propanol, 2-propanol and the like. Sometimes the reaction can also be conducted without a solvent.

(3) The case wherein the ring A is 5-tetrazole

The compound represented by the above formula I wherein the group A is 5-tetrazole can be prepared by the following synthetic method.

(Synthetic method-8)

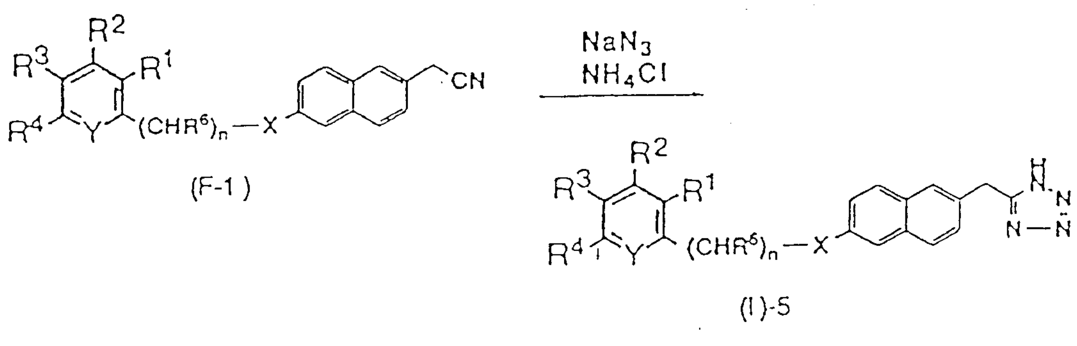


(F-1)

(I)-5

In the above formulae, X, Y, n, $R^1$, $R^2$, $R^3$, $R^4$, and $R^6$ are as defined above.

The reaction of the conversion of compound (F-1) into compound (I)-5 can be carried out by reacting compound (F-1) with sodium azide and ammonium chloride. Solvents used include DMF, DMSO, methanol, ethanol, dioxane and dimethoxymethane.

(3) Methods of preparation of starting materials and intermediates in the preparation of the compounds of the present invention

The starting material (A-1) in Synthetic method-1 described above can be prepared for example by the following synthetic method.

(Synthetic method of starting materials-1)

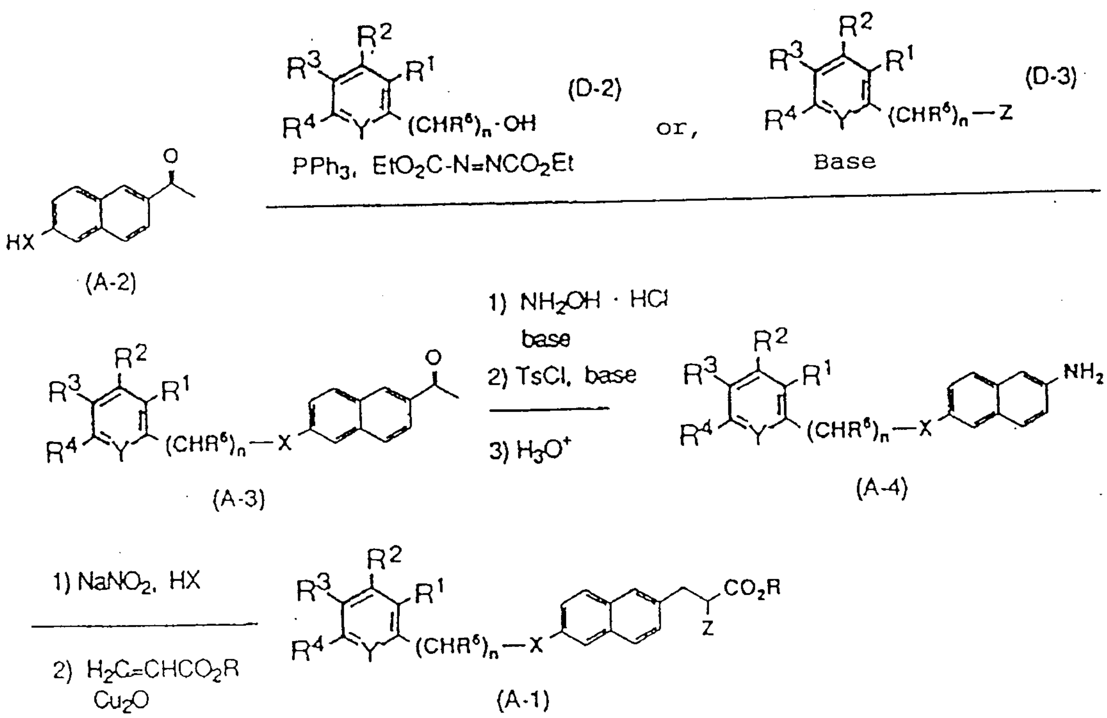


In the above formulae, X, Y, n, $R^1$, $R^2$, $R^3$, $R^4$, $R^6$, Z and R are as defined above.

The reaction of the conversion of compound (A-2) into compound (A-3) is carried out by reacting compound (A-2) with alcohol (D-2) in the presence of triphenylphosphine and diethyl azodicarboxylate. At this time, as a solvent, toluene, THF, diethyl ether, dioxane or the like is used. Compound (A-3) can be also synthesized by reacting compound (A-2) with halide (D-3) in the presence of a base.

Bases to be used include sodium hydride, potassium hydride, potassium carbonate and sodium carbonate. When n=0, a transition metal such as palladium, copper or the like is added occasionally as a catalyst, and THF, dioxane, diethyl ether, DMF, DMSO, N-methylpyrrolidone or the like is used as a solvent.

The reaction of the conversion of compound (A-3) into compound (A-4) is first started by converting the acetyl group of compound (A-3) into an oxime group using hydroxylamine hydrochloride and a base. At this time, sodium carbonate, potassium carbonate, sodium acetate, potassium acetate, sodium methoxide, sodium ethoxide or the like is used as a base and water, methanol, ethanol, acetone, a mixture thereof or the like is used as a solvent. Subsequently, the oxime group is reacted with p-toluenesulfonyl chloride in the presence of a base to convert into an aminoacetyl group by the Beckmann rearrangement. At this time, a tertiary amine such as pyridine, triethylamine or the like is used as a base. Dichloromethane, dichloroethane or the like is used as a solvent. Next, the amineacetyl group is hydrolyzed under acidic conditions to be converted into an amino group.

The reaction of the conversion of compound (A-4) into compound (A-1) is carried out by reacting the amino group of compound (A-4) with sodium nitrite in the presence of aqueous solution of hydrogen chloride, hydrogen bromide or hydrogen iodide to form a diazonium salt followed by reacting the diazonium salt with an acrylate ester in the presence of cuprous oxide catalyst. At this time, water or a mixture of water and acetone is used as a solvent.

Starting materials (B-1), (C-1) and (F-1) in Synthetic methods 2, 3, 6, 7 and 8 described above can be prepared by for example the following synthetic methods.

(Synthetic method of starting materials-2)

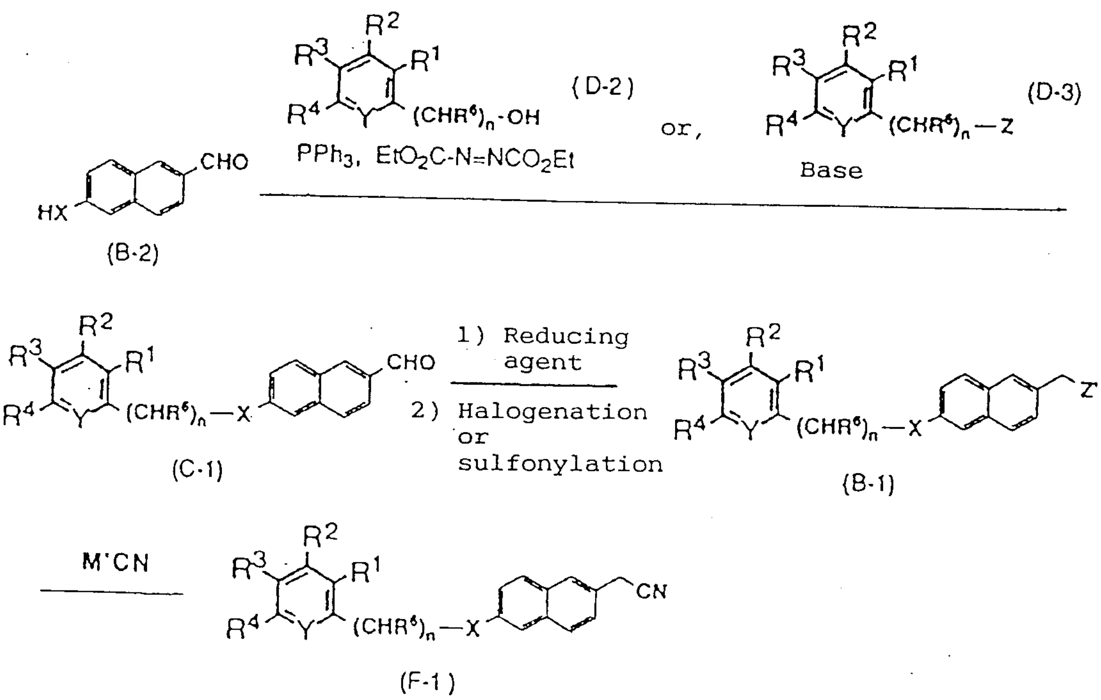


(B-2) (D-2) (D-3) (C-1) (B-1) (F-1)

In the above formulae, X, Y, n, $R^1$, $R^2$, $R^3$, $R^4$, $R^6$, Z and Z' are as defined above and M' represents a metal such as sodium, potassium or the like.

The reaction of the conversion of compound (B-2) into compound (C-1) is performed by reacting compound (B-2) with alcohol (D-2) in the presence of triphenylphosphine and diethyl azodicarboxylate. At this time, toluene, THF, diethyl ether, dioxane or the like is used as a solvent. Compound (C-1) can be also obtained by reacting compound (B-2) with halide (D-3) in the presence of a base. At this time, sodium hydride, potassium hydride, potassium carbonate, sodium carbonate or the like is used as the base, and when n=0 a transition metal such as palladium, copper or the like is added as a catalyst sometimes. THF, dioxane, diethyl ether, DMF, DMSO, N-methylpyrrolidone or the like is used as a solvent.

In the reaction of the conversion of compound (C-1) into compound (B-1), the formyl group in compound (C-1) is first converted into a hydroxyl group using a reducing agent. At this time, sodium borohydride, lithium aluminium hydride, diisobutylalminium hydride or the like is used as a reducing agent. An inert solvent such as diethyl ether, THF, dioxane, dimethoxymethane, toluene or the like is used as a solvent, and as the case may be an alcohol such as ethanol, methanol, 1-propanol, 2-propanol or the like is used.

Next the above hydroxyl group is halogenated using a suitable halogenating agent for example, thionyl halide such as thionyl chloride, thionyl bromide or the like, phosphorus oxychloride, a halogenated hydroacid such as hydrobromicacid or the like, carbon tetrachloride, carbontetrabromide, bromine, iodine or the like: or sulfonated using a suitable sulfonating agent for example, sulfonyl chloride such as methanesulfonyl chloride, p-toluenesulfonyl chloride or the like, methanesulfonic anhydride, p-toluenesulfonic anhydride, trifluoromethanesulfonic anhydride or the like to obtain compound (B-1).

The reaction of the conversion of compound (B-1) into compound (F-1) can be performed by reacting compound (B-1) with sodium cyanide or potassium cyanide. At this time, DMF, DMSO, methanol, ethanol, dioxane, dimethoxymethane or the like is used as a solvent.

Starting materials (D-1), or (E-1) in Synthetic methods- 4 and 5 described above can be prepared by for example the following synthetic methods.

(Synthetic method of starting materials-3)

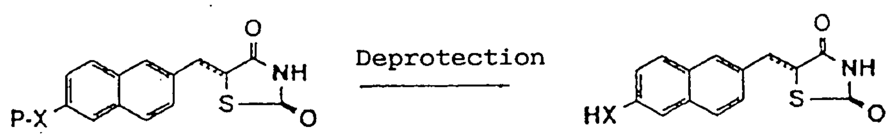


(D-4) or (E-2) (D-1) or (E-1)

In the above formulae, X and the dotted lines are as defined above, and P represents a protecting group such as methoxymethyl, ethoxymethyl, 1-(1-ethoxy)-ethyl, 2-tetrapyranyl, trimethylsilyl, t-butyldimethylsilyl, trityl or the like.

Compounds (D-1) and (E-1) can be synthesized by deprotecting compounds (D-4) and (E-2) respectively wherein the dotted line in compound (D-4) does not represent a bond and the dotted line in compound (E-2) represents a bond under acidic conditions or in the presence of fluoride anions. At this time, methanol, ethanol, acetone, THF, dioxane, DMF, DMSO or a mixture of these solvents and water is used as a solvent.

Compound (E-2) can be also prepared by for example the following synthetic method.

(Synthetic method of intermediates-1)

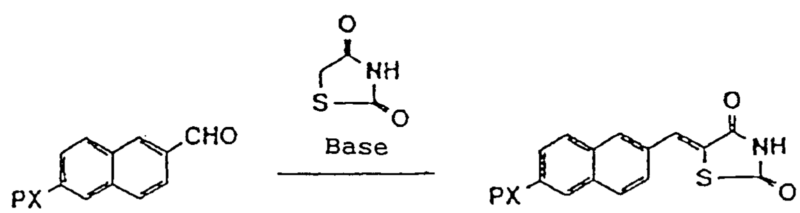


(E-3) (E-2 )

The reaction of the conversion of compound (E-3) into compound (E-2) is carried out by condensing compound (E-3) with 2,4-thiazolidinedione in the presence of a base under dehydration. At this time, bases to be used include inorganic bases such as sodium hydroxide, potassium hydroxide, sodium carbonate, potassium carbonate, sodium acetate, potassium acetate and the like and amines such as triethylamine, pyridine, piperidine, pyrrolidine, N-methylpiperidine, N-methylmorpholine and the like. Solvents used include alcohols such as methanol, ethanol, 1-propanol, 2-propanol and the like and sometimes the reaction can be also performed without solvent.

Compound (D-4) can be also prepared for example by two following methods.

(Synthetic method of intermediates-2)

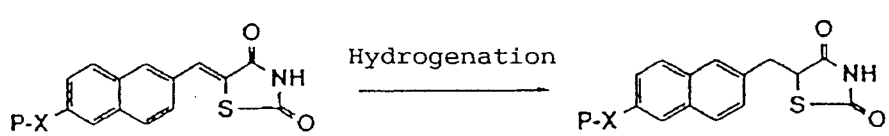


(E-2 ) (D-4)

In the above formulae, X and P are as defined above.

The reaction of the conversion of compound (E-2) into compound (D-4) can be carried out by catalytically hydrogenating compound (E-2) with a transition metal catalyst such as palladium, platinum, rhodium or the like, or a catalyst which carrys said metal, under hydrogen or in the presence of cyclohexene. At this time, an alcohol such as methanol, ethanol, 1-propanol, 2-propanol or the like, THF, dioxane, acetic acid or the like is used as a solvent.

(Synthetic method of intermediates-3)

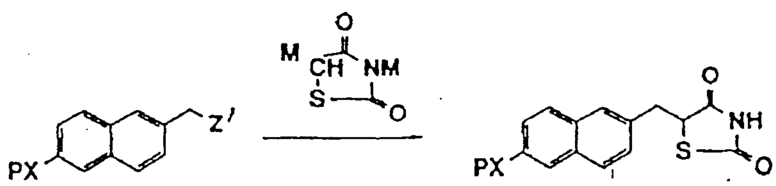

(D-5) (D-4)

In the above formulae, X, P, Z' and M are as defined above.

The reaction of the conversion of compound (D-5) into compound (D-4) is conducted by reacting compound (D-5) with a metal salt of a dianion of 2,4-thiazolidinedione. Metal salts used include those of alkali metals such as lithium, sodium, potassium and the like and alkaline earth metals such as magnesium and the like. Solvents used include inert solvents such as diethyl ether, THF, dioxane, dimethoxymethane and the like.

⟨4⟩Use of the compounds of the present invention

The compounds of the present invention have excellent effects on reduction of blood sugar and blood lipid levels and can be used as medicaments. They can be formulated to various preparations suitable for various administration routes, using conventional carriers. For example, for oral administration, they are formulated in the form of tablet, capsule, granule, powder, liquid preparation and the like. Conventional excipients, binders, lubricants, coloring matters, disintegrators and the like can be used upon preparing solid preparations for oral administration.

Excipients include, for example, lactose, starch, talc, magnesium stearate, microcrystalline cellulose, methyl cellulose, carboxymethyl cellulose, glycerol, sodium alginate and arabic gum. Binders used include polyvinyl alcohol, polyvinylether, ethyl cellulose, arabic gum, shellac and sucrose, and lubricants used include magnesium stearate, and talc. Further, coloring materials and disintegrators known in the art can be used. Tablets may be coated by well known methods.

Liquid preparations may be aqueous or oily suspension, solution, syrup, elixir and the like, and they can be prepared by conventional methods. When injectable preparations are formulated, to the compounds of the present invention are added pH regulating agent, buffering agent, stabilizing agent, isotonicity, local anesthetic and the like and then preparations for subcutaneous, intramuscular or intravenous injections can be made by conventional methods. When suppository is made, oily bases such as cacao butter, polyethylene glycols, Witepsol® (Dynamite Nobel Company) and the like may be used as base.

The dosage of such preparations is varied depending upon the condition, body weight, age, etc. of the patient and is not the same for all the patients. Preferably it is set such that the dosage of the compounds of the present invention is in the range of about 0.01 to 2000 mg/day per adult patient. The preparation is preferably divided and administered from one to four times per day.

<u>Example</u>

The present invention will be more specifically explained by the following Preparations, Examples and Experiments. However, the present invention is not limited to such Preparation, Examples and Experiments in any aspects.

Preparation 1

Synthesis of 6-(2-pyridyl)-methyloxy-2-acetylnaphthalene

To a solution of 6-hydroxy-2-acetylnaphthalene (1.04 g) in DMF (20 ml) were added sodium hydride (60%, 0.65 g) and 2-picolyl chloride hydrochloride (1.28 g) under ice-cooling and the resultant-mixture was stirred at room temperature for 12 hours. The reaction mixture was partitioned between toluene and water. The organic layer was washed with a saturated saline solution and dried over anhydrous magnesium sulfate. After concentration in vacuo, the residue was subjected to column chromatography on silica gel eluting with ethyl acetate/hexane to obtain the title compound (1.17 g, yield =75.5%). The NMR spectrum is as follows.

NMR ($CDCl_3$);
2.70 (s, 3H),
5.35 (s, 2H),
7.23-7.27 (m, 2H),
7.33 (dd, 1H, J=2.6Hz, 9.1Hz),
7.56 (d, 1H, J=7.8Hz),
7.72 (dd, 1H, J=1.9Hz, 7.6Hz),
7.77 (d, 1H, J=1.4Hz),
7.89 (d, 1H, J=8.9Hz),
8.00 (dd, 1H, J=1.8Hz, 8.7Hz),
8.41 (s, 1H),
8.65 (dd, 1H, J=0.9Hz, 6.0Hz)

Preparation 2

Synthesis of 6-(2-pyridyl)-methyloxy-2-(1-hydroxyiminoethyl)-naphthalene

To a solution of 6-(2-pyridyl)-methoxy-2-acetylnaphthalene (1.17 g) in methanol (50 ml) was added a solution of hydroxylamine hydrochloride (0.59 g) and potassium carbonate (1.17 g) in water (10 ml) and the resultant-mixture was heated under reflux with stirring for 3 hours.

After cooling to room temperature, water (50 ml) was added to the mixture. The precipitated solid was filtered off and dried in vacuo with heating to obtain the title compound (1.22 g). The NMR spectrum is as follows.

NMR (DMSO d-6);
2.24 (s, 3H),
5.30 (s, 2H),
7.28 (dd, 1H, J=2.5Hz, 9.0Hz),
7.37 (dd, 1H, J=1.8HZ, 6.8Hz),
7.42 (d, 1H, J=2.5Hz),
7.58 (d, 1H, J=7.8Hz),
7.74 (d, 1H, J=8.8Hz),
7.81-7.93 (m, 3H),
8.05 (s, 1H),
8.59 (dd, 1H, J=0.8Hz, 4.8Hz),
11.2 (s, 1H)

Preparation 3

Synthesis of 2-acetylamino-6-(2-pyridylmethyloxy)-naphthalene

To a solution of 2-(2-pyridylmethyloxy)-6-(1-hydroxyiminoethyl)-naphthalene (1.23 g) in pyridine (15 ml) was added p-toluenesulfonyl chloride (1.45 g) and the resultant-mixture was stirred at room temperature for 24 hours. The reaction mixture was made acid with hydrochloric acid and extracted with ethyl acetate. The organic layer was washed with an aqueous solution of sodium hydroxide and a saturated saline solution, dried over anhydrous magnesium sulfate, and concentrated in vacuo to obtain a residue. The residue was subjected to column chromatography on silica gel eluting with $CHCl_3$/MeOH to obtain the title compound (0.76 g, yield=62%). The NMR spectrum is as follows.

$^1$H-NMR (DMSO d-6);
2.07 (s, 3H),
5.26 (s, 2H),
7.22 (dd, 1H, J=2.5Hz, 9.0Hz),

7.32-7.36 (m, 2H),
7.50-7.57 (m, 2H),
7.69-7.76 (m, 2H),
7.81 (dt, 1H, J=1.5Hz, 7.5Hz),
8.20 (s, 1H),
8.59 (dd, 1H, J=0.5Hz, 3.8Hz),
10.03 (s, 1H)

Preparation 4

Synthesis of 2-amino-6-(2-pyridylmethyloxy)naphthalene

To a solution of 2-acetylamino-6-(2-pyridylmethyloxy)-naphthalene (0.76 g) in 2-methoxyethanol (15 ml) was added 1N-hydrochloric acid (15 ml) and the resulting mixture was stirred with heating under reflux for 3 hours. After completion of the reaction, the reaction mixture was cooled to room temperature, made basic with an aqueous solution of sodium hydroxide, and then extracted with ethyl acetate, The organic layer was washed with a saturated saline solution, dried over anhydrous magnesium sulfate, and concentrated in vacuo to obtain the title compound (0.65 g) as the crude product. The NMR spectrum is as follows.

$^1$H-NMR ($CDCl_3$);
5.28 (s, 2H),
6.90-6.97 (m, 2H),
7.10 (d, 1H, 2.5 Hz),
7.16-7.25 (m, 2H),
7.52-7.58 (m, 3H),
7.71 (dt, 1H, J=1.8Hz, 7.8Hz),
8.61 (dd, 1H, J=0.5Hz, 3.8Hz)

Preparation 5

Synthesis of methyl 3-[6-(2-methylpyridyloxy)naphthyl]-methyl-2-chloro-propionate

To a solution of 2-amino-6-(2-pyridylmethyloxy) naphthalene (0.65 g) in acetone (10 ml) were added concentrated hydrochloric acid (0.65 ml) and a solution of sodium nitrite (0.22 g) in water (1 ml). The resultant mixture was stirred under ice-cooling for 30 minutes. Methyl acrylate (1.4 ml) and cuprous oxide were then added to the mixture, and the latter was vigorously stirred for about 3 hours. After reaction, the reaction mixture was made basic with an aqueous solution of sodium hydroxide and extracted with ethyl acetate. The organic layer was washed with a saturated saline solution, dried over anhydrous magnesium sulfate, and concentrated in vacuo to obtain a residue.

The resulting residue was subjected to column chromatography on silica gel eluting with chloroform/methanol to obtain the title compound (0.22 g, yield=24%). The NMR spectrum is as follows.

NMR ($CDCl_3$);
3.29 (dd, 1H, J=7.5Hz, 14.0Hz),
3.53 (dd, 1H, J=7.5Hz, 14.0Hz),
3.73 (s, 3H),
4.52 (t, 1H, J=7.4Hz),
5.32 (s, 2H),
7.18-7.31 (m, 4H),
7.54-7.75 (m, 5H),
8.62 (dd, 1H, J=0.5Hz, 3.8Hz)

Example 1

Synthesis of 5-[6-(2-pyridylmethyloxy)-2-naphthyl]-methyl-thiazolidine-2,4-dione (compound No. 772 in Table-4)

To a solution of methyl 3-[6-(2-pyridylmethyloxy)-naphthyl]-methyl-2-chloro-propionate (0.22 g) in 2-methoxyethanol (5 ml) were added thiourea (95 mg) and sodium acetate (76 mg) and the resultant mixture was stirred at 80°C for 3 hours. After it had been confirmed by TLC that the starting material had disappeared, 1N hydrochloric acid (2.5 ml) was added to the mixture and it was stirred with heating under reflux for 6 hours.

After reaction, the mixture was cooled to room temperature, made basic with an aqueous solution of sodium hydroxide and extracted with ethyl acetate. The organic layer was washed with a saturated saline solution, dried over

anhydrous magnesium sulfate and concentrated in vacuo to obtain a residue. The resulting residue was subjected to column chromatography on silica gel eluting with chloroform/methanol to obtain an amorphous solid. The solid was recrystallized from ethyl acetate to obtain the title compound (131 mg, yield =58%). The NMR spectrum, IR spectrum and melting point are as follows.

NMR (DMSO d-6);
3.21 (dd, 1H, J=4.6Hz, 12.5Hz),
3.51 (dd, 1H, J=4.6Hz, 12.5Hz),
4.95 (dd, 1H, J=4.1Hz, 8.5Hz),
5.28 (s, 2H),
7.26 (dd, 1H, J=2.5Hz, 9.0Hz),
7.33-7.39 (m, 3H),
7.56 (d, 1H, J=7.9Hz),
7.67 (s, 1H),
7.72-7.87 (m, 3H),
8.59 (dd, 1H, J=0.5Hz, 3.8Hz),
12.02 (s, 1H)
IR (KBγ);
3054, 2796, 1742, 1703, 1601, 1483, 1437, 1395, 1312, 1267, 1229 $cm^{-1}$
m.p.; 225 - 227°C

The compounds of Examples 2 and 3 were obtained with the method similar to that in Example 1.

The spectral data and yield of such products are described in Table 5.

Preparation 6

Synthesis of 6-(2-fluorobenzyloxy)-2-naphthylmethyl alcohol

6-(2-fluorobenzyloxy)-2-naphthylaldehyde (1.07 g) was dissolved in a mixed solvent of ethanol/THF (1:1) (22 ml). Sodium borohydride (144 mg) was added to the solution and it was stirred at room temperature for 1 hour.

After reaction, 1N hydrochloric acid was added to the above mixture, and the resultant mixture was extracted with chloroform. The organic layer was washed with a saturated saline solution, dried over anhydrous magnesium sulfate and concentrated in vacuo to obtain the title compound (1.07 g) as the crude product. The product was used in the next reaction without purification. The NMR spectrum is as follows.

NMR ($CDCl_3$);
4.82 (s, 2H),
5.25 (s, 2H),
7.08-7.35 (m, 5H),
7.45 (dd, 1H, J=1.5Hz, 8.4Hz),
7.56 (dt, 1H, J=1.5Hz, 7.4Hz),
7.73-7.77 (m, 3H)

Preparation 7

Synthesis of 6-(2-fluorobenzyloxy)-2-naphthylmethyl iodide

To a solution of 6-(2-fluorobenzyloxy)-2-naphthyl-methylalcohol (1.07 g) in THF (20 ml) were added triphenylphosphine (1.51 g) and imidazole (0.39 g), and a solution of iodine (1.21 g) in THF (10 ml) was gradually and dropwise added thereto under ice-cooling. Further the resulting mixture was stirred under ice-cooling for 30 minutes.

After reaction, ethyl acetate was added to the above mixture. The resulting mixture was washed with an aqueous solution of sodium hydrogenthiosulfate and a saturated saline solution, dried over anhydrous magnesium sulfate, and concentrated in vacuo to obtain a residue. The residue was subjected to column chromatography on silica gel eluting with ethyl acetate/hexane to obtain the title compound (0.24 g, yield=16%).

Example 4

Synthesis of 5-[6-(2-fluorobenzyloxy)-2-naphthyl]-methyl-thiazolidine-2,4-dione (Compound No. 5 in Table-1)

To a solution of 2,4-thiazolidinedione (108 mg) in THF (5 ml) was added hexamethylphosphoric triamide (0.5 ml) and the resulting mixture was cooled to -30°C, and n-butyllithium (1.6M, a solution in hexane) (1.1 ml) was added

thereto. The mixture was stirred at -30°C for 30 minutes and a solution of 6-(2-fluorobenzyloxy)-2-naphthylmethyl iodide (0.24 g) in THF (3 ml) was added. The resulting mixture was gradually warmed from -30°C to room temperature and stirred for 6 hours. After reaction, ethyl acetate was added to the above reaction mixture. The organic layer was washed with an aqueous solution of ammonium chloride and a saturated saline solution, dried over anhydrous magnesium sulfate, and concentrated in vacuo to obtain a residue. The residue was subjected to column chromatography on silica gel eluting with ethyl acetate/hexane to obtain an amorphous solid. The solid was recrystallized from ethyl acetate/hexane to obtain the title compound (152 mg, yield=65%). The NMR spectrum, IR spectrum and melting point are as follows.

NMR (DMSO d-6);
3.23 (dd, 1H, J=9.5Hz, 14.0Hz),
3.51 (dd, 1H, J=4.3Hz, 14.0Hz),
4.99 (dd, 1H, J=4.3Hz, 9.5Hz),
5.24 (s, 2H),
7.20-7.30 (m, 3H),
7.38 (t, 1H, J=8.8Hz),
7.45 (s, 1H),
7.61 (t, 1H, J=7.5Hz),
7.70 (s, 1H),
7.76 (d, 1H, J=5.8Hz),
7.79 (d, 1H, J=6.0Hz),
12.03 (s, 1H)
IR (KBγ);
3254, 3055, 1759, 1674, 1607, 1493, 1393, 1325, 1269, 1231

Compounds of Examples 5, 6, 7, 8, 9, 10 and 11 were obtained by a method similar to that described in Example 4. These compounds are represented by the following formula (I-e).

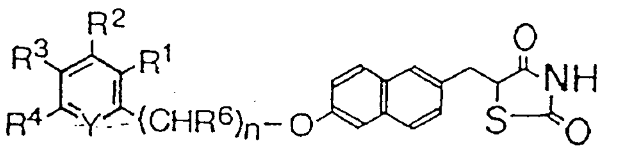

(I-e)

The spectral data and yield values of the above compounds are described in Table 5 together with those of the compounds obtained in Examples 2 and 3.

Table 5

| Example No. | 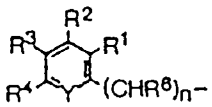 | mp (℃)<br>Yield (%) | NMR (ppm) | IR (cm$^{-1}$) |
|---|---|---|---|---|
| 2 | 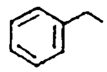 | 181 ~ 183<br>58 | 3.23 (dd, 1H, J=9.4Hz, 14.2Hz)<br>3.51 (dd, 1H, J=4.4Hz, 14.1Hz)<br>4.99 (dd, 1H, J=4.4Hz, 9.4Hz)<br>5.20 (s, 2H)<br>7.22 (dd, 1H, J=2.5Hz, 8.9Hz)<br>7.33-7.52 (m, 8H)<br>7.67 (s, 1H), 7.70 (d, 1H, J=9.5Hz)<br>7.78 (d, 1H, J=9.5Hz), 12.41 (s, 1H) | 3260, 3063<br>1759, 1691<br>1606, 1504<br>1454, 1391<br>1336, 1263<br>1231 |
| 3 | 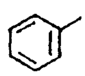 | 137 ~ 138<br>32 | 3.24 (dd, 1H, J=4.8Hz, 14.2Hz)<br>3.53 (dd, 1H, J=4.1Hz, 13.9Hz)<br>5.01 (dd, 1H, J=4.5Hz, 9.0Hz)<br>7.08 (dd, 2H, J=0.8Hz, 7.7Hz)<br>7.17 (t, 1H, J=7.3Hz)<br>7.27 (dd, 1H, J=2.3Hz, 8.9Hz)<br>7.30-7.50 (m, 4H)<br>7.77 (d, 2H, J=8.8Hz)<br>7.91 (d, 1H, J=8.9Hz) | 3431, 3260<br>3059, 1745<br>1685, 1591<br>1491, 1475<br>1323, 1228<br>1143 |
| 5 | 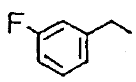 | 156 ~ 159<br>83 | 3.23 (dd, 1H, J=9.3Hz, 14.1Hz)<br>3.51 (dd, 1H, J=4.3Hz, 14.1Hz)<br>4.98 (dd, 1H, J=4.3Hz, 9.3Hz)<br>5.23 (s, 2H), 7.16-7.46 (m, 7H)<br>7.68 (s, 1H), 7.74 (d, 1H, J=8.5Hz)<br>7.79 (d, 1H, J=9.1Hz), 12.03 (s, 1H) | 3179, 3057<br>1755, 1692<br>1607, 1487<br>1460, 1381<br>1335, 1262<br>1233 |
| 6 | 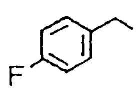 | 151 ~ 153<br>76 | 3.23 (dd, 1H, J=9.4Hz, 14.2Hz)<br>3.51 (dd, 1H, J=4.3Hz, 14.2Hz)<br>4.98 (dd, 1H, J=4.3Hz, 9.4Hz)<br>5.18 (s, 2H), 7.19-7.58 (m, 5H)<br>7.70 (d, 1H, J=13.7Hz)<br>7.78 (d, 1H, J=9.4Hz) | 3256, 3061<br>1763, 1691<br>1607, 1512<br>1391, 1333<br>1271, 1231 |

Table 5 (continued)

| Example No. | 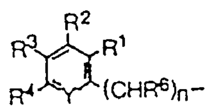 | mp (℃) / Yield (%) | NMR (ppm) | IR ($cm^{-1}$) |
|---|---|---|---|---|
| 7 | 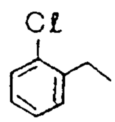 | 171 ~ 173 / 23 | 3.23 (dd, 1H, J=9.0Hz, 14.6Hz)<br>3.50 (dd, 1H, J=4.2Hz, 14.6Hz)<br>4.99 (dd, 1H, J=4.2Hz, 9.0Hz)<br>5.22 (s, 2H)<br>7.21-7.48 (m, 5H)<br>7.63-7.82 (m, 5H) | 3204, 3063<br>1757, 1682<br>1605, 1395<br>1335, 1263<br>1233, 1155 |
| 8 | 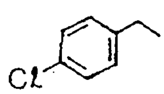 | 150 ~ 152 / 23 | 3.21 (dd, 1H, J=9.3Hz, 18.0Hz)<br>3.51 (dd, 1H, J=4.3Hz, 18.0Hz)<br>4.98 (dd, 1H, J=4.3Hz, 9.3Hz)<br>5.20 (s, 2H)<br>7.22 (dd, 1H, J=2.3Hz, 8.8Hz)<br>7.35-7.55 (m, 5H)<br>7.67-7.77 (m, 5H) , 12.04 (s, 1H) | 3158, 3054<br>1744, 1701<br>1605, 1491<br>1393, 1337<br>1267, 1229 |
| 9 | 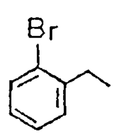 | 158 ~ 161 / 52 | 3.27 (dd, 1H, J=9.0Hz, 18.3Hz)<br>3.52 (dd, 1H, J=4.3Hz, 18.3Hz)<br>5.26 (s, 2H)<br>7.24 (d, 1H, J=9.0Hz)<br>7.35-7.43 (m, 5H)<br>7.52-7.82 (m, 4H), 12.04 (s, 1H) | 3204, 3061<br>1757, 1682<br>1604, 1393<br>1335, 1263<br>1231, 1026 |
| 10 | 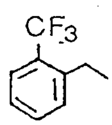 | 149 ~ 151 / 88 | 3.24 (dd, 1H, J=9.3Hz, 14.0Hz)<br>3.53 (dd, 1H, J=4.3Hz, 14.0Hz)<br>4.99 (dd, 1H, J=4.3Hz, 9.3Hz)<br>5.33 (s, 2H)<br>7.22 (dd, 1H, J=2.3Hz, 9.3Hz)<br>7.35-7.41 (m, 2H)<br>7.60 (t, 1H, J=7.8Hz)<br>7.69-7.83 (m, 6H), 12.04 (s, 1H) | 3142, 3044<br>1765, 1707<br>1607, 1452<br>1397, 1314<br>1269, 1230<br>1182 |
| 11 | 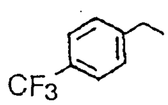 | 162 ~ 164 / 64 | 3.24 (dd, 1H, J=9.3Hz, 14.0Hz)<br>3.51 (dd, 1H, J=4.3Hz, 14.0Hz)<br>4.98 (dd, 1H, J=4.3Hz, 9.3Hz)<br>5.34 (s, 2H)<br>7.25 (dd, 1H, J=2.3Hz, 9.0Hz)<br>7.35-7.40 (m, 1H)<br>7.68-7.82 (m, 7H), 12.03 (s, 1H) | 3162, 3056<br>1753, 1699<br>1607, 1481<br>1397, 1323<br>1261, 1209 |

Preparation 8

Synthesis of 5-(6-hydroxy-2-naphthyl)-methyl-thiazolidine-2,4-dione

To a solution of 5-(t-butyldimethylsilyloxy-2-naphthyl)-methyl-thiazolidine-2,4-dione (897 mg) in DMF (7 ml) were added potassium fluoride (269 mg) and 47% hydrobromic acid (0.12 ml). The reaction mixture was stirred at room temperature for 1.5 hours, and then the reaction mixture was added to 3N hydrochloric acid (50 ml) and extracted with chloroform.

The organic layers were collected, washed with a saturated saline solution and concentrated to obtain a crude product. The product was subjected to column chromatography on silica gel eluting with chloroform/methanol to obtain the title compound (250 mg, yield=40%). The NMR spectrum is as follows.

$^1$H NMR (250MHz, DMSO);
3.20 (dd, 1H, J=9.3Hz, 14.3Hz),
3.48 (dd, 1H, J=4.3Hz, 14.0Hz),
4.97 (dd, 1H, J=4.3Hz, 9.3Hz),
7.06 (d, 1H, J=8.4Hz),
7.08 (s, 1H),
7.27 (d, 1H, J=8.5Hz),
7.60 (s, 1H),
7.62 (d, 1H, J=9.0Hz),
7.69 (d, 1H, J=9.0Hz)

Example 12

Synthesis of 5-[6-(2,4,6-trifluorobenzyloxy)-2-naphthyl]-methyl-thiazolidine-2,4-dione (compound No. 153 in Table 1)

To a suspension of sodium hydride in DMF (6 ml) which had been washed three times with hexane was added dropwise a solution of (6-hydroxy-2-naphthyl)-methylthiazolidinedione (250 mg) in DMF (1 ml) followed by 2,4,6-trifluorobenzyl bromide (149 mg). The resulting mixture was stirred at room temperature for 2 hours. The reaction mixture was added to an aqueous saturated ammonium chloride solution, and the mixture was extracted with ethyl acetate.

The resultant organic layers was washed with a saturated saline solution and concentrated to obtain a residue. The residue was subjected to column chromatography on silica gel eluting with ethyl acetate/hexane to obtain the title compound (97 mg, yield=39%). The spectral data and melting point are as follows.

$^1$H NMR ($CDCl_3$, 250Hz);
3.27 (dd, 1H, J=9.8Hz, 14.1Hz),
3.68 (dd, 1H, J=3.9Hz, 14.0Hz),
4.62 (dd, 1H, J=4.0Hz, 9.9Hz),
5.18 (s, 2H),
6.68-6.78 (m, 2H),
7.17-7.34 (m, 3H),
7.55-7.75 (m, 3H),
9.71 (s, 1H),
12.03 (s, 1H)
IR;
1688, 1667, 1630, 1606, 1227, 1122, 1017, 845 $cm^{-1}$
m.p.; 166-167°C

Preparation 9

Synthesis of 6-benzyloxy-2-naphthylaldehyde

6-benzyloxy-2-naphthylaldehyde (0.36 g) was dissolved in a mixture of THF (10 ml) and DMF (1 ml). The solution was cooled to 0°C, and 60% sodium-hydride in oil (0.23 g) was added thereto. The resulting mixture was stirred at 0°C for 30 minutes, and then benzyl bromide (1 ml) was slowly added dropwise. After addition, the resulting mixture was warmed to room temperature and stirred for 5 hours.

After reaction, methanol (0.5 ml) and water (5 ml) were poured into the reaction mixture and it was extracted three times with ethyl acetate (50 ml). The ethyl acetate layer was washed with a saturated saline solution, dried over $MgSO_4$, and the ethyl acetate was distilled off. The oily residue was subjected to column chromatography on silica gel (30 g) eluting with hexane/ethyl acetate. The resulting solution was concentrated and dried to obtain the objective title compound (220 mg, yield=40%). The NMR spectrum is as follows.

$^1$H NMR (DMSO);
5.27 (s, 2H),
7.34-7.44 (m, 5H),
7.50-7.56 (m, 3H),
7.84 (d, 1H, J=8.8Hz),
7.93 (d, 1H, J=8.3Hz),
8.08 (d, 1H, J=9.0Hz),
8.49 (s, 1H),
10.07 (s, 1H)

Preparation 10

Synthesis of 6-(2-fluorobenzyloxy)-2-naphthylaldehyde

6-hydroxy-2-naphthylaldehyde (520 mg) and triphenylphosphine (0.87 g) was dissolved in THF (20 ml), and then 2-fluorobenzyl alcohol (0.49 ml) was added thereto. The reaction mixture was stirred, and diethyl azodicarboxylate (0.57 ml) was slowly added. The mixture was stirred at room temperature for 36 hours.

After reaction, the solvent was distilled off, and the residue was subjected to column chromatography on silica gel (50 g) eluting with hexane/ethyl acetate. The combined solutions were concentrated, and dried to obtain the objective title compound (654 mg, yield=81%). The NMR spectrum is as follows.

$^1$H NMR ($CDCl_3$);
5.41 (s, 2H),
7.22 (d, 1H, J=2.5Hz),
7.32 (dd, 1H, J=9.0Hz, 2.5Hz),
7.44 (t, 1H, J=7.8Hz),
7.58 (t, 1H, J=8.3Hz),
7.72 (d, 2H, J=8.5Hz),
7.78 (d, 1H, J=8.5Hz),
7.8-8.0 (m, 2H),
8.26 (s, 1H),
10.09 (s, 1H)

Example 13

Synthesis of 5-(6-benzyloxy-2-naphthyl)-methylene-thiazolidine-2,4-dione (Compound No. 296 in Table-1)

A mixture of 6-benzyloxy-2-naphthylaldehyde (220 mg), 2,4-thiazolidinedione (128 mg) and sodium acetate (0.17 g) was heated at 115°C for 30 minutes. After reaction, the reaction mixture was allowed to cool to room temperature, washed with water and acetone (0.5 ml), and extracted with ethyl acetate. The extract was dried and the solvent was distilled off. The resulting product was recrystallized from ethyl acetate to obtain the title compound (140 mg, yield=46%). The spectral data are as follows.

$^1$H NMR (DMSO);
5.23 (s, 2H),
7.27 (dd, 1H, J=8.9Hz, 2.5Hz),
7.3-7.5 (m, 4H),
7.51 (d, 2H, J=6.7Hz),
7.62 (d, 2H, J=9.3Hz),
7.86 (d, 1H, J=8.7Hz),
7.91 (d, 1H, J=9.1Hz),
8.02 (s, 1H)
IR (KBγ);
3437, 3028, 1689, 1599, 1566, 1307, 1267, 1213 $cm^{-1}$, m.p; 291°C (decomposition)

Example 15

Synthesis of 5-[6-(2-fluorobenzyloxy)-2-naphthyl]-methylene-thiazolidine-2,4-dione (compound No. 299 in Table 1)

A mixture of 6-(2-fluorobenzyloxy)-2-naphthylaldehyde (300 mg), 2,4-thiazolidinedione (144 mg) and sodium acetate (226 mg) was heated at 120°C for 30 minutes, allowed to cool to room temperature on standing, washed with acetic acid (1 ml), water (10 ml) and ethyl acetate (10 ml), and filtered. The resulting precipitate was recrystallized from ethyl acetate to obtain the title compound (361 mg, yield=89%). The spectral data and melting point are as follows.

$^1H$ NMR (DMSO);
5.27 (s, 2H),
7.2-7.3 (m, 3H),
7.43 (t, 1H, J=7.9Hz),
7.51 (d, 1H, J=2.2Hz),
7.6-7.7 (m, 3H),
7.90 (t, 2H, J=8.5Hz),
8.04 (s, 1H)
IR (KBγ);
3435, 3124, 3022, 2775, 1736, 1691, 1585, 1493, 1394, 1325, 1271, 1190, 1008 $cm^{-1}$,
m.p.; 247°C (decomposition)

The compounds of Examples 14, 16 - 39, 41, 43 - 56 were obtained by methods similar to that described in Examples 13 and 15. These compounds were represented by the following formula (I-f).

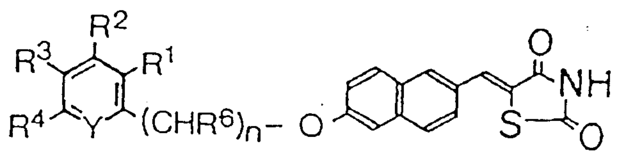

(I-f)

The spectral data and yield values of the above compounds are described in Table 6 wherein Me, Ac and Ph represent methyl, acetyl and phenyl, respectively.

Table 6

| Example No. | 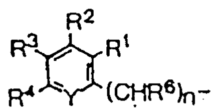 | mp (℃) / Yield (%) | NMR (ppm) | IR (cm⁻¹) |
|---|---|---|---|---|
| 14 | 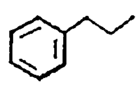 | 195 ~ 197 / 89 | 3.10 (t, 2H, J=6.9Hz)<br>4.31 (t, 2H, J=7.0Hz)<br>7.14 (dd, 1H, J=2.6Hz, 9.2Hz)<br>7.23 (d, 1H, J=6.8Hz)<br>7.28-7.38 (m, 7H)<br>7.60 (d, 1H, J=8.3Hz)<br>7.78-7.84 (m, 2H), 7.93 (s, 1H) | 3422, 3059<br>3026, 2926<br>1689, 1564<br>1309, 1271<br>1182, 1026 |
| 16 | 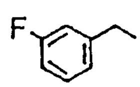 | 195 (Decomposition) / 82 | 5.25 (s, 2H), 7.17 (t, 1H, J=8.7Hz)<br>7.26 (d, 1H, J=10.7Hz)<br>7.35 (d, 2H, J=7.9Hz)<br>7.41-7.50 (m, 3H)<br>7.63 (d, 1H, J=8.6Hz)<br>7.83 (d, 1H, J=8.7Hz)<br>7.88 (d, 1H, J=9.1Hz), 7.97 (s, 1H) | 3022, 2893<br>1691, 1593<br>1568, 1309<br>1273, 1211<br>1186 |
| 17 | 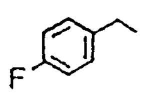 | 261 (Decomposition) / 62 | 5.22 (s, 2H), 7.20-7.30 (m, 3H)<br>7.47 (s, 1H), 7.50-7.70 (m, 2H)<br>7.67 (d, 2H, J=9.1Hz)<br>7.90 (m, 2H), 8.04 (s, 1H) | 3431, 3128<br>3026, 2787<br>1730, 1689<br>1593, 1514<br>1329, 1222<br>1178, 1157 |
| 18 | 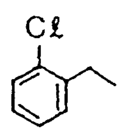 | 244 (Decomposition) / 84 | 5.30 (s, 2H)<br>7.31 (d, 1H, J=8.5Hz)<br>7.38-7.42 (m, 2H)<br>7.52 (brs, 2H), 7.62-7.66 (m, 2H)<br>7.78 (s, 1H)<br>7.92 (d, 1H, J=8.2Hz)<br>7.96 (d, 1H, J=8.7Hz), 8.08 (s, 1H) | 3427, 3136<br>3024, 2793<br>1734, 1689<br>1587, 1323<br>1271, 1182 |
| 19 | 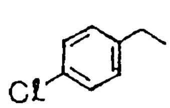 | 260 / 70 | 5.61 (s, 2H)<br>7.66 (d, 1H, J=8.4Hz)<br>7.84 (brs, 3H)<br>7.90 (d, 2H, J=8.0Hz)<br>7.99 (d, 1H, J=9.1Hz), 8.19 (s, 1H)<br>8.26 (d, 1H, J=8.6Hz)<br>8.32 (d, 1H, J=8.9Hz), 8.45 (s, 1H) | 3447, 3105<br>2982, 2791<br>1741, 1687<br>1581, 1334<br>1271, 1184<br>1170 |

Table 6 (continued)

| Example No. | 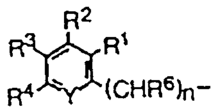 | m p (°C)<br>Yield (%) | NMR (ppm) | I R ($cm^{-1}$) |
|---|---|---|---|---|
| 2 0 | 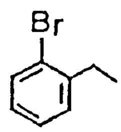 | 300 or over<br>36 | 5.25 (s, 2H)<br>7.27-7.90 (m, 10H)<br>8.02 (s, 1H) | 3429, 1690<br>1599, 1568<br>1395, 1312<br>1273, 1208<br>1181, 1026 |
| 2 1 | 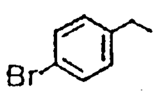 | 208 (Decomposition)<br>51 | 5.23 (s, 2H)<br>7.29 (d, 1H, J=7.8Hz)<br>7.47 (d, 3H, J=7.5Hz)<br>7.60 (d, 2H, J=7.8Hz)<br>7.65 (s, 1H), 7.77 (s, 1H)<br>7.89 (d, 1H, J=9.1Hz)<br>7.94 (d, 1H, J=9.3Hz), 8.07 (s, 1H) | 3445, 3107<br>2987, 1741<br>1687, 1583<br>1475, 1334<br>1269, 1184<br>1170 |
| 2 2 | 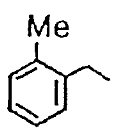 | 266 ~ 268 (Decomposition)<br>70 | 2.37 (s, 3H), 5.22 (s, 2H)<br>7.25 (m, 4H), 7.44 (s, 1H)<br>7.49 (d, 2H, J=2.1Hz)<br>7.65 (d, 1H, J=8.4Hz)<br>7.87 (t, 2H, J=7.5Hz, J=8.3Hz)<br>7.98 (s, 1H) | 3429, 3059<br>3026, 1689<br>1599, 1566<br>1394, 1311<br>1273, 1211<br>1176 |
| 2 3 | 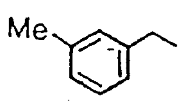 | 222 ~ 223<br>76 | 2.33 (s, 3H), 5.20 (s, 2H)<br>7.16 (s, 1H), 7.20-7.40 (m, 4H)<br>7.48 (s, 1H)<br>7.63 (d, 1H, J=8.3Hz)<br>7.81 (s, 1H), 7.88-7.96 (m, 2H)<br>8.08 (s, 1H) | 3425, 3132<br>3022, 1745<br>1693, 1606<br>1340, 1263<br>1186, 997 |
| 2 4 | 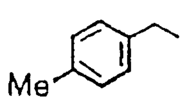 | 194 (Decomposition)<br>52 | 2.30 (s, 3H), 5.19 (s, 2H)<br>7.21 (d, 2H, J=7.7Hz)<br>7.28 (d, 1H, J=9.0Hz)<br>7.39 (d, 2H, J=7.9Hz)<br>7.47 (s, 1H), 7.63 (d, 1H, J=8.5Hz)<br>7.82 (s, 1H), 7.89 (d, 1H, J=8.9Hz)<br>7.94 (d, 1H, J=9.1Hz), 8.08 (s, 1H) | 3443, 3169<br>3051, 1743<br>1689, 1606<br>1586, 1336<br>1265, 1180 |

Table 6 (continued)

| Example No. | 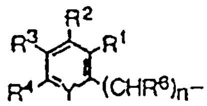 | m p (℃) / Yield (%) | NMR (ppm) | I R ($cm^{-1}$) |
|---|---|---|---|---|
| 2 5 | 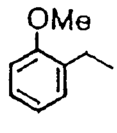 | 283 ~ 284 (Decomposition) / 86 | 3.85 (s, 3H), 5.19 (s, 2H)<br>6.99 (t, 1H, J=6.7Hz)<br>7.09 (d, 1H, J=8.2Hz)<br>7.23 (d, 1H, J=9.1Hz)<br>7.33-7.49 (m, 4H)<br>7.64 (d, 1H, J=8.7Hz)<br>7.84 (d, 1H, J=3.6Hz)<br>7.87 (d, 1H, J=4.3Hz), 7.98 (s, 1H) | 3431, 3059<br>1689, 1602<br>1562, 1413<br>1311, 1275<br>1246, 1114<br>1041 |
| 2 6 | 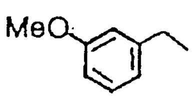 | 236 (Decomposition) / 66 | 3.76 (s, 3H), 5.22 (s, 2H)<br>6.91 (dd, 1H, J=7.5Hz, 2.0Hz)<br>7.08 (brs, 2H), 7.27-7.35 (m, 2H)<br>7.46 (s, 1H), 7.63 (d, 1H, J=8.5Hz)<br>7.76 (s, 1H), 7.89 (d, 1H, J=8.7Hz)<br>7.94 (d, 1H, J=9.2Hz), 8.07 (s, 1H) | 3445, 3121<br>3018, 2779<br>1739, 1682<br>1585, 1479<br>1332, 1273<br>1186, 1151 |
| 2 7 | 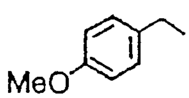 | 221 ~ 223 (Decomposition) / 57 | 3.76 (s, 3H), 5.15 (s, 2H)<br>6.97 (d, 2H, J=8.3Hz)<br>7.25 (d, 1H, J=9.3Hz)<br>7.45 (d, 3H, J=8.0Hz)<br>7.63 (d, 2H, J=10.6Hz)<br>7.89 (m, 2H), 8.02 (s, 1H) | 3429, 3009<br>1687, 1610<br>1516, 1304<br>1251, 1174<br>1032 |
| 2 8 | 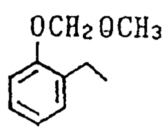 | 234 ~ 238 (Decomposition) / 43 | 3.39 (s, 3H), 5.22 (s, 2H)<br>5.28 (s, 2H), 7.03 (t, 1H, J=7.2Hz)<br>7.16 (d, 1H, J=8.1Hz)<br>7.25 (dd, 1H, J=9.0Hz, 2.3Hz)<br>7.33 (t, 1H, J=6.8Hz)<br>7.46-7.51 (m, 2H)<br>7.63 (t, 2H, J=3.4Hz)<br>7.89 (dd, 2H, J=9.0Hz, 5.2Hz)<br>8.02 (s, 1H) | 3427, 2953<br>1689, 1593<br>1566, 1494<br>1309, 1271<br>1178, 1153<br>999 |
| 2 9 | 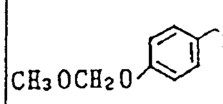 | 213 (Decomposition) / 68 | 3.36 (s, 3H), 5.16 (s, 2H)<br>5.18 (s, 2H), 7.04 (d, 2H, J=7.5Hz)<br>7.26 (d, 1H, J=9.2Hz)<br>7.40-7.50 (m, 3H)<br>7.62 (d, 1H, J=8.1Hz)<br>7.80 (s, 1H), 7.87-7.95 (m, 2H)<br>8.07 (s, 1H) | 3454, 3138<br>3016, 1741<br>1685, 1591<br>1514, 1327<br>1271, 1242<br>1186, 1003 |

Table 6 (continued)

| Example No. | 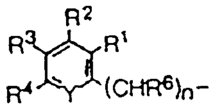 | mp (℃)<br>Yield (%) | NMR (ppm) | IR ($cm^{-1}$) |
|---|---|---|---|---|
| 30 | 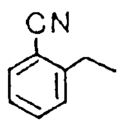 | 164 ~ 167 (Decomposition)<br>53 | 5.38 (s, 2H)<br>7.28 (dd, 1H, J=9.0Hz, 2.2Hz)<br>7.52 (s, 1H)<br>7.57-7.67 (m, 4H)<br>7.73-7.82 (m, 2H)<br>7.91 (m, 3H), 8.03 (s, 1H) | 3435, 2226<br>1689, 1599<br>1566, 1396<br>1311, 1273<br>1186, 1022 |
| 31 | 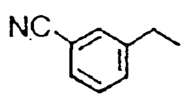 | 256 ~ 258 (Decomposition)<br>63 | 5.31 (s, 2H)<br>7.33 (dd, 1H, J=9.0Hz, 2.1Hz)<br>7.48 (s, 1H), 7.63 (t, 2H, J=7.4Hz)<br>7.81 (brs, 2H), 7.85 (s, 1H)<br>7.89 (d, 1H, J=4.2Hz)<br>7.94 (d, 1H, J=5.6Hz), 7.99 (s, 1H)<br>8.09 (s, 1H) | 3433, 3134<br>3028, 2789<br>2231, 1732<br>1687, 1589<br>1479, 1327<br>1273, 1186 |
| 32 | 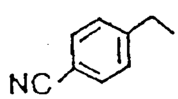 | 240 (Decomposition)<br>39 | 5.37 (s, 2H)<br>7.33 (d, 1H, J=8.6Hz)<br>7.47 (s, 1H), 7.60-7.70 (m, 3H)<br>7.81 (s, 1H), 7.89 (d, 3H, J=8.2Hz)<br>7.97 (d, 1H, J=8.8Hz)<br>8.09 (s, 1H) | 3431, 3204<br>3061, 2235<br>1741, 1705<br>1595, 1394<br>1305, 1271<br>1188, 1151 |
| 33 | 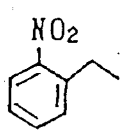 | 241 (Decomposition)<br>76 | 5.61 (s, 2H)<br>7.30 (d, 1H, J=8.6Hz)<br>7.46 (s, 1H), 7.64 (brs, 3H)<br>7.70-7.90 (m, 4H)<br>8.04 (s, 1H)<br>8.16 (d, 1H, J=7.7Hz) | 3431, 3059<br>2920, 1689<br>1599, 1566<br>1529, 1340<br>1309, 1271<br>1182 |
| 34 | 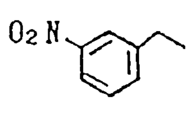 | 180 (Decomposition)<br>92 | 5.41 (s, 2H), 7.33 (d, 1H, J=7.6Hz)<br>7.49 (s, 1H), 7.60-7.80 (m, 2H)<br>7.80-8.10 (m, 3H)<br>8.20 (d, 1H, J=8.0Hz)<br>8.38 (s, 1H) | 3431, 3059<br>1691, 1535<br>1348, 1315<br>1273, 1186 |

Table 6 (continued)

| Example No. | 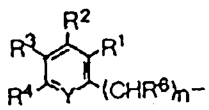 | mp (℃) / Yield (%) | NMR (ppm) | IR (cm⁻¹) |
|---|---|---|---|---|
| 35 | 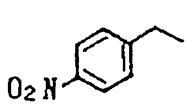 | 195 (Decomposition) / 40 | 5.43(s,2H), 7.35(d,1H,J=8.8Hz)<br>7.48(s,1H), 7.64(d,1H,J=8.6Hz)<br>7.79(d,3H,J=6.6Hz)<br>7.89(d,1H,J=8.5Hz)<br>7.97(d,1H,J=9.0Hz)<br>8.08(s,1H), 8.28(d,1H,J=8.4Hz) | 3431, 3302<br>3051, 1741<br>1705, 1593<br>1516, 1341<br>1271, 1186 |
| 36 | 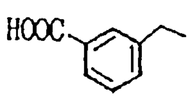 | 289 (Decomposition) / 52 | 5.34(s,1H), 7.33(d,1H,J=7.5Hz)<br>7.50(s,1H), 7.56(d,1H,J=7.8Hz)<br>7.63(d,1H,J=8.3Hz)<br>7.76(d,1H,J=7.4Hz)<br>7.84(s,1H), 7.89-7.89(m,3H)<br>8.08(d,2H,J=4.7Hz) | 3435, 3142<br>3047, 2361<br>1734, 1682<br>1593, 1319<br>1271, 1186<br>1155 |
| 37 | 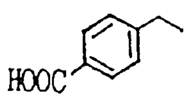 | 226 ~ 228 (Decomposition) / 71 | 5.35(s,2H)<br>7.34(d,1H,J=8.7Hz)<br>7.48(s,1H), 7.62(d,3H,J=7.6Hz)<br>7.85(s,1H), 7.90(d,2H,J=8.9Hz)<br>7.97(d,2H,J=8.3Hz)<br>8.10(s,1H) | 3422, 3020<br>1738, 1685<br>1591, 1394<br>1350, 1273<br>1184, 1012 |
| 38 | 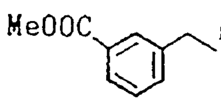 | 195 (Decomposition) / 79 | 3.86(s,3H), 5.34(s,2H)<br>7.31(dd,1H,J=8.9Hz,2.4Hz)<br>7.47(d,1H,J=2.1Hz)<br>7.57(t,1H,J=7.7Hz)<br>7.64(d,1H,J=8.5Hz), 7.72(s,1H)<br>7.79(d,1H,J=7.6Hz)<br>7.88(d,1H,J=8.7Hz)<br>7.94(d,2H,J=8.9Hz)<br>8.08(d,2H,J=10.2Hz), 8.31(s,1H) | 3429, 3192<br>3063, 1693<br>1597, 1394<br>1302, 1274<br>1205 |
| 39 | 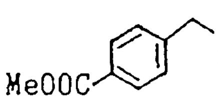 | 228 (Decomposition) / 63 | 3.85(s,3H), 5.35(s,2H)<br>7.33(dd,1H,J=9.0Hz,2.3Hz)<br>7.46(s,1H), 7.60-7.70(m,3H)<br>7.75(s,1H), 7.88(d,1H,J=8.7Hz)<br>7.90-8.00(m,3H), 8.07(s,1H) | 3435, 3184<br>3063, 2957<br>1711, 1597<br>1394, 1275<br>1186, 1018 |

Table 6 (continued)

| Example No. | 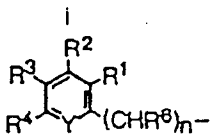 | mp (℃) / Yield (%) | NMR (ppm) | IR ($cm^{-1}$) |
|---|---|---|---|---|
| 41 | 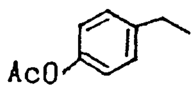 | 212 (Decomposition) / 69 | 2.27(s,3H), 5.24(s,2H)<br>7.16(d,2H,J=8.5Hz)<br>7.29(dd,1H,J=8.9Hz,2.3Hz)<br>7.48(d,1H,J=2.1Hz)<br>7.57(d,2H,J=8.5Hz)<br>7.64(dd,1H,J=8.6Hz,1.6Hz)<br>7.71(s,1H), 7.91(m,2H)<br>8.05(s,1H) | 3439, 3036<br>1741, 1689<br>1593, 1271<br>1219, 1186<br>1016 |
| 43 | 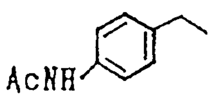 | 200 (Decomposition) / 62 | 2.03(s,3H), 5.16(s,2H)<br>7.28(d,1H,J=10.0Hz)<br>7.42(d,2H,J=8.3Hz)<br>7.48(s,1H), 7.60(d,2H,J=8.0Hz)<br>7.64(s,1H), 7.83(s,1H)<br>7.92(t,2H,J=9.9Hz)<br>8.09(s,1H), 9.99(s,1H) | 3302, 3126<br>3034, 2775<br>1739, 1685<br>1589, 1523<br>1331, 1271<br>1184, 1001 |
| 44 | 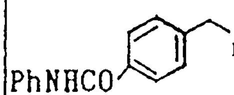 | 249 (Decomposition) / 91 | 5.35(s,1H), 7.09(t,1H,J=7.5Hz)<br>7.29-7.37(m,3H), 7.46(s,1H)<br>7.53(s,1H), 7.60-7.70(m,3H)<br>7.76(d,2H,J=8.1Hz)<br>7.84(d,1H,J=8.8Hz)<br>7.91(d,1H,J=8.9Hz)<br>7.90-8.00(m,3H) | 3306, 3055<br>1649, 1601<br>1545, 1442<br>1325, 1265<br>1178 |
| 45 | 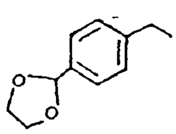 | 251 (Decomposition) / 63 | 3.92-3.98(m,2H)<br>3.99-4.05(m,2H)<br>5.26(s,2H), 5.73(s,1H)<br>7.28(dd,1H,J=9.0Hz,2.4Hz)<br>7.44-7.48(m,3H)<br>7.53(d,2H,J=8.2Hz)<br>7.63(d,2H,J=11.2Hz)<br>7.86(d,1H,J=8.7Hz)<br>7.92(d,1H,J=9.1Hz), 8.04(s,1H) | 3433, 3140<br>3030, 2885<br>1739, 1687<br>1595, 1564<br>1394, 1305<br>1271, 1184<br>1082, 1018 |
| 46 | 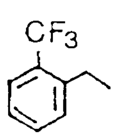 | 225 ~ 226 (Decomposition) / 43 | 5.37(s,2H)<br>7.29(dd,1H,J=9.0Hz,1.7Hz)<br>7.51(s,1H), 7.60-7.70(m,2H)<br>7.75(t,1H,J=7.1Hz)<br>7.83(m,3H), 7.95(t,2H,J=9.1Hz)<br>8.11(s,1H) | 3437, 3030<br>2779, 1739<br>1693, 1593<br>1315, 1271<br>1118, 1033 |

Table 6 (continued)

| Example No. | 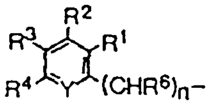 | mp (℃)<br>Yield (%) | NMR (ppm) | IR (cm⁻¹) |
|---|---|---|---|---|
| 47 | 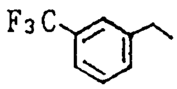 | 266 ~ 267 (Decomposition)<br>63 | 5.36 (s, 2H)<br>7.33 (d, 1H, J=9.5Hz)<br>7.50 (s, 1H)<br>7.62-7.75 (m, 4H)<br>7.82-7.92 (m, 3H)<br>7.96 (d, 1H, J=8.9Hz)<br>8.08 (s, 1H) | 3427, 3117<br>3016, 2777<br>1743, 1691<br>1585, 1332<br>1273, 1203<br>1188, 1155<br>1114 |
| 48 | 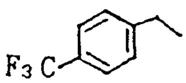 | 206 (Decomposition)<br>47 | 5.36 (s, 2H)<br>7.30 (d, 1H, J=8.4Hz)<br>7.45 (s, 1H), 7.58-7.65 (m, 2H)<br>7.75 (d, 4H, J=3.8Hz)<br>7.85 (d, 1H, J=9.0Hz)<br>7.92 (d, 1H, J=9.1Hz), 8.02 (s, 1H) | 3429, 3022<br>1691, 1608<br>1566, 1267<br>1213, 1172<br>1122, 1068<br>1018 |
| 49 | 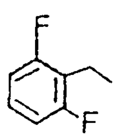 | 279 ~ 282<br>54 | 5.26 (s, 2H)<br>7.18-7.29 (m, 3H)<br>7.52-7.68 (m, 3H)<br>7.90-7.99 (m, 3H)<br>8.13 (s, 1H) | 3135, 3034<br>1738, 1678<br>1591, 1470<br>1331, 1186<br>1152, 1055 |
| 50 | 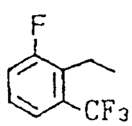 | 232 ~ 235<br>27 | 5.31 (s, 2H)<br>7.25 (dd, 1H, J=2.5Hz, 9.0Hz)<br>7.63-7.74 (m, 5H), 7.91 (s, 1H)<br>7.97 (d, 1H, J=8.5Hz)<br>7.98 (d, 1H, J=9.3Hz)<br>7.99 (s, 1H), 12.61 (s, 1H) | 3140, 3036<br>1736, 1692<br>1591, 1321<br>1188, 1171<br>1127, 1009 |
| 51 | 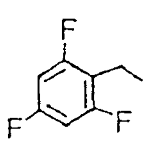 | 253 ~ 255<br>72 | 5.23 (s, 2H), 7.24-7.35 (m, 3H)<br>7.59 (d, 1H, J=2.3Hz)<br>7.66 (d, 1H, J=7.0Hz)<br>7.90-7.99 (m, 2H), 8.16 (s, 1H)<br>12.61 (s, 1H) | 3154, 3057<br>1748, 1682<br>1624, 1591<br>1505, 1443<br>1325, 1269 |

Table 6 (continued)

| Example No. | 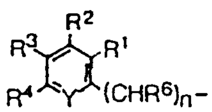 | m p (℃)<br>Yield (%) | NMR (ppm) | IR ($cm^{-1}$) |
|---|---|---|---|---|
| 52 | 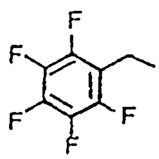 | 239 ~ 240<br>39 | 5.35 (s, 2H)<br>7.27 (d, 1H, J=8.7Hz)<br>7.61 (s, 1H)<br>7.67 (d, 1H, J=8.8Hz)<br>7.87 (s, 1H)<br>7.97 (t, 2H, J=9.5Hz), 8.13 (s, 1H) | 3443, 3155<br>3057, 1747<br>1703, 1664<br>1606, 1597<br>1471, 1392<br>1182, 1138 |
| 53 | 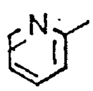 | 263 (Decomposition)<br>83 | 5.33 (s, 2H)<br>7.30-7.40 (m, 2H)<br>7.49 (s, 1H), 7.58 (d, 1H, J=8.0Hz)<br>7.63 (d, 1H, J=9.0Hz)<br>7.80-8.00 (m, 4H)<br>8.11 (s, 1H), 8.60 (d, 1H, J=5.3Hz) | 3435, 2924<br>1709, 1601<br>1392, 1292<br>1267, 1188<br>1149, 1093 |
| 54 | 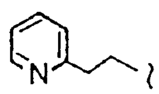 | 166 ~ 167 (Decomposition)<br>27 | 3.25 (t, 2H, J=7.1Hz)<br>4.49 (t, 2H, J=6.5Hz)<br>7.12 (dd, 1H, J=2.3Hz, 9.2Hz)<br>7.24 (t, 1H, J=6.1Hz)<br>7.37 (d, 1H, J=2.0Hz)<br>7.40 (d, 1H, J=7.9Hz)<br>7.50-7.70 (m, 1H)<br>7.73 (dt, 1H, J=1.7Hz, 9.4Hz)<br>7.83 (d, 2H, J=8.7Hz), 7.95 (s, 1H)<br>8.51 (d, 1H, J=4.7Hz) | 3431, 2926<br>1697, 1566<br>1413, 1298<br>1261, 1155<br>1018 |
| 55 | 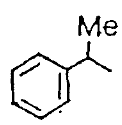 | 271 (Decomposition)<br>61 | 1.60 (d, 3H, J=5.0Hz)<br>5.68 (q, 1H, J=6.5Hz)<br>7.19-7.26 (m, 3H), 7.31 (s, 1H)<br>7.35 (t, 2H, J=3.4Hz)<br>7.47 (d, 2H, J=7.0Hz)<br>7.56 (d, 1H, J=8.6Hz)<br>7.68 (d, 1H, J=8.7Hz)<br>7.80 (d, 1H, J=9.7Hz), 7.90 (s, 1H) | 3429, 3065<br>3032, 1666<br>1608, 1564<br>1413, 1307<br>1267, 1232<br>1187 |
| 56 | 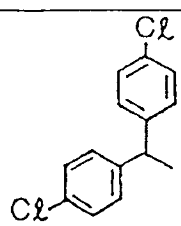 | 202 (Decomposition)<br>13 | 6.80 (s, 1H), 7.34 (d, 2H, J=7.7Hz)<br>7.43 (d, 4H, J=8.4Hz), 7.49 (s, 1H)<br>7.54-7.60 (m, 5H)<br>7.72 (d, 1H, J=8.4Hz)<br>7.88 (d, 1H, J=9.4Hz), 7.96 (s, 1H) | 3369, 3173<br>3059, 1745<br>1689, 1676<br>1593, 1491<br>1178, 1012 |

Example 40

Synthesis of 5-[6-(4-formylbenzyloxy)-2-naphthyl]-methylene-thiazolidine-2,4-dione (compound No. 430 in Table 1)

5-{6-[4-(1,3-ethylenedioxy)-methylbenzyloxy]-2-naphthyl}-methylene-thiazoline-2,4-dione (157 mg) obtained in Example 45 was suspended in acetone (90 ml) and then p-toluenesulfonic acid (10 mg) was added to the supension. The resultant suspension was stirred at room temperature for 36 hours. After reaction, the acetone was distilled off. The resulting residue was recrystallized from hexane/ethyl acetate, washed with water and dried to obtain the title compound (80 mg, yield=57%). The spectral data and melting point are as follows.

$^1$H NMR (DMSO);
5.38 (s, 2H),
7.34 (d, 1H, J=9.0Hz),
7.49 (s, 1H),
7.63 (d, 1H, J=8.5Hz),
7.72 (d, 2H, J=7.8Hz),
7.8-8.0 (m, 5H),
10.01 (s, 1H)
IR (KBγ);
3126, 3026, 2779, 1738, 1697, 1595, 1396, 1273, 1186 cm$^{-1}$,
m.p.; 281°C (decomposition)

Example 42

Synthesis of 5-[6-(3-aminobenzyloxy)-2-naphthyl]-methylene-thiazolidine-2,4-dione (compound No. 400 in Table 1)

5-[6-(3-nitrobenzyloxy)-2-naphthyl]-methylene-thiazoline-2,4-dione (300 mg) obtained in Example 34 was suspended in a mixture of methanol (50 ml) and methoxyethanol (75 ml) and then palladium on carbon (0.4 g) was added to the suspension under an inert atmosphere. After replacing the atmosphere with a hydrogen atmosphere, the resulting suspension was stirred overnight at room temperature at ordinary pressure.

After reaction, methanol (100 ml) was added, and the reaction mixture was vigorously stirred to dissolve the objective material, and filtered through celite. The solvent was distilled off. The resulting residue was recrystallized from ethyl acetate to obtain the title compound (130 mg, yield=49%). The spectral data and melting point are as follows.

$^1$H NMR (DMSO);
5.07 (s, 2H),
6.51 (d, 1H, J=8.5Hz),
6.61 (d, 1H, J=7.8Hz),
6.67 (s, 1H),
7.02 (t, 1H, J=7.8Hz),
7.24 (dd, 1H, J=9.0, 2.3Hz),
7.41 (s, 1H),
7.62 (d, 2H, J=6.5Hz),
7.84 (d, 1H, J=8.5Hz),
7.90 (d, 1H, J=9.0Hz),
8.02 (s, 1H)
IR (KBγ);
3437, 3030, 1689, 1597, 1560, 1307, 1269, 1186, 1020 cm$^{-1}$,
m.p.; 227-229°C (decomposition)

Example 57

Synthesis of 5-[6-(2-trifluoromethylbenzyloxy)-2-naphthyl]-methylene-2-thioxy-thiazolidine-4-one (compound No. 703 in Table 2)

To a mixture of 6-(2-trifluoromethylbenzyloxy)-2-naphthylaldehyde (594 mg), rhodanine (266 mg) and sodium acetate (443 mg) was added acetic acid (2.3 ml). The mixture was heated to reflux for 2 hours and cooled gradually, and water (10 ml) was added thereto. The resulting mixture was sufficiently stirred and filtered. The resultant solid was recrystallized from ethanol, filtered and dried to obtain the title compound (543 mg, yield=68%). The spectral data and melting point are as follows.

$^1$H NMR (DMSO);

5.38 (s, 2H),
7.31 (dd, 1H, J=9.0Hz, 2.5Hz),
7.53 (d, 1H, J=2.2Hz),
7.58-7.67 (m, 2H),
7.75 (m, 2H),
7.8-7.9 (m, 2H),
7.96 (d, 1H, J=8.7Hz),
8.02 (d, 1H, J=91Hz),
8.14 (s, 1H)
IR (KBγ);
3431, 3140, 3055, 2854, 1697, 1585, 1448, 1396, 1317, 1236, 1174, 1126 $cm^{-1}$
m.p.; 221-224°C

Preparation 11

Synthesis of 2-(6-benzyloxy)-naphthyl-methyl cyanide

2-(6-Benzyloxy)-naphthyl-methyl chloride (3.0 g) is dissolved in a mixture of DMF (30 ml) and EtOH (30 ml), and potassium cyanide (1.38 g) is added to the slolution. The resulting mixture is stirred with heating under reflux for 48 hours. After reaction, the mixture is cooled to room temperature and toluene is added. The organic layer was washed with water and a saturated saline solution, dried over anhydrous magnesium sulfate, and concentrated in vacuo to obtain a residue. To the residue is added ethyl acetate (30 ml). The resulting crystals are washed under heating, cooled and then filtered to obtain the tile compound (2.25 g, yield=78%). The NMR spectrum is as follows.

NMR ($CDCl_3$)
3.88 (s, 2H),
5.19 (s, 2H),
7.22-7.42 (m, 7H),
7.48 (dt, 1H, J=1.5Hz, 7.0Hz),
7.73-7.76 (m, 3H)

Example 58

Synthesis of 5-(6-benzyloxy-2-naphthyl)-methyl-1H-tetrazole (compound No. 720 in Table 3)

To a solution of 6-benzyloxy-2-naphthyl-methyl cyanide (0.40 g) in DMF (6 ml) were added sodium azide (0.48 g) and ammonium chloride (0.39 g). The mixture was stirred at 135°C for 24 hours.

After reaction, the mixture is cooled to room temperature and ethyl acetate was added. The organic layer was washed, dried and concentrated in vacuo to obtain a residue. The resultant residue was subjected to column chromatography on silica gel eluting with chloroform/methanol to obtain an amorphous solid. It was recrystallized from ethyl acetate to obtain the tile compound (0.15 g, yield=32%). The spectral data and melting point are as follows.

NMR (DMSO d-6);
4.46 (s, 2H),
5.20 (s, 2H),
7.22 (dd, 1H, J=2.4Hz, 9.0Hz),
7.33-7.42 (m, 5H),
7.50 (d, 2H, J=6.8Hz),
7.70 (s, 1H),
7.77 (t, 1H, J=7.9Hz)
IR (KBγ);
3437, 3135, 3036, 2897, 2751, 1607, 1559, 1391, 1263, 1229, 1178 $cm^{-1}$
m.p.; 215-217

Example 59

Synthesis of 5-[6-(2-fluorobenzyloxy)-2-naphthyl]-methyl-thiazolidine-2,4-dione sodium salt (sodium salt of compound 5 in Table-1)

5-[6-(2-fluorobenzyloxy)-2-naphthyl]-methylthiazolidine-2,4-dione (3.81 g) obtained in Example 4 was suspended in methanol (100 ml) and sodium methoxide (28% methanol solution, 2.2 g) was added thereto. The mixture was stirred at room temperature for 1 hour.

After reaction, ethyl ether (40 ml) was added to the reaction mixture, so that the sodium salt was obtained as crystals. The crystals were washed with ethanol (40 ml) to obtain the title compound (3.70 g, yield=92%). The NMR, IR spectrum and melting point are as follows.

NMR (DMSO d-6);
2.77 (dd, 1H, J=10.4Hz, 13.7Hz),
3.49 (dd, 1H, J=3.6Hz, 13.6Hz),
4.20 (dd, 1H, J=3.5Hz, 10.6Hz),
5.22 (S, 1H),
7.10-7.30 (m, 3H),
7.32 (t, 1H, J=6.3Hz),
7.40-7.50 (m, 2H),
7.62 (t, 2H, J=7.3Hz),
7.70 (d, 1H, J=8.5Hz),
7.76 (d, 1H, J=9.0Hz)
IR (KBγ);
3427, 3042, 1660, 1560, 1491, 1325, 1267, 1232, 1047
m.p.; >300°C (decomposition)

Test examples

The effect of the compound of the present invention on reducing blood sugar and blood lipid levels based on the ability of improving insulin resistance has been determined by the following test.

KK-Ay male mice of five to six week age were obtained from Nihon CREA. The mice have been bred with a powder feed (MF powders for breeding rats and mice, Oriental Yeast Co.) from 7 days prior to the test. The mice of nine to eighteen week age having body weights of 35 g or more were used for the test.

The blood sugar values were measured by withdrawing blood (20 μl) from animal's tail vein using a heparin-treated capillary, centrifuging the blood to obtain plasma, and measuring the glucose level in the plasma by the glucose-oxidase method. The triglyceride (TG) levels in plasma were measured by the glycerol enzyme method. Five mice in a group having 200 mg/dl or more of the blood sugar level were used for one test.

The test compounds were mixed with powder food such that the average dosage of the former is 10 - 100 mg/kg/day, and the mixture was administered to the mice for four days. Blood was withdrawn from the animal's tail vein before administration, and five days after administration, and blood sugar and TG levels were measured using the methods mentioned above. The amount of the food ingested was measured every day during the test period, and the average of the amounts for four days was calculated.

The ability of reducing blood sugar level was determined as described below. Namely, the means of the blood sugar values at the time before administration of the test compound in a control group (a group to which the test compound was not administered) and an administration group (a group to which the test compound was administered) (such values are referred to as Mcon and Mad, respectively) and the means of the blood sugar values of the control group and the administration group on 5th day after administration (such values are referred to as Con and Ad, respectively) were determined. The blood sugar lowering effect found in the administration group was expressed by the following formula.

$$\textit{Blood sugar lowering effect}\ (\%) = 1 - \frac{Ad/Mad}{Con/Mcon} \times 100$$

The blood TG lowering ratio (%) was measured by the same procedure as that described above. All the values were statistically evaluated under significance level P=0.05.

The results are shown in Tables 7 and 8. The data of the known compounds pioglitazone [the following formula (II)] and CS-045 [the following formula (III)] are also listed in the tables.

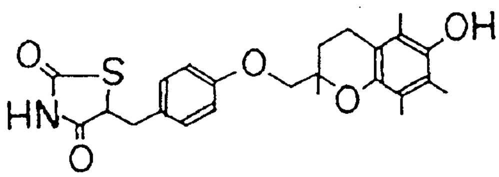

(II)

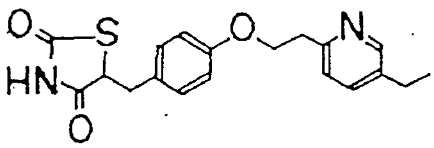

(III)

Table 7

| Dose: c.a. 10 mg/kg/day | | |
|---|---|---|
| Compound (Example No.) | Blood sugar lowering ratio (%) | TG lowering ratio (%) |
| 1 a) | 31.6** | 56.4 |
| 2 | 27.6** | 9.1* |
| 3 a) | 13.5 | 31.3 |
| 4 | 37.5** | 41.6* |
| 6 | 37.2*** | 37.7* |
| 7 | 33.7* | 28.3 |
| 8 | 10.9 | 9.4 |
| 10 | 56.2*** | 37.2 |
| Pioglitazone | 17.5* | -4.0 |
| CS-045 a) | 40.2** | -18.5 |

(***: p<0.001,
**: <0.01,
*: <0.05)
a) : Dose c.a. 100 mg/kg/day

Table 8

| Dose: c.a. 50 mg/kg/day | | |
|---|---|---|
| Compound (Example No.) | Blood sugar lowering ratio (%) | TG lowering ratio (%) |
| 13 a) | 41.5** | 21.0 |
| 14 b) | 35.2** | 19.9 |
| 15 a) | 48.6*** | 38.8 |
| 16 a) | 37.2* | 22.3 |
| 17 a) | 35.9*** | 24.4 |
| 18 | 54.4*** | 53.8* |
| 19 | 34.4*** | 21.7 |
| 21 | 12.7 | 14.7 |
| 22 | 31.2* | 9.5 |
| 32 | 31.7* | 39.6* |
| 33 | 28.9 | 16.0 |
| 35 | 45.8*** | 56.9 |
| 39 | 17.1 | 16.0 |
| 46 | 48.1*** | 51.0** |
| 48 | 58.5*** | 25.6 |
| 49 a) | 39.6** | 43.4*** |
| 50 a) | 41.1* | 27.7** |
| 51 a) | 53.1*** | 46.1** |
| 52 | 45.9*** | 44.2 |
| 53 | 7.6 | 34.5* |
| 55 | 26.3 | 17.4 |

(***: P<0.001,
**: <0.01,
*: <0.05)
a) : Dose c.a. 30 mg/kg/day
b) : Dose c.a. 100 mg/kg/day

As apparent from the above results, the compounds of the present invention are useful for reducing blood sugar and blood lipid levels in the dosage ranging from 10 to 100 mg/kg/ day.

## Claims

1. A naphthalene derivative represented by the following formula (I):

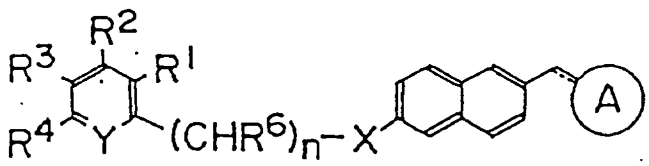

(I)

wherein the symbol

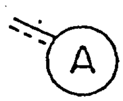

represents

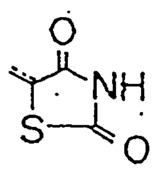 , 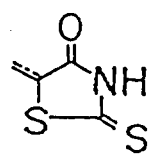 or 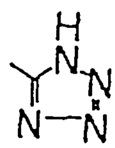 ;

-X- represents -O- or -S-; =Y- represents =N- or =$CR^5$-; each of $R^1$, $R^2$, $R^3$, $R^4$ and $R^5$ represents independently hydrogen, halogen, $C_1$-$C_8$ alkyl, $C_6$-$C_{12}$ aryl, $C_1$-$C_8$ alkoxy, $C_2$-$C_6$ alkoxyalkoxy, $C_6$-$C_{12}$ aryloxy, $C_2$-$C_9$ alkanoyloxy, $C_7$-$C_{13}$ arylcarbonyloxy, carboxy, $C_2$-$C_9$ alkoxycarbonyl, $C_7$-$C_{13}$ aryloxycarbonyl, carbamoyl, $C_2$-$C_9$ alkylaminocarbonyl, $C_7$-$C_{13}$ arylaminocarbonyl, amino, $C_1$-$C_8$ alkylamino, $C_2$-$C_9$ alkanoylamino, $C_7$-$C_{13}$ arylcarbonylamino, ethylenedioxymethyl, formyl, cyano, nitro or trihalomethyl; $R^6$ represents hydrogen, $C_1$-$C_8$ alkyl which may contain one or more substituents selected from the group consisting of phenyl, halogen, nitro and cyano, or $C_6$-$C_{12}$ aryl which may contain one or more substituents selected from the group consisting of $C_1$-$C_8$ alkyl, halogen, nitro and cyano n represents an integer of 0 to 3; and the dotted and solid lines show that the bond may be a single or double bond; or a pharmaceutically acceptable salt thereof.

2. A compound as claimed in Claim 1 characterized in that each of $R^1$, $R^2$, $R^3$, $R^4$ and $R^5$ represents independently hydrogen, halogen, $C_1$-$C_8$ alkyl, $C_1$-$C_8$ alkoxy, $C_2$-$C_6$ alkoxyalkoxy, $C_2$-$C_9$ alkanoyloxy, $C_7$-$C_{13}$ arylcarbonyloxy, carboxy, $C_2$-$C_9$ alkoxycarbonyl, carbamoyl, $C_2$-$C_9$ alkylaminocarbonyl, $C_7$-$C_{13}$ arylaminocarbonyl, amino, $C_1$-$C_8$ alkylamino, $C_2$-$C_9$ alkanoylamino, $C_7$-$C_{13}$ arylcarbonylamino, ethylenedioxymethyl, formyl, cyano, nitro or trihalomethyl; $R^6$ represents hydrogen, $C_1$-$C_8$ alkyl, or $C_6$-$C_{12}$ aryl which may be substituted by halogen.

3. A compound as claimed in Claim 1 characterized in that X represents -O-; Y represents =$CR^5$-; each of $R^1$, $R^2$, $R^3$, $R^4$ and $R^5$ represents independently hydrogen, halogen, $C_1$-$C_5$ alkyl, $C_1$-$C_5$ alkoxy, $C_2$-$C_6$ alkoxyalkoxy, $C_2$-$C_6$ alkanoyloxy, carboxy, $C_2$-$C_6$ alkoxycarbonyl, $C_7$-$C_{13}$ arylaminocarbonyl, amino, $C_2$-$C_6$ alkanoylamino, ethylenedioxymethyl, formyl, cyano, nitro or trihalomethyl; $R^6$ represents hydrogen, $C_1$-$C_5$ alkyl or $C_6$-$C_{12}$ aryl which may be substituted by halogen.

4. A compound as claimed in Claim 1 characterized in that the symbol

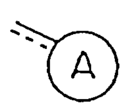

represents

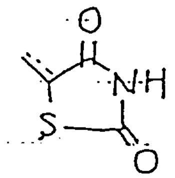

X represents -O-; Y represents $=CR^5-$; each of $R^1$, $R^2$, $R^3$ and $R^4$ represents independently hydrogen or halogen; $R^5$ represents hydrogen; $R^6$ represents hydrogen; n represents 1; and the dotted and solid lines represent that the bond is a single bond.

**5.** A compound as claimed in Claim 1 characterized in that the symbol

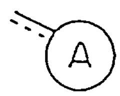

represents

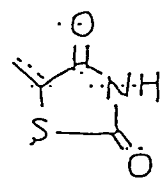

X represents -O-; Y represents $=CR^5-$; $R^1$ represents fluorine; each of $R^2$, $R^3$ and $R^4$ represents hydrogen; $R^5$ represents hydrogen; $R^6$ represents hydrogen; n represents 1; and the dotted and solid lines represent that the bond is a single bond.

**6.** A pharmaceutical composition which comprises as an active ingredient a compound as claimed in Claim 1 and a pharmaceutically acceptable carrier.

**7.** A pharmaceutical composition for diabetes which comprises as an active ingredient a compound as claimed in Claim 1 and a pharmaceutically acceptable carrier.

## Patentansprüche

**1.** Naphthalin-Derivat mit der folgenden Formel (I):

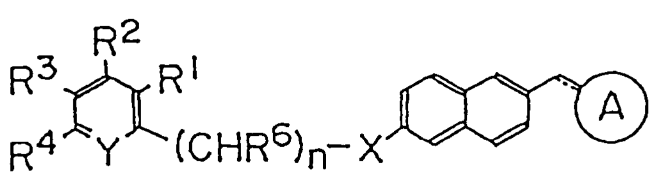

(I)

worin gilt:
das Symbol

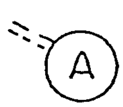

stellt

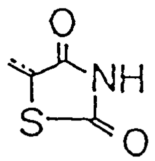 , 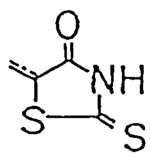 oder 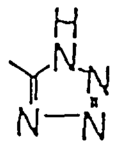

dar; -X- bedeutet -O- oder -S-; =Y- bedeutet =N- oder =CR$^5$-;
$R^1$, $R^2$, $R^3$, $R^4$ und $R^5$ bedeuten jeweils unabhängig Wasserstoff, Halogen, $C_1$-$C_8$-Alkyl, $C_6$-$C_{12}$-Aryl, $C_1$-$C_8$-Alkoxy, $C_2$-$C_6$-Alkoxyalkoxy, $C_6$-$C_{12}$-Aryloxy-, $C_2$-$C_9$-Alkanoyloxy, $C_7$-$C_{13}$-Arylcarbonyloxy, Carboxy, $C_2$-$C_9$-Alkoxycarbonyl, $C_7$-$C_{13}$-Aryloxycarbonyl, Carbamoyl, $C_2$-$C_9$-Alkylaminocarbonyl, $C_7$-$C_{13}$-Arylaminocarbonyl, Amino, $C_1$-$C_8$-Alkylamino, $C_2$-$C_9$-Alkanoylamino, $C_7$-$C_{13}$-Arylcarbonylamino, Ethylendioxymethyl, Formyl, Cyano, Nitro oder Trihalomethyl; $R^6$ bedeutet Wasserstoff, $C_1$-$C_8$-Alkyl, das einen oder mehrere Substituenten, ausgewählt aus der Gruppe aus Phenyl, Halogen, Nitro und Cyano enthalten kann, oder $C_6$-$C_{12}$- Aryl, das einen oder mehreren Substituenten, ausgewählt aus der Gruppe aus $C_1$-$C_8$-Alkyl, Halogen, Nitro und Cyano enthalten kann; n bedeutet eine Zahl von 0 bis 3; und die gestrichelten und durchgezogenen Linien bedeuten, daß die Bindung eine Einfach- oder Doppelbindung sein kann; oder eines seiner pharmazeutisch akzeptablen Salze.

**2.** Verbindung gemäß Anspruch 1,
dadurch **gekennzeichnet**, daß
jeder der Reste $R^1$, $R^2$, $R^3$, $R^4$ und $R^5$ unabhängig Wasserstoff, Halogen, $C_1$-$C_8$-Alkyl, $C_1$-$C_8$-Alkoxy, $C_2$-$C_6$-Alkoxyalkoxy, $C_2$-$C_9$-Alkanoyloxy, $C_7$-$C_{13}$-Arylcarbonyloxy, Carboxy, $C_2$-$C_9$-Alkoxycarbonyl, Carbamoyl, $C_2$-$C_9$-Alkylaminocarbonyl, $C_7$-$C_{13}$-Arylaminocarbonyl, Amino, $C_1$-$C_8$-Alkylamino, $C_2$-$C_9$-Alkanoylamino, $C_7$-$C_{13}$-Arylcarbonylamino, Ethylendioxymethyl, Formyl, Cyano, Nitro oder Trihalomethyl bedeutet; $R^6$ bedeutet Wasserstoff, $C_1$-$C_8$-Alkyl oder $C_6$-$C_{12}$-Aryl, das mit Halogen substituiert sein kann.

**3.** Verbindung gemäß Anspruch 1,
dadurch **gekennzeichnet**, daß
X -O- bedeutet; Y =CR$^5$- bedeutet; $R^1$, $R^2$, $R^3$, $R^4$ und $R^5$ jeweils unabhängig Wasserstoff, Halogen, $C_1$-$C_5$-Alkyl, $C_1$-$C_5$-Alkoxy, $C_2$-$C_6$-Alkoxyalkoxy, $C_2$-$C_6$-Alkanoyloxy, Carboxy, $C_2$-$C_6$-Alkoxycarbonyl, $C_7$-$C_{13}$-Arylaminocarbonyl, Amino, $C_2$-$C_6$-Alkanoylamino, Ethylendioxymethyl, Formyl, Cyano, Nitro oder Trihalomethyl bedeuten; $R^6$ bedeutet Wasserstoff, $C_1$-$C_5$-Alkyl oder $C_6$-$C_{12}$-Aryl, das mit Halogen substituiert sein kann.

**4.** Verbindung gemäß Anspruch 1,
dadurch **gekennzeichnet**, daß
das Symbol

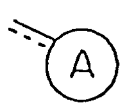 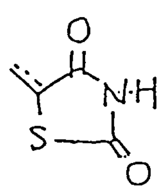

bedeutet;
X bedeutet -O-; Y bedeutet =CR$^5$-; $R^1$, $R^2$, $R^3$ und $R^4$ bedeuten jeweils unabhängig Wasserstoff oder Halogen; $R^5$ bedeutet Wasserstoff; $R^6$ bedeutet Wasserstoff; n bedeutet 1 und die gestrichelten und durchgezogenen Linien bedeuten, daß die Bindung eine Einfachbindung ist.

**5.** Verbindung gemäß Anspruch 1
dadurch **gekennzeichnet**, daß
das Symbol

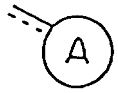

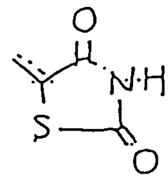

bedeutet;

X bedeutet -O-; Y bedeutet =CR$^5$-; R$^1$ bedeutet Fluor; R$^2$, R$^3$ und R$^4$ bedeuten alle Wasserstoff; R$^5$ bedeutet Wasserstoff; R$^6$ bedeutet Wasserstoff; n bedeutet 1 und die gestrichelten und durchgezogenen Linien bedeuten, daß die Bindung eine Einfachbindung ist.

6. Pharmazeutische Zusammensetzung, umfassend als Wirkstoff eine Verbindung gemäß Anspruch 1 und einen pharmazeutisch akzeptablen Träger.

7. Pharmazeutische Zusammensetzung für Diabetes, die als Wirkstoff eine Verbindung gemäß Anspruch 1 und einen pharmazeutisch akzeptablen Träger umfaßt.

**Revendications**

1. Dérivé du naphtalène représenté par la formule (I) suivante :

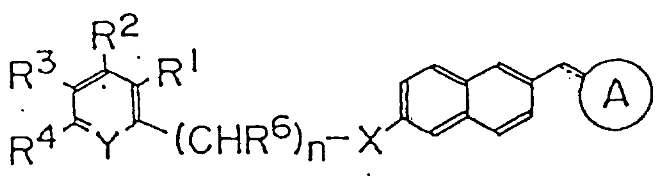

(I)

dans laquelle le symbole

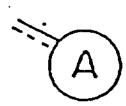

représente

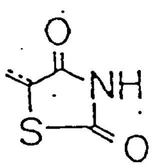 ,

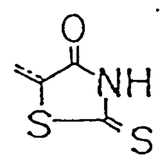 ou

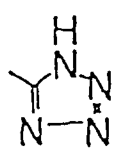 ;

-X- représente -O- ou -S- ; =Y- représente =N- ou =CR$^5$ - ; chacun de R$^1$, R$^2$, R$^3$, R$^4$ et R$^5$ représente indépendamment un atome d'hydrogène, un atome d'halogène ou un groupe alkyle en $C_1$-$C_8$, aryle en $C_6$-$C_{12}$, alcoxy en $C_1$-$C_8$, alcoxyalcoxy en $C_2$-$C_6$, aryloxy en $C_6$-$C_{12}$, alcanoyloxy en $C_2$-$C_9$, arylcarbonyloxy en $C_7$-$C_{13}$, carboxy, alcoxycarbonyle en $C_2$-$C_9$, aryloxycarbonyle en $C_7$-$C_{13}$, carbamoyle, alkylaminocarbonyle en $C_2$-$C_9$, arylaminocarbonyle en $C_7$-$C_{13}$, amino, alkylamino en $C_1$-$C_8$, alcanoylamino en $C_2$-$C_9$, arylcarbonylamino en $C_7$-$C_{13}$, éthylènedioxyméthyle, formyle, cyano, nitro ou trihalogénométhyle ; R$^6$ représente un atome d'hydrogène ou un groupe alkyle en $C_1$-$C_8$ qui peut contenir un ou plusieurs substituants choisis dans le groupe constitué par le radical phényle, un atome d'halogène, le radical nitro et le radical cyano, ou un radical aryle en $C_6$-$C_{12}$ qui peut contenir un ou plusieurs substituants choisis dans le groupe constitué par un radical alkyle en $C_1$-$C_8$, un atome d'halogène, un radical nitro et un radical cyano ; n représente un entier de 0 à 3 ; et les traits discontinu et continu indiquent que la liaison peut être une simple liaison ou une double liaison ; ou un sel pharmaceutiquement acceptable de celui-ci.

2. Composé selon la revendication 1, caractérisé en ce que chacun de $R^1$, $R^2$, $R^3$, $R^4$ et $R^5$ représente indépendamment un atome d'hydrogène, un atome d'halogène ou un groupe alkyle en $C_1$-$C_8$, alcoxy en $C_1$-$C_8$, alcoxyalcoxy en $C_2$-$C_6$, alcanoyloxy en $C_2$-$C_9$, arylcarbonyloxy en $C_7$-$C_{13}$, carboxy, alcoxycarbonyle en $C_2$-$C_9$, carbamoyle, alkylaminocarbonyle en $C_2$-$C_9$, arylaminocarbonyle en $C_7$-$C_{13}$, amino, alkylamino en $C_1$-$C_8$, alcanoylamino en $C_2$-$C_9$, arylcarbonylamino en $C_7$-$C_{13}$, éthylènedioxyméthyle, formyle, cyano, nitro ou trihalogénométhyle ; et $R^6$ représente un atome d'hydrogène, un groupe alkyle en $C_1$-$C_8$ ou un groupe aryle en $C_6$-$C_{12}$ qui peut être substitué avec un halogène.

3. Composé selon la revendication 1, caractérisé en ce que X représente -O- ; Y représente =$CR^5$- ; chacun de $R^1$, $R^2$, $R^3$, $R^4$ et $R^5$ représente indépendamment un atome d'hydrogène, un atome d'halogène ou un groupe alkyle en $C_1$-$C_5$, alcoxy en $C_1$-$C_5$, alcoxyalcoxy en $C_2$-$C_6$, alcanoyloxy en $C_2$-$C_6$, carboxy, alcoxycarbonyle en $C_2$-$C_6$, arylaminocarbonyle en $C_7$-$C_{13}$, amino, alcanoylamino en $C_2$-$C_6$, éthylènedioxyméthyle, formyle, cyano, nitro ou trihalogénométhyle ; et $R^6$ représente un atome d'hydrogène, un groupe alkyle en $C_1$-$C_5$ ou un groupe aryle en $C_6$-$C_{12}$ qui peut être substitué avec un halogène.

4. Composé selon la revendication 1 caractérisé en ce que le symbole

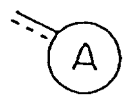

représente

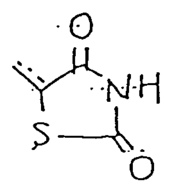 ;

X représente -O- ; Y représente =$CR^5$- ; chacun de $R^1$, $R^2$, $R^3$ et $R^4$ représente indépendamment un atome d'hydrogène ou un atome d'halogène ; $R^5$ représente un atome d'hydrogène ; $R^6$ représente un atome d'hydrogène ; n représente 1 ; et les traits discontinu et continu indiquent que la liaison est une simple liaison.

5. Composé selon la revendication 1 caractérisé en ce que le symbole

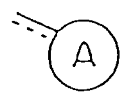

représente

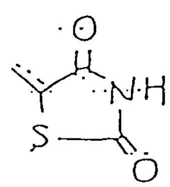 ;

X représente -O- ; Y représente =$CR^5$- ; $R^1$ représente un atome de fluor ; chacun de $R^2$, $R^3$ et $R^4$ représente un atome d'hydrogène ; $R^5$ représente un atome d'hydrogène ; $R^6$ représente un atome d'hydrogène ; n représente 1 ; et les traits discontinu et continu indiquent que la liaison est une simple liaison.

6. Composition pharmaceutique qui comprend comme ingrédient actif un composé selon la revendication 1 et un véhicule pharmaceutiquement acceptable.

7. Composition pharmaceutique contre le diabète, qui comprend comme ingrédient actif un composé selon la revendication 1 et un véhicule pharmaceutiquement acceptable.